# EUROPEAN PATENT SPECIFICATION

(11) **EP 3 010 890 B1**
(45) Date of publication and mention of the grant of the patent: **02.01.2019**
(21) Application number: 14732618.5
(22) Date of filing: 19.06.2014
(51) Int. Cl.: C07D 239/54, A61K 31/513, A61P 9/00, A61P 25/08, A61P 35/00

(54) **BIOORTHOGONAL METHODS AND COMPOUNDS**
BIOORTHOGONALE VERFAHREN UND VERBINDUNGEN
COMPOSÉS ET PROCÉDÉS BIO-ORTHOGONAUX

(30) Priority: 21.06.2013 GB 201311107
(43) Date of publication of application: 27.04.2016
(73) Proprietor: The University Court of the University of Edinburgh, South Bridge Midlothian EH8 9YL (GB)
(72) Inventor: UNCITI-BROCETA, Asier, Edinburgh EH8 9YL (GB); WEISS, Jason, Edinburgh EH8 9YL (GB)
(74) Representative: HGF Limited
(86) International application number: PCT/GB2014/051894
(87) International publication number: WO 2014/202994

(56) References cited:
- WO-A1-91/00724
- WO-A1-2011/057935
- CN-A- 103 044 391
- GB-A- 2 463 514
- US-A- 3 856 790
- US-A- 3 959 311
- US-A- 4 008 068
- US-A1- 2002 169 329
- US-A1- 2008 221 134
- JASON T. WEISS ET AL: "Extracellular palladium-catalysed dealkylation of 5-fluoro-1-propargyl-uracil as a bioorthogonally activated prodrug approach", NATURE COMMUNICATIONS, vol. 5, 13 February 2014 (2014-02-13), XP055134981, DOI: 10.1038/ncomms4277
- D. RAMBABU ET AL: "Pd/C-mediated depropargylation of propargyl ethers/amines in water", TETRAHEDRON LETTERS, vol. 54, no. 9, 1 February 2013 (2013-02-01), pages 1169-1173, XP055134984, ISSN: 0040-4039, DOI: 10.1016/j.tetlet.2012.12.093
- MANOJIT PAL ET AL: "Palladium-Catalyzed Cleavage of O/N-Propargyl Protecting Groups in Aqueous Media under a Copper-Free Condition 1", ORGANIC LETTERS, vol. 5, no. 3, 1 February 2003 (2003-02-01), pages 349-352, XP055134982, ISSN: 1523-7060, DOI: 10.1021/ol027382t
- ALCAIDE B ET AL: "HEXACARBONYL DICOBALT COMPLEXED N-PROP-YNYL-2-AZETIDINONES: A NEW ENTRY TO N-UNSUBSTITUTED-BETA-LACTAMS THROUGH A NICHOLAS-TYPE REACTION", JOURNAL OF THE CHEMICAL SOCIETY, CHEMICAL SOCIETY, LETCHWORTH; GB, vol. 5, 1 January 1994 (1994-01-01), page 587/588, XP001056462, ISSN: 0368-1769
- JOSEPH W. TUCKER ET AL: "Tandem Visible Light-Mediated Radical Cyclization-Divinylcyclopropane Rearrangement to Tricyclic Pyrrolidinones", ORGANIC LETTERS, vol. 13, no. 20, 21 October 2011 (2011-10-21), pages 5468-5471, XP055134968, ISSN: 1523-7060, DOI: 10.1021/ol202178t
- Benjamin B Thompson ET AL: "Enone-alkyne reductive coupling: a versatile entry to substituted pyrroles", Organic letters, 1 July 2011 (2011-07-01), pages 3289-3291, XP055134370, United States DOI: 10.1021/ol201133n Retrieved from the Internet: URL:http://www.ncbi.nlm.nih.gov/pubmed/216 57241
- GU YU GUI ET AL: "Synthesis and structure-activity relationships of N-{3-[2-(4-alkoxyphenoxy)thiazol-5-yl]-1-m ethylprop-2-ynyl}carboxy derivatives as selective acetyl-CoA carboxylase 2 inhibitors", JOURNAL OF MEDICINAL CHEMISTRY, AMERICAN CHEMICAL SOCIETY, US, vol. 49, no. 13, 1 June 2006 (2006-06-01), pages 3770-3773, XP002476341, ISSN: 0022-2623, DOI: 10.1021/JM060484V
- Gholam Hossein Hakimelahi ET AL: "A Novel Approach towards Studying Non-Genotoxic Enediynes as Potential Anticancer Therapeutics", Bioorganic & Medicinal Chemistry, vol. 10, no. 5, 1 May 2002 (2002-05-01), pages 1321-1328, XP055413986, GB ISSN: 0968-0896, DOI: 10.1016/S0968-0896(01)00393-5
- KATOONO R ET AL: "[10]Paracyclophanediamides and their octadehydro derivatives: novel exotopic receptors with hydrogen-bonding sites on the bridge", TETRAHEDRON LET, ELSEVIER, AMSTERDAM, NL, vol. 45, no. 46, 8 November 2004 (2004-11-08), pages 8455-8459, XP004602236, ISSN: 0040-4039, DOI: 10.1016/J.TETLET.2004.09.115

## Description

### TECHNICAL FIELD

This invention relates to a new bioorthogonal deprotection method for general application in preparing heterocyclic compounds. Compounds for use in such methods are also provided, including new prodrugs that can be converted to active drug in a spatially-controlled manner *in situ* by palladium catalysis. Thus, the use of such prodrugs in therapy is also provided, particularly anti-cancer therapy.

### BACKGROUND

### Bioorthogonal chemistry

As reported by Bertozzi, et al. in the early 2000's (Bertozzi, C. R. et al. Science, 2000, 287, 2007-2010 and Bertozzi, C. R. et al. J. Am. Chem. Soc. 2004, 126, 15046-15047), artificial synthetic chemistry can be conducted in a biological environment without adverse biological effects using highly chemospecific reactive partners. Such reactions which proceed in a biological environment without adverse biological consequences are now commonly referred to as being "bioorthogonal".

Initial bioorthogonal studies focussed on the development of labelling strategies based on the selective conjugation of two biologically-inert functional groups. This development has since enabled the real-time study of a wide range of biomolecules in their native environs (see, e.g. Bertozzi, C. R. Acc Chem Res. 2011, 44, 651-653).

Studies of the range of chemical reactions that can be conducted in a bioorthogonal manner are still progressing. It has been reported that the Staudinger ligation (Bertozzi, C. R. et al. Science, 2000, 287, 2007-2010) and conjugation reactions between "springloaded" reactive partners (e.g. strain-promoted [3+2] azide-alkyne cycloaddition (Bertozzi, C. R. et al. J. Am. Chem. Soc. 2004, 126, 15046-15047), nitrone-cyclooctyne 1,3-dipolar cycloaddition (Ning, X. et al. Angew. Chem. Int. Ed. 2010, 49, 3065-3068) and trans-cyclooctene tetrazine ligation (Blackman, M. L. et al. J. Am. Chem. Soc. 2008, 130, 13518-13519) amongst others can be performed in a bioorthogonal manner.

### Transition metal catalysed reactions

Transition metal catalysed reactions are an extremely powerful tool in organic synthesis as they provide chemospecific reaction profiles and facilitate a wide range of chemical transformations. From a bioorthogonal synthetic perspective, it is therefore desirable to develop bioorthogonal transition metal catalysed reactions that can perform efficiently in a biological environment to provide the biosynthetic chemist with more synthetic flexibility.

A large variety of transition metal catalysed reactions have been reported in the literature. However, there has been limited success in the application of such reactions in a biological environment. This is undoubtedly because a large number of reported reaction conditions are simply incompatible with a biological environment, e.g. requiring organic solvents and / or high temperatures, etc. For instance, the palladium-mediated cleavage of propargyl protecting groups from aryl amines requires biologically incompatible temperatures of at least 80 °C (see, e.g. Pal, M. et al., Org. Lett. 2003, 5(3), 349-352, or Rambabu, D. et al., Tetrahedron Letters 2013, 54(9), 1169-1173). Examples of propargyl group-containing compounds include those shown in, e.g. Ancaide, B. et al., J. Chem. Soc. 1994, 5, 587/588, Tucker, J.W. et al., Org. Lett. 2011, 13(20) 5468-5471, Thompson, B.B. et al., Org. Lett. 2011, 3289-3291, Gui, G.Y. et al., J. Med. Chem. 2006, 49, 3770-3773, and in US patent no. US 3,856, 790 to Freed et al., International patent application no. WO 91/00724 to Abbott Lab, US patent no. US 3,959,311 to Dahlbom et al, US patent application no. US 2002/169329 to Mori Tatsuya et al., US patent no. US 4,008,068 to Krenzer, International patent application no. WO 2011/057935 to BASF SE, Chinese patent application no. CN 103044391 to Guangzhou Inst Biomed & Health, US patent application no. US 2008/221134 to Muller et al., and UK patent application no. GB 2463514 to Galapagos NV. Further examples of propargyl group-containing compounds include those shown in, e.g. Gholam Hossein Hakimelahi et al. Bioorganic & Medicinal Chemistry, vol. 10 no. 5, 1 May 2002, pages 1321-1328, and Katoono et al. Tetrahedron Letters, vol. 45, no.46, 8 November 2004, pages 8455-8459.

Certain non-biological transition metal-catalysed reactions have however been shown to be promising candidates for use in bioorthogonal synthesis (e.g. Unciti-Broceta, A. et al. Nature Protocols, 2012, 7, 1207-1218 and Meggers, E. et al. Chem Commun. 2013, 49, 1581-1587). Such bioorthogonal organometallic (BOOM) reactions are biocompatible and involve chemospecific transformations undertaken usually by synthetic materials and mediated by a non-biotic metal source as described below.

In 2006, Meggers et al. described the application of a water-soluble ruthenium-based catalyst to carry out Allyl carbamate (Alloc) deprotection of bis-*N,N'-*allyloxycarbonyl rhodamine 110 inside human cells without adversely affecting cell viability (Meggers, E. et al., Angew. Chem. Int. Ed. 2006, 45, 5645-5648). The use of Pd⁰-functionalized microspheres as a heterogeneous catalyst medium for promoting BOOM chemistry inside cells has also been reported (Bradley, M. et al., Nat. Chem. 2011, 3, 239-243 and Unciti-Broceta, A. et al. Nature Protocols, 2012, 7, 1207-1218). The palladium-functionalized microspheres were shown to be able to enter cells *in vitro* and catalyse Alloc deprotection and Suzuki-Miyaura cross-coupling in the cell cytoplasm without any observed cytotoxicity. Palladium(II)-catalysed Sonogashira coupling of homopropargylglycine (HPG)-encoded ubiquitin protein with aryl iodides in basic aqueous media has also been disclosed (Li, N. et al., J Am Chem Soc. 2011, 133, 15316-15319). This methodology has been shown to be suitable for labelling (HPG)-encoded ubiquitin protein with fluorescein iodide in *E. coli* cells. The use of a palladium-catalysed Suzuki reaction to label *E. coli cell* surface components (Spicer, C. D. et al., J. Am. Chem. Soc. 2012, 134, 800-803) and the application of palladium-mediated carbonylation in the detection of intracellular carbon monoxide has also been disclosed (Michel, B. W. et al. J Am Chem Soc. 2012, 134, 15668-15671).

The main focus of research on bioorthogonal chemical reactions has thus been on labelling biomolecules with application in the development of biochemical probes for detecting certain chemical species in cells. Where transition metal catalysts are required to perform the required chemistry, the transition metal must therefore be provided in a form that is able to enter the cell.

As research into bioorthogonal reactions continues to develop, there is an increasing desire to provide access to alternative reaction systems that may be used to effect bioorthogonal chemical transformations selectively in a biological environment. Such alternative reaction systems would therefore provide useful methodological tools in the synthetic chemist's armoury, opening up a wider variety of chemistry that can be performed in a bioorthogonal manner. In particular, the provision of new protection or deprotection methods that may be performed in a biological environment would provide a step towards performing more complex syntheses in a controlled way in a biological environment and could find utility in preparing biochemical probes and prodrugs.

There is therefore a desire for new biocompatible and, preferably, bioorthogonal methods and compounds that are primed to react in a bioorthogonal way and which may thus be useful in a biological environment.

### Biomedical applications

In biomedicine, bioorthogonal deprotection methods could be utilised to transform a bioorthogonal chemical into a bioactive material. Prodrugs, for example, are drug precursors that are converted to active drug following administration to a patient, typically by chemical rearrangement of the prodrug and / or by cleavage of a pro-moiety by natural biological metabolism. Typically, prodrugs are based on drugs that have been protected with a cleavable protecting group or pro-moiety. By providing a drug precursor that produces the drug in the body, the medicinal chemist can provide compounds that exhibit improved pharmacokinetic properties compared to the active drug, such as greater oral bioavailability and sustained release profiles.

For safety and simplicity, it is desirable to provide prodrugs that do not exhibit biological activity themselves. The activity profile of the prodrug is then entirely dependent on the metabolic conversion of the prodrug to the active drug, providing a greater degree of predictability of biological activity *in vivo.*

Typically, prodrugs are converted to the respective active drugs in the gut (for orally administered drugs), and / or by general cellular and / or plasma-based metabolic pathways. Conventional prodrugs are thus converted to active drug in a non-bioselective manner, leading to general systemic exposure of the body cells to the active drug, which may result in undesirable side effects.

It is therefore desirable from a toxicological perspective to be able to deliver drugs specifically to the relevant target / disease site. Thus, prodrugs that may be converted to active drug in a spatially controlled manner may offer a way to enable active drug to be produced only where it is needed, i.e. at specific target sites, such as a specific disease site in the body, thus minimising the general systemic exposure of the patient to the active drug. Importantly, a spatially-targeted approach would serve to expand the therapeutic window and scope of potent cytotoxic drugs such as 5-FU, which have a long history in oncology practice but a clinical activity limited by its safety profile (Chu, et al. J. Natl. Cancer Inst. 101, 1543 (2009)), and to allow the medical application of highly-promising experimental drugs that failed to progress through clinical trials to approval due to toxicological issues. An appropriate prodrug strategy may therefore allow a wide range of drugs to reach the clinic in an optimized manner.

Bioorthogonal chemistry provides a possible way in which this can be achieved, for example, by providing prodrugs that can be converted to the drug *in vivo* using BOOM chemistry. Thus, it is desirable that such prodrugs are (i) bioorthogonal and (ii) highly sensitive to metal-based catalysis.

Ideally, the bioorthogonality of the prodrug should be two-fold: the prodrug should preferably neither interact with the therapeutic target/s nor be biochemically metabolized into the drug (unlike conventional prodrugs). This behaviour may be attained by modifying a drug structure at a position that is mechanistically-relevant to its pharmacological activity with chemical groups that cannot be easily recognized by human enzymes. Effective masking strategies should ideally reduce the pharmacological properties of the active drug over 100 fold (Bagshawe, K. D. Expert Rev Anticancer Ther, 2006, 6, 1421-1431). The design of a suitable masking strategy is therefore an important aspect of this approach. On the other hand, the corresponding metallic agent needs to be biocompatible (ideally bioorthogonal) and able to coordinate with and cleave the drug's masking group in physiological conditions (aqueous solvent, physiological temperature, pH, etc.). Importantly, the active oxidation state of the metal therefore needs to be compatible with the inherent redox potential of the biological environment. Preferably, the BOOM reaction should also be catalytic, to allow a repetitive dosing regimen to be implemented.

### SUMMARY OF INVENTION

On a general level, the inventors propose a new bioorthogonal synthetic process comprising the palladium-mediated cleavage of an optionally substituted propargyl protecting group (i.e. pro-moiety) from an endocyclic nitrogen atom in a heterocyclic system comprising a ring carbonyl group adjacent to the endocyclic nitrogen atom. Suitably, the bond between the optionally substituted propargyl protecting group and the endocyclic nitrogen of the heterocyclic group is resistant to cleavage by biological metabolic pathways but is readily cleaved under biological conditions using palladium. Such heterocyclic systems encompass a variety of useful compound classes, such as drugs, biomarkers, and fluorescent dyes etc. Thus, the processes of the invention provide the skilled person with a powerful new synthetic tool for preparing useful heterocyclic compounds in a controlled manner and in a biological environment. These methods would therefore have general synthetic utility in bioorthogonal synthetic processes.

The present invention also provides new heterocyclic compounds useful in such bioorthogonal synthetic processes. As described above, the compounds comprise an optionally substituted propargyl protecting group (pro-moiety) bonded to an endocyclic nitrogen atom of a heterocyclic group wherein the endocyclic nitrogen is adjacent to a ring carbonyl group. In a particularly useful application, the heterocyclic compounds are bioorthogonal prodrugs wherein the bond between the optionally substituted propargyl group protecting group and the endocyclic nitrogen is resistant to general metabolic cleavage, but is susceptible to palladium-mediated cleavage in a biological environment to provide effective quantities of the free active heterocyclic drug in a controlled chemospecific manner. Thus, such compounds may be used as bioorthogonal prodrugs in methods of treatment involving the co-administration of palladium, e.g. where palladium implants are used.

### DETAILED DESCRIPTION

In an aspect of the invention is provided a method of preparing a heterocyclic compound or a salt thereof, the method comprising:
a) providing a first compound comprising a first group defined according to formula (II): 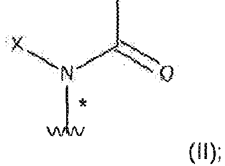 bonded to a second group defined according to formula (III) at the positions indicated by asterisks 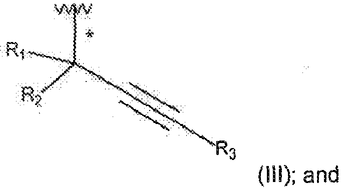
b) cleaving the bond between the first and second groups by reacting the first compound with palladium Pd(0) in aqueous conditions, wherein
   X and Y taken together with the endocyclic nitrogen atom and ring carbonyl group to which they are attached form a heterocyclic group; and
   R₁, R₂ and R₃ are selected independently from the group consisting of H,
   C₁₋₄alkyl, C₂₋₄alkenyl, and phenyl,
   wherein the heterocyclic compound or salt is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine.

### Bond cleavage

Suitably, the bond between group (II) and (III) as referred to herein is a covalent bond. As discussed in the examples, this bond is not cleaved readily under natural metabolic conditions. However, advantageously, the bond is cleavable using palladium. Surprisingly, the bond may be cleaved efficiently under ambient conditions. For instance, the bond between the first and second group in compounds of the invention may be cleaved under biocompatible conditions (e.g. aqueous conditions, such as in PBS, at physiological pH and at around ∼37 °C or less). Thus, suitably, the bond cleavage may be performed in aqueous media. The reaction may be performed at around physiological pH. Furthermore, the reaction may be performed at around 37 °C or less. In embodiments, the method of the invention is performed at a temperature of 100 °C or less, such as 90, °C or less, for example, 80, 70, 60, 50, or 40 °C or less. For biological applications, the reaction temperature is preferably 40 °C or less, typically less than 40 °C, preferably around 37 °C.

As seen in the examples, the bond cleavage in the present methods proceeds efficiently in biocompatible conditions to provide the desired heterocyclic compounds (i.e. the drug compound 5-flurouracil (5FU) in the examples). In embodiments, the methods of the invention proceed to at least 10 % completion (i.e. wherein at least 10% of the starting compound has been cleaved to provide the desired heterocyclic product, e.g. drug compound) within 72 h from when reaction with the palladium commences. Suitably, the methods of the invention proceed to at least 20% completion within 72 h, such as at least 30 %, 40%, 50%, 60%, 70%, 80%, 90%, 95%, 98%, preferably 99% and more preferably 100% completion within 72 h, more preferably within 48 h, such as within 24 h, e.g. within 10 h.

As seen in the examples, Pro-5FU is cleaved to form 5FU in biocompatible conditions in a useful timeframes (i.e. ∼24h) to provide useful amounts of the free compound. This advantageous aspect of the present methods was unexpected, particularly given the biologically incompatible temperatures and reaction conditions reported to be required for promoting analogous palladium-mediated propargyl deprotection of aryl amines in the literature (see, e.g. Pal, M. et al., Org. Lett. 2003, 5(3), 349-352). Thus, the present methods provide the skilled person with a new bioorthogonal synthetic tool which can be exploited in bioorthogonal syntheses with general application in accessing a wide range of heterocyclic compounds in a controlled manner in biological environments, e.g. *in vivo.* Of course, the heterocyclic compounds and salts thereof that are produced by the present methods encompass a variety of useful compound classes, such as drugs, and diagnostic biomarkers, etc. For prodrug applications, i.e. where the compound of the invention is cleaved by a method of the invention to form a drug compound, it is desirable for the cleavage reaction to proceed efficiently *in vivo* to maximise the amount of drug which is generated by reaction with palladium at the specific target site. As the free drug is released by reaction of the prodrug with palladium in a spatially controlled manner, it is not necessary that all prodrug administered in the sample is cleaved *in vivo* to form the free drug, provided the amount of free drug that is released by the prodrug bond cleavage is adequate for providing a therapeutic effect. The amount of free drug necessary to achieve a therapeutic effect will depend on the drug and the condition being treated.

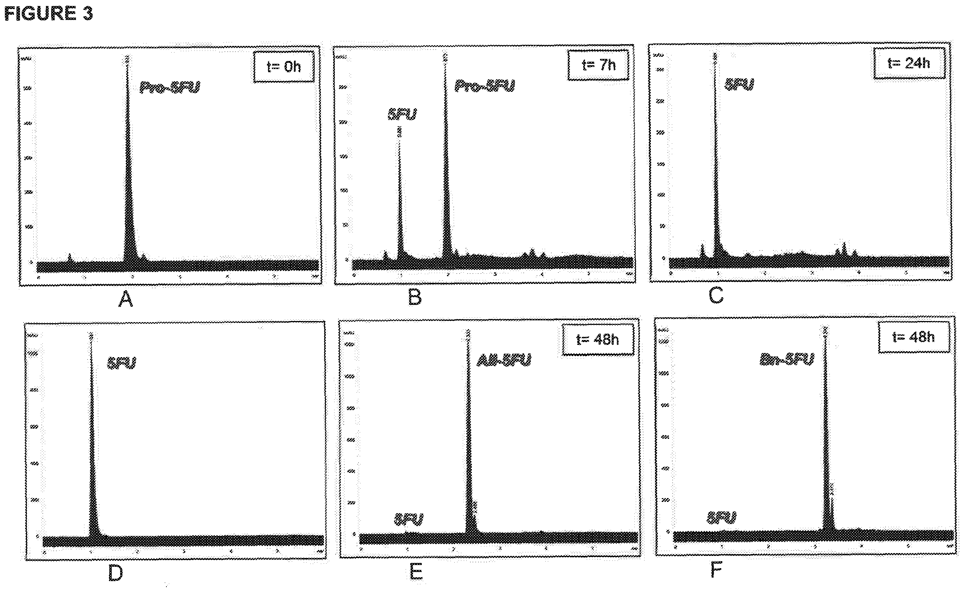

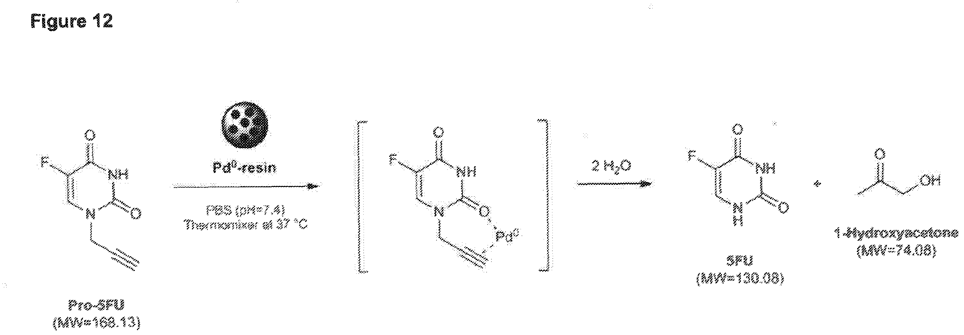

Furthermore, the cleavage of the bond between the first and second groups is observed to proceed cleanly to provide the free heterocyclic compound and the deprotected second group as the only by-product (see Figures 3A-C). The reaction is therefore extremely atom-efficient. Advantageously, the respective ketone by-product formed from cleavage of the propargyl-based protecting group is typically benign. For instance, when the group of formula (III) is a propargyl group (i.e. wherein R₁-R₃ are each H), the reaction by-product formed (as detected by mass spectroscopy) is 1-hydroxyacetone, which is a natural product formed during lipid metabolism (see, for example, Figure 12).

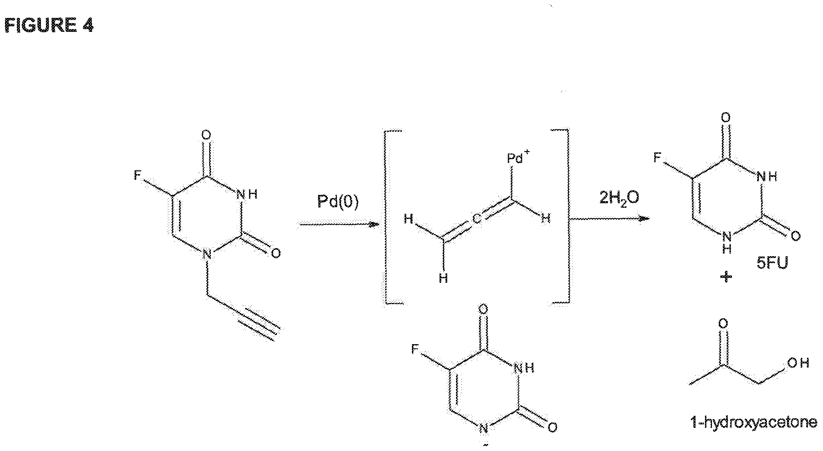

It is believed that the mechanism of cleavage of such propargyl groups by palladium as described in the present methods principally involves the steps of coordination of palladium to the triple bond of formula (III), followed by insertion of the palladium into the triple bond (oxidatively or ionically). Oxidative addition for instance is typically observed when palladium(0) is used as the palladium source, as described in Figure 4, resulting in formation of an allenyl palladium intermediate. The heterocyclic group is eliminated, thus breaking the bond between the first (heterocyclic) group and the second (alkyne containing) group.

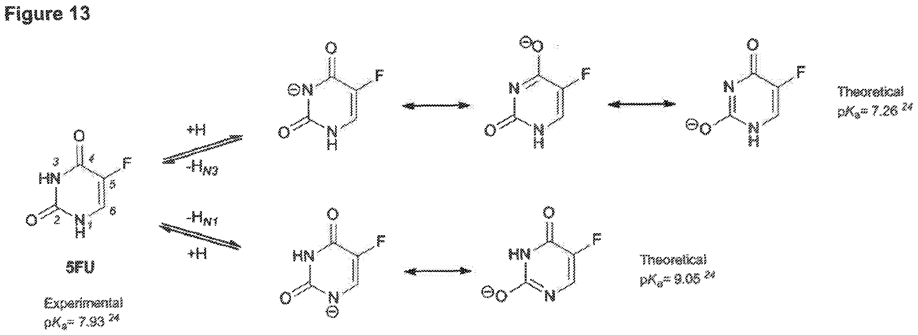

Without wishing to be bound by theory, the present inventors postulate that the ability of the bond between the first and second groups to be cleaved in such ambient biological conditions in the present methods is a result of two key structural factors:
a) the presence of a ring carbonyl group adjacent to the endocyclic nitrogen allows the negative charge residing at the endocyclic nitrogen following bond cleavage to be delocalised in the heterocyclic ring, thus increasing the stability of the heterocyclic anionic intermediate formed. This anionic delocalisation is exemplified in Figure 13 using 5-fluorouracil as an example; and
b) the fixed cyclic conformation of the heterocyclic ring in the group as defined in formula (II) ensures that the alkyne triple bond and ring carbonyl group in the group of formula (III) are free to coordinate to the palladium, thus providing an entropic benefit. The spatial arrangement of the propargyl group of formula (III) relative to the ring carbonyl group is probably optimal for reducing the activation energy of the cleavage reaction (possibly via promotion of a cyclic palladium intermediate as illustrated in Figure 12), as opposed to the allyl group configuration (see e.g. All-5FU in the examples), which was shown to exhibit no significant reactivity to cleavage by palladium at analogous ambient reactions conditions. This was surprising as propargyl and allyl ethers and amines reported in the literature are typically cleaved by palladium under similar reaction conditions respectively.

Suitably, the methods of the present invention may therefore be performed in a biological environment, such as in a cell, a tissue and / or a patient using a suitable palladium source.

In embodiments, the method of the invention is performed *in vivo.* Accordingly, in an embodiment is provided a method of preparing a heterocyclic compound or salt thereof *in vivo,* the method comprising:
a) providing a first compound comprising a first group defined according to formula (II): 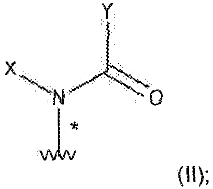 bonded to a second group defined according to formula (III) at the positions indicated by asterisks 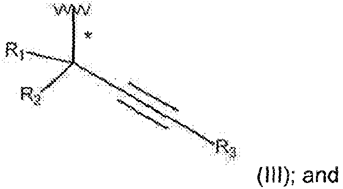
b) cleaving the bond between the first and second groups by reacting the first compound with palladium *in vivo,*
   wherein
   X and Y taken together with the endocyclic nitrogen atom and ring carbonyl group to which they are attached form a heterocyclic group; and
   R₁, R₂ and R₃ are selected independently from the group consisting of H, C₁₋₄alkyl, C₂₋₄alkenyl, and phenyl,
   wherein the heterocyclic compound or salt thereof is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine.

The reaction may suitably be performed in vivo by administration of the compound comprising the groups (II) and (III) and palladium to a subject. Modes of administration are discussed further below. In embodiments, the method is not a method of treatment of the human or animal body by therapy.

In embodiments, the method is an *in vitro* method. Accordingly, in an embodiment is provided a method of preparing a heterocyclic compound or salt thereof *in vitro,* the method comprising:
a) providing a first compound comprising a first group defined according to formula (II): 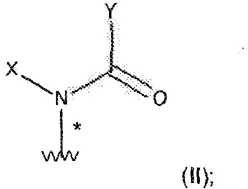 bonded to a second group defined according to formula (III) at the positions indicated by asterisks 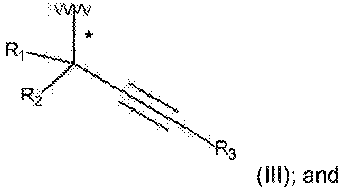
b) cleaving the bond between the first and second groups by reacting the first compound with palladium *in vitro,*
   wherein
   X and Y taken together with the endocyclic nitrogen atom and ring carbonyl group to which they are attached form a heterocyclic group; and
   R₁, R₂ and R₃ are are selected independently from the group consisting of H, C₁₋₄alkyl, C₂₋₄alkenyl, and phenyl,
   wherein the heterocyclic compound or salt thereof is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine.

### The heterocyclic compound or salt thereof

The methods of the present invention defined in the above aspects and embodiments provide heterocyclic compounds or salts thereof. The heterocyclic compounds are produced by cleavage of the bond (i.e. the covalent bond) between the group of formula (II) and the group of formula (III) as defined above. Thus, in embodiments the method of the present invention provides a heterocyclic compound as defined according to formula (I): 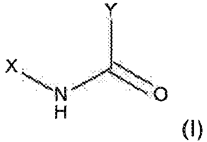 or a salt thereof,
wherein X and Y are taken together with the endocyclic nitrogen atom and ring carbonyl group to which they are attached form a heterocyclic compound or a salt thereof.

In embodiments, the heterocyclic compound or salt thereof as defined in any of the above aspects and embodiments (such as the heterocyclic compound of formula (I) or salt thereof) is a purine or pyrimidine compound or analog thereof, wherein the compound or salt thereof is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine. For example, the heterocyclic compound or salt may be a pyrimidine compound or analog thereof. In typical embodiments, the heterocyclic compound or salt thereof is a nucleobase or nucleobase analog thereof, preferably wherein the nucleobase or nucleobase analog is selected from cytosine, guanine, thymine, uracil and analogs thereof, particularly analogs thereof.

In further embodiments, the heterocyclic compound or salt thereof is selected from the group consisting of: 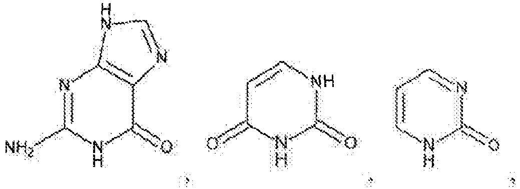 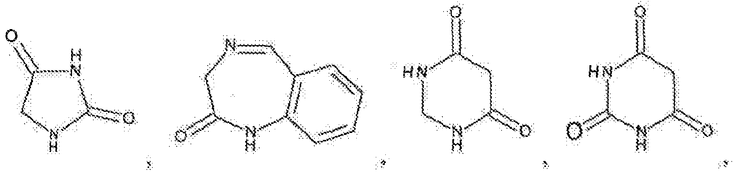 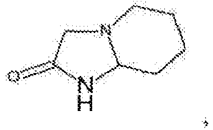 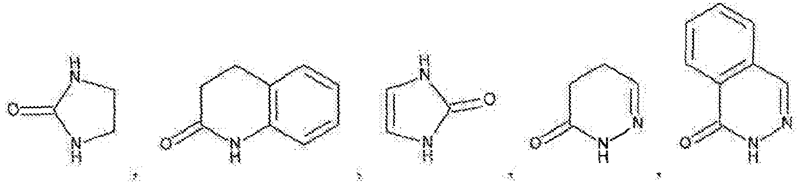 and 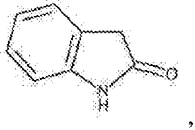 wherein said heterocyclic compound is optionally substituted.

For instance, in embodiments, the heterocyclic compound is selected from the group consisting of 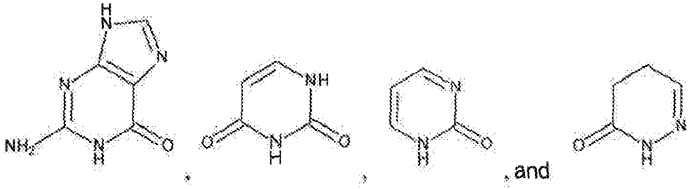 wherein said heterocyclic compound is optionally substituted.

In embodiments, the heterocyclic compound is selected from the group consisting of: 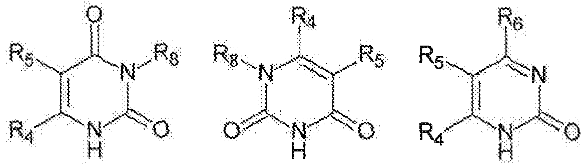 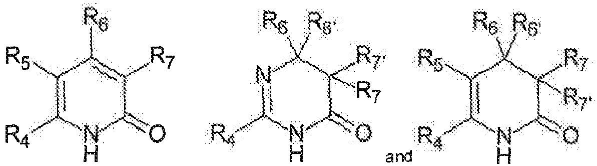 wherein
each R₄, R₅, R₆ and R₇ is independently selected from the group consisting of H, OH, NO₂, N₃, halo, cyano, optionally substituted amino, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted C₂₋₁₀heteroalkynyl, optionally substituted C₆₋₁₄aryl and optionally substituted C₅₋₁₄heteroaryl; optionally wherein any of R₄, R₅, R₆ and R₇ are taken together with an adjacent group and the carbon atoms to which they are attached to form a cyclic group;
each R_{6'} and R_{7'} is independently selected from the group consisting of H, optionally substituted C₁₋₁₀alkyl, optionally substituted C₂₋₁₀alkenyl, optionally substituted C₂₋₁₀alkynyl, optionally substituted C₂₋₁₀heteroalkyl, optionally substituted C₂₋₁₀heteroalkenyl, and optionally substituted C₂₋₁₀heteroalkynyl;
each R₈ is independently selected from the group consisting of H, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted c₂₋₁₀heteroalkynyl, optionally substituted C₆₋₁₄aryl and optionally substituted C₅₋₁₄heteroaryl.

In embodiments, the heterocyclic compound is selected from the group consisting of: 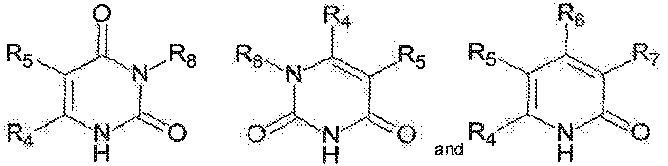 wherein
R₄, R₅, R₆, R₇ and R₈ are as defined above; preferably wherein the heterocyclic compound is selected from the group consisting of 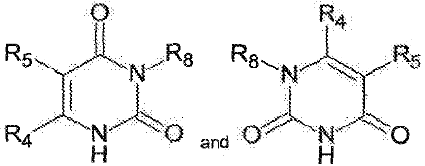 wherein R₄, R₅, R₆, R₇ and R₈ are as defined above.

In the methods of the invention described above, the heterocyclic compound or salt thereof is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine. In other words, in embodiments, the heterocyclic compound is a drug compound comprising an endocyclic nitrogen adjacent to a ring carbonyl according to any embodiment provided above. Thus, compounds used in the present methods comprising the first and second bonded groups of formula (II) and (III) are useful prodrugs that may be suitably deprotected in a controlled manner using palladium to reveal the active drug compound, e.g. *in vitro* or *in vivo.*

In typical embodiments, the heterocyclic compound or salt thereof is a drug compound selected from the group consisting of an anti-cancer drug, an anti-Parkinson's disease drug, an antibiotic, an anti-fungal drug, an anti-viral drug, an anti-psychotic drug, an anti-convulsant and a heart disease drug (i.e. a drug for treating heart disease). In embodiments, the heterocyclic compound or salt thereof is a drug compound selected from the group consisting of an anti-cancer drug and an anti-viral drug, preferably an anti-cancer drug. In embodiments, the anti-cancer is a drug for treating pancreatic or colorectal cancer, for instance, wherein the colorectal cancer includes cancerous HCT116 cells and / or wherein the pancreatic cancer includes cancerous BXPc-3 cells. Preferably the drug compound is an antimetabolite.

In embodiments, the anti-cancer drug is selected from the group consisting of 5-fluorouracil (5-FU), pemetrexed, olaparib, sunitinib, floxuridine and uramustine; or a derivative thereof, preferably selected from 5-fluorouracil (5-FU) and floxuridine; or a derivative thereof, e.g. 5-fluorouracil (5-FU). In embodiments, the anti-Parkinson's disease drug is ropinirole or a derivative thereof. Suitably, the antibiotic may be nitrofurantoin or a derivative thereof. In embodiments, the anti-fungal drug is flucytosine or a derivative thereof. In embodiments, the anti-viral drug is selected from the group consisting of aciclovir, iodoxuridine, stavudine, telbivudine, zidovudine, trifluridine and entecavir; or a derivative thereof. In embodiments, the anti-psychotic drug is selected from the group consisting of phenobarbital, methylphenobarbital, primidone, lorazepam, nitrazepam, clonazepam, ethotoin, phenytoin, mephenytoin and fosphenytoin; or a derivative thereof. In embodiments, the anti-convulsant drug is selected from the group consisting of aripiprazole, sertindole, ziprasidone and mosapramine; or a derivative thereof. In embodiments the heart disease drug is selected from the group consisting of amrinone, milrinone, pimobendan, enoximone and cilostazol; or a derivative thereof, preferably selected from amrinone and milrinone; or a derivative thereof. Typically, said drugs are not selected from drug derivatives.

Thus, in embodiments, the heterocyclic compound or salt thereof is a drug selected from the group consisting of 5-fluorouracil (5-FU), pemetrexed, olaparib, ropinirole, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine; or a derivative thereof. For example, the heterocyclic compound or salt thereof may be a drug selected from the group consisting of 5-fluorouracil (5-FU), pemetrexed, olaparib, ropinirole, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine and entecavir. In typical embodiments, the heterocyclic compound or salt thereof is a drug selected from the group consisting of 5-fluorouracil (5-FU), ropinirole, milrinone, amrinone, floxuridine, flucytosine, aciclovir, iodoxuridine, stavudine, telbivudine, zidovudine, trifluridine and entecavir; or a derivative thereof. In more preferred embodiments, the drug is 5-fluorouracil (5-FU) or a derivative thereof, such as 5-fluorouracil (5-FU).

### Compounds for use in the methods of the invention

The method of the invention as defined in the above aspect includes providing a first compound comprising a first group defined according to formula (II): 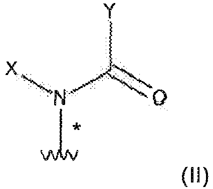 bonded to a second group according to formula (III)

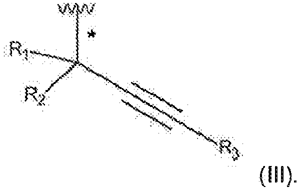

In other words, the method includes providing a compound as defined according to formula (IV): 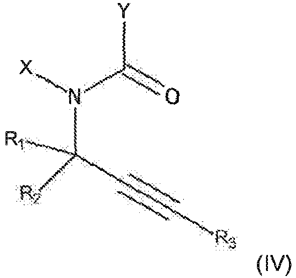 wherein X, Y, R₁, R₂ and R₃ are as defined above

### First group according to formula (II)

In the first group of formula (II) or in the compound of formula (IV) above, X and Y are taken together to form a heterocyclic group. The said heterocyclic group is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine.

In further embodiments, the group according to formula (II) is selected from the group consisting of: 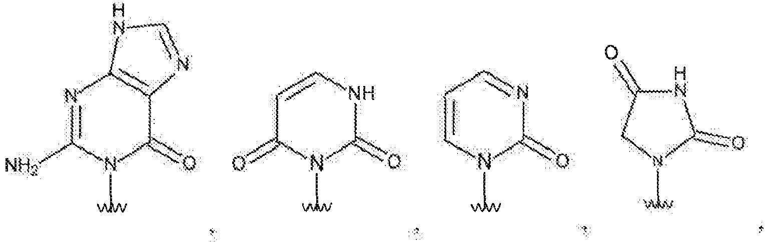 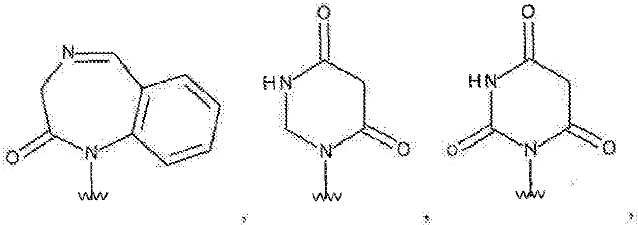 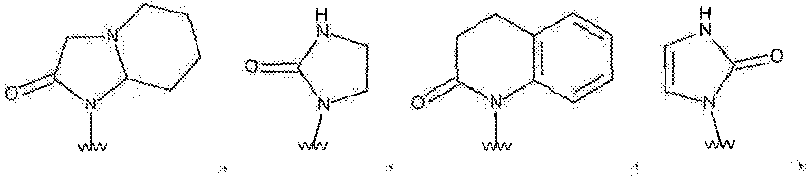 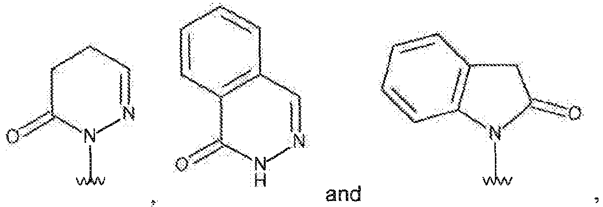 wherein said heterocyclic group is optionally substituted.

For instance, in embodiments, the group according to formula (II) is selected from the group consisting of 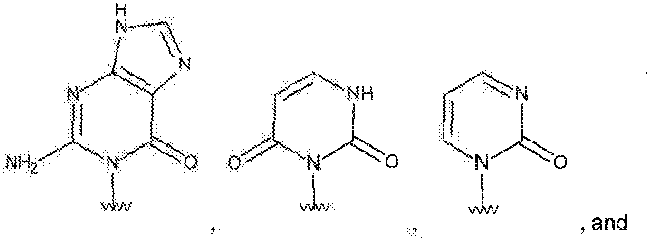 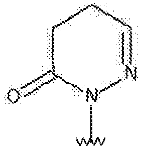 wherein said group is optionally substituted.

In embodiments, the group according to formula (II) is selected from the group consisting of: 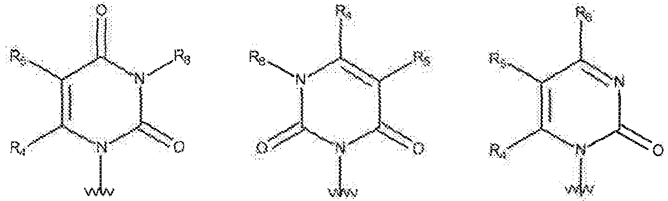 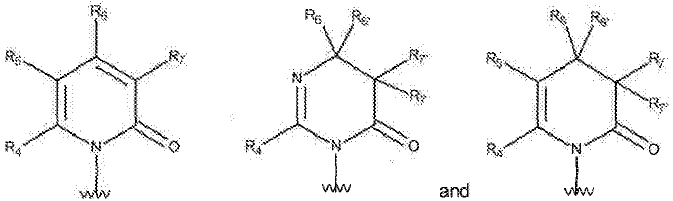 wherein
each R₄, R₅, R₆ and R₇ is independently selected from the group consisting of H, OH, NO₂, N₃, halo, cyano, optionally substituted amino, optionally substituted C₁₋₁₀alkyl, optionally substituted c₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, C₂₋₁₀heteroalkynyl, optionally substituted C₆₋₁₄aryl and optionally substituted C₅₋₁₄heteroaryl; optionally wherein any of R₄, R₅, R₆ and R₇ are taken together with an adjacent group and the carbon atoms to which they are attached to form a cyclic group;
each R_{6'} and R_{7'} is independently selected from the group consisting of H, optionally substituted C₁₋₁₀alkyl, optionally substituted C₂₋₁₀alkenyl, optionally substituted C₂₋₁₀alkynyl, optionally substituted C₂₋₁₀heteroalkyl, optionally substituted C₂₋₁₀heteroalkenyl, and optionally substituted C₂₋₁₀heteroalkynyl; and
each R₈ is independently selected from the group consisting of H, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted C₂₋₁₀heteroalkynyl, optionally substituted C₆₋₁₄aryl and optionally substituted C₅₋₁₄heteroaryl.

In embodiments, the group according to formula (II) is selected from the group consisting of: 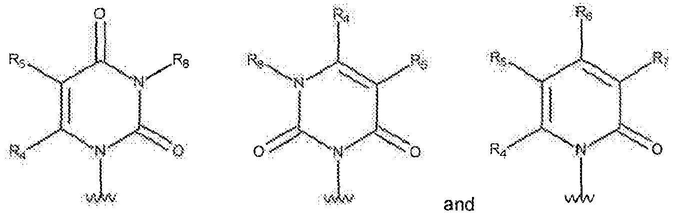 wherein
R4, R₅, R₆, R₇ and R₈ are as defined above; preferably wherein the heterocyclic group is selected from the group consisting of 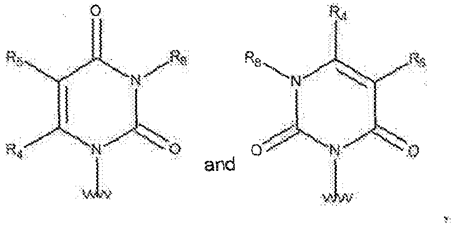 wherein R₄, R₅, R₆, R₇ and R₈ are as defined above.

In the methods of the invention described above, the heterocyclic group according to formula (II) may suitably be a drug residue (i.e. a drug residue according to formula (II)), i.e. wherein the compound or salt is a prodrug. The term drug residue in this context is intended to refer to a group based on an active drug compound, but wherein the bond between the group of formula (II) and the group of formula (III) notionally replaces a hydrogen atom bonded to the endocyclic nitrogen adjacent to the ring carbonyl in the active drug.

As above, the notional replacement is not intended to mean that the compounds have been necessarily formed by actual replacement of the hydrogen atom in a synthetic process of preparation.

Thus, compounds used in the present methods comprising the first and second bonded groups are useful prodrugs that may be suitably deprotected in a controlled manner using palladium to reveal the active drug compound, e.g. *in vitro* or *in vivo.*

In typical embodiments, the group according to formula (II) is a drug residue selected from the group consisting of residues of an anti-cancer drug, an anti-Parkinson's disease drug, an antibiotic, an anti-fungal drug, an anti-viral drug, an anti-psychotic drug, an anti-convulsant and a heart disease drug (i.e. a drug for treating heart disease). In embodiments, the drug residue is selected from the group consisting of residues of an anti-cancer drug and an anti-viral drug, preferably an anti-cancer drug. In preferred embodiments, said residues of an anti-cancer drug may be selected from residues of drugs for treating pancreatic and / or colorectal cancer, for instance, wherein the colorectal cancer includes cancerous HCT116 cells and / or wherein the pancreatic cancer includes cancerous BXPc-3 cells. Preferably the drug residue is a residue of an antimetabolite.

In embodiments, the anti-cancer drug residue is selected from the group consisting of residues of 5-fluorouracil (5-FU), pemetrexed, olaparib, sunitinib, floxuridine and uramustine; or a derivative thereof, preferably selected from residues of 5-fluorouracil (5-FU) and floxuridine; or a derivative thereof, e.g. a residue of 5-fluorouracil (5-FU). In embodiments, the anti-Parkinson's disease drug is ropinirole or a derivative thereof. Suitably, the antibiotic drug residue may be a residue of nitrofurantoin or a derivative thereof. In embodiments, the anti-fungal drug residue is a residue of flucytosine or a derivative thereof. In embodiments, the anti-viral drug residue is selected from the group consisting of residues of aciclovir, iodoxuridine, stavudine, telbivudine, zidovudine, trifluridine and entecavir; or a derivative thereof. In embodiments, the anti-psychotic drug residue is selected from the group consisting of residues of phenobarbital, methylphenobarbital, primidone, lorazepam, nitrazepam, clonazepam, ethotoin, phenytoin, mephenytoin and fosphenytoin; or a derivative thereof. In embodiments, the anti-convulsant drug residue is selected from the group consisting of residues of aripiprazole, sertindole, ziprasidone and mosapramine; or a derivative thereof. In embodiments the heart disease drug residue is selected from the group consisting of residues of amrinone, milrinone, pimobendan, enoximone and cilostazol; or a derivative thereof, preferably selected from residues of amrinone and milrinone; or a derivative thereof. Typically, said drug residues do not include derivatives thereof.

Thus, in embodiments, the group according to formula (II) is a drug residue selected from the group consisting of residues of 5-fluorouracil (5-FU), pemetrexed, olaparib, ropinirole, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine; or a derivative thereof. For example, the drug residue may be selected from the group consisting of residues of 5-fluorouracil (5-FU), pemetrexed, olaparib, ropinirole, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine and entecavir; or a derivative thereof In typical embodiments, the heterocyclic group is a drug residue selected from the group consisting of residues of 5-fluorouracil (5-FU), ropinirole, milrinone, amrinone, floxuridine, flucytosine, aciclovir, iodoxuridine, stavudine, telbivudine, zidovudine, trifluridine and entecavir; or a derivative thereof. In more preferred embodiments, the drug residue is a residue of 5-fluorouracil (5-FU) or a derivative thereof, such as a residue of 5-fluorouracil (5-FU).

The first group according to formula (II) may be a drug residue selected from the following group: 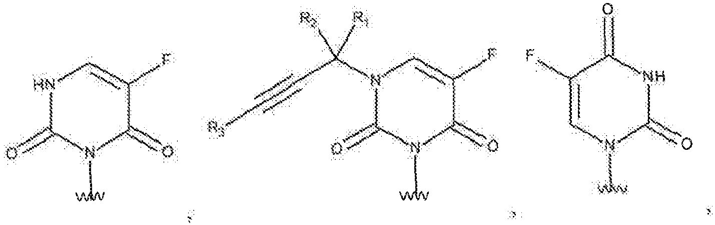 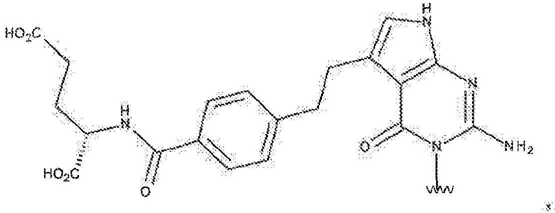 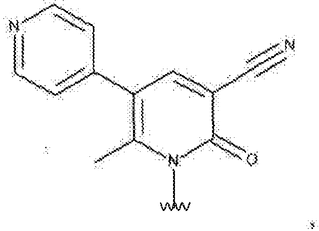 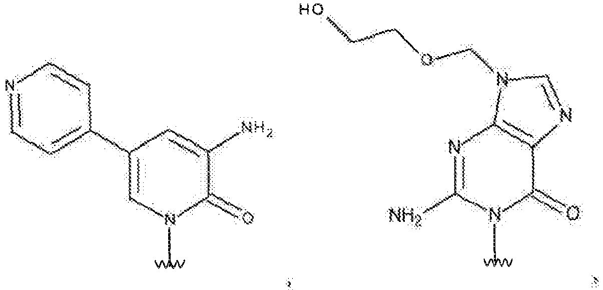 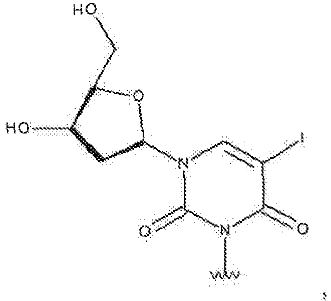 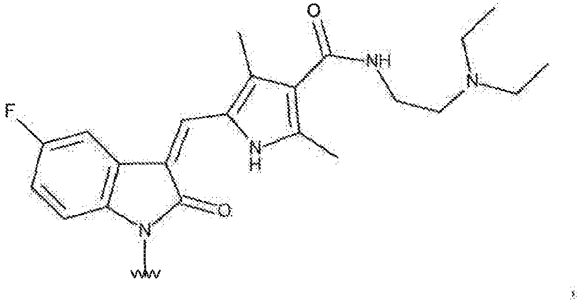 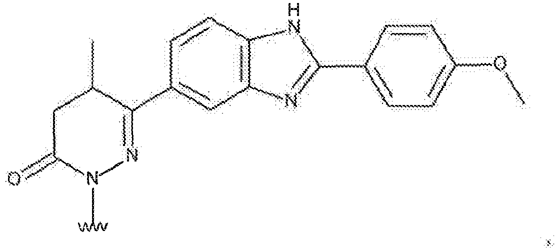 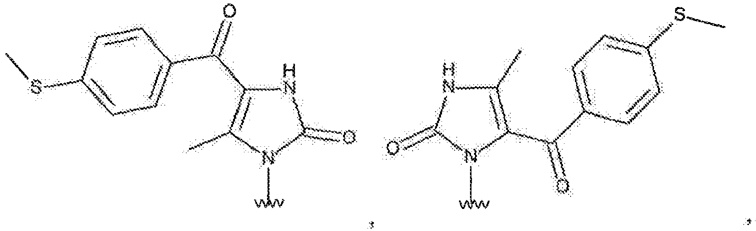 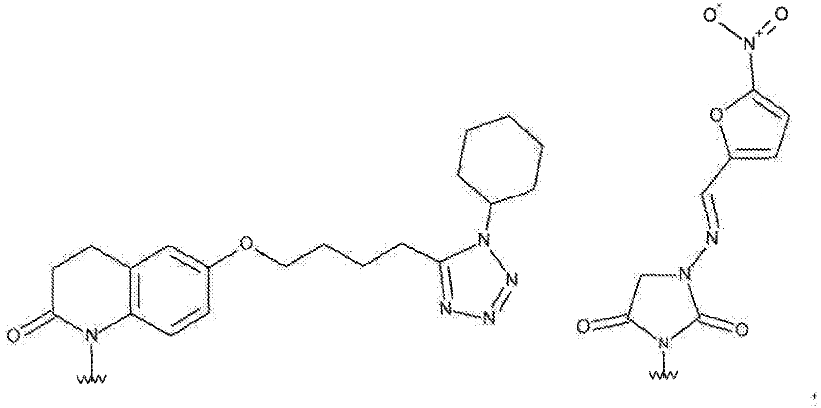 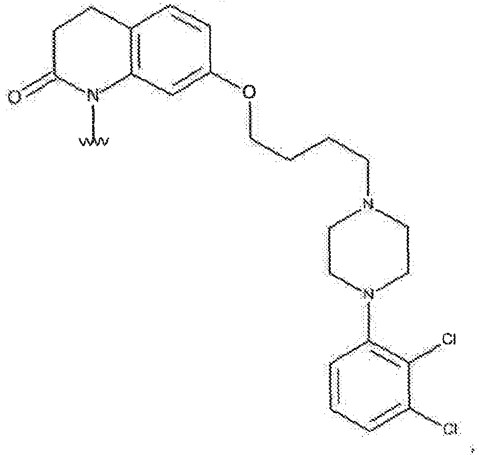 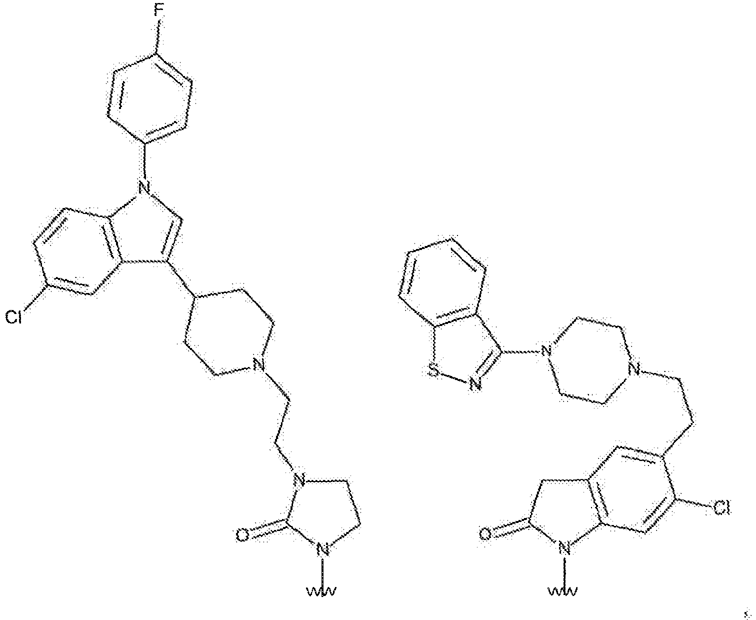 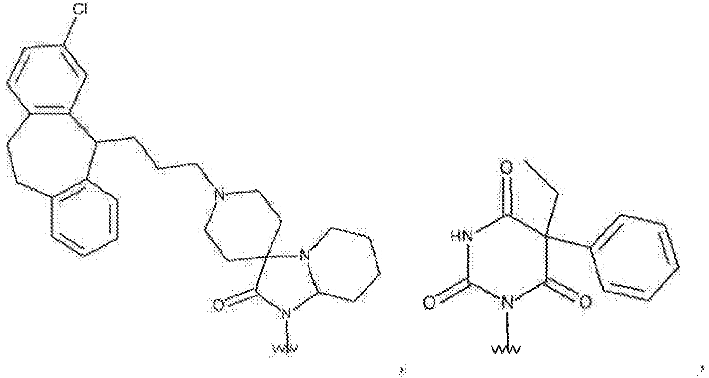 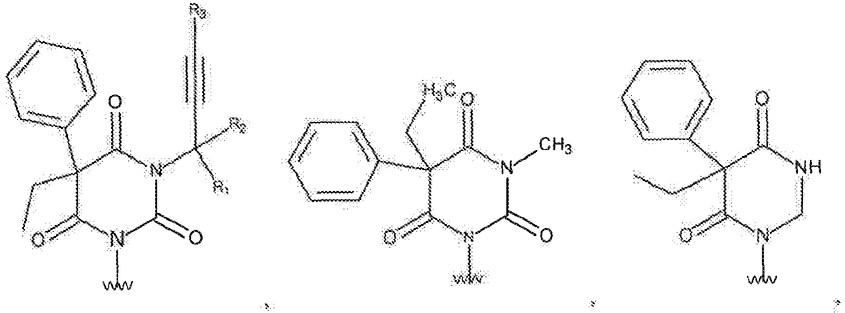 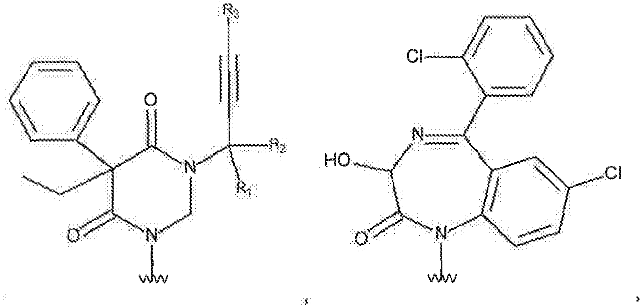 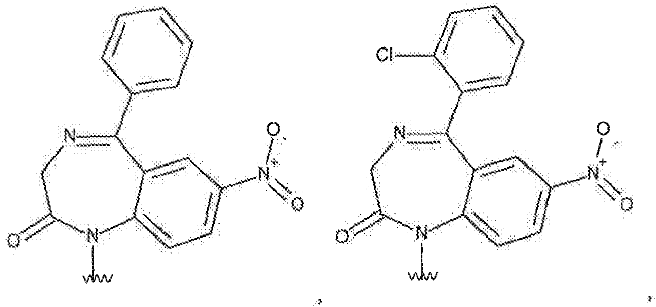 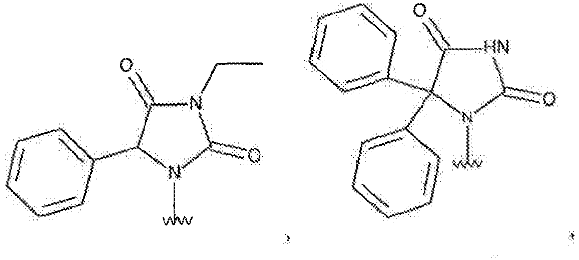 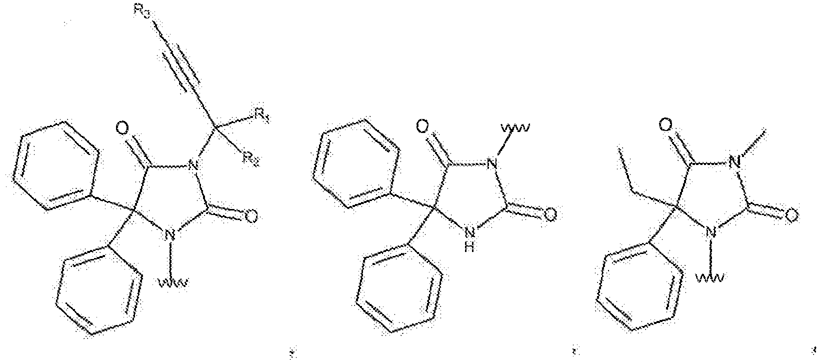 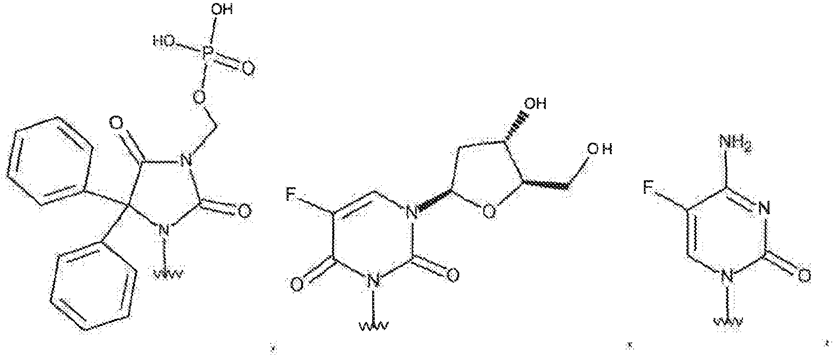 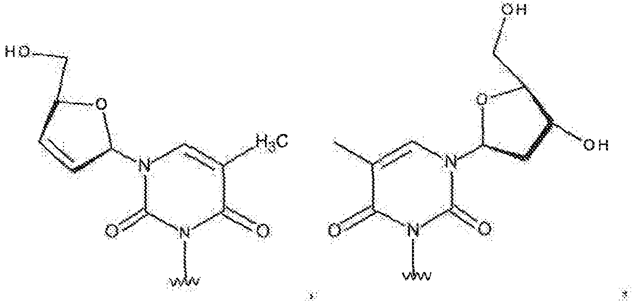 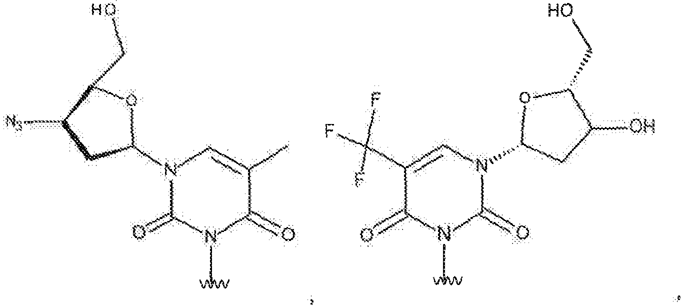 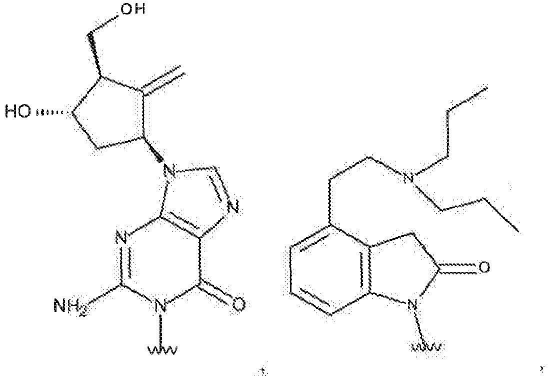 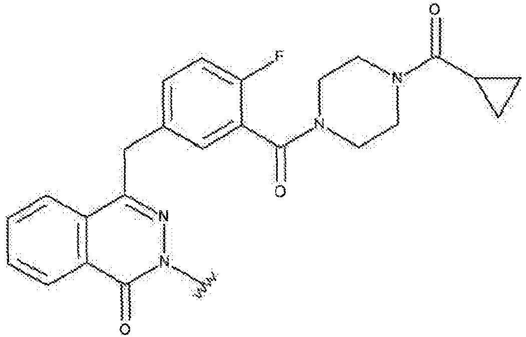 and salts thereof, and optionally, derivatives thereof wherein R₁, R₂ and R₃ are as defined above.

The first group according to formula (II) may be a drug residue selected from the following group: 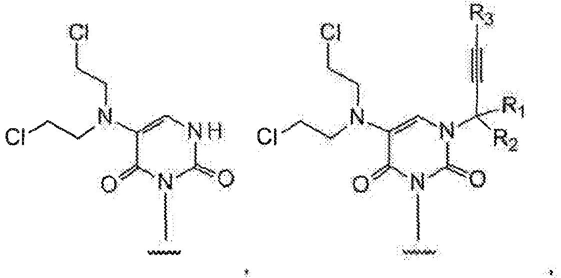 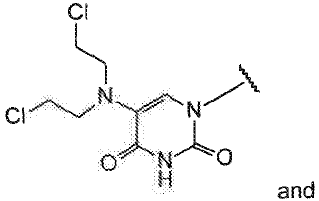 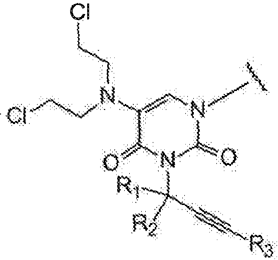 and salts thereof, and optionally, derivatives thereof wherein R₁, R₂ and R₃ are as defined above.

### Group defined according to formula (III)

Suitably, one or more groups defined according to formula (III) may be present if the compound comprises a plurality of endocyclic nitrogen atoms adjacent to one or more ring carbonyls in the first group of formula (II). In embodiments, one, some or all endocyclic nitrogen atoms in the compound adjacent to a ring carbonyl group in the first group of formula (II) may be bonded to groups of formula (III). In embodiments, all endocyclic nitrogen atoms adjacent to a ring carbonyl in group (II) may be bonded to groups of formula (III). Suitably, some (i.e. two or more) endocyclic nitrogen atoms adjacent to a carbonyl group the first group of formula (II) ring may be bonded to groups of formula (III). In preferred embodiments of the compounds of the invention, only one endocyclic nitrogen atom adjacent to a ring carbonyl group in the first group of formula (II) is bonded to a group of formula (III).

For instance, the heterocyclic drug compound 5-fluorouracil contains two available endocyclic nitrogen atoms adjacent to a ring carbonyl group in the heterocyclic group corresponding to formula (II). Thus, as depicted below, either one (a or b), or both (c) of the 5-fluorouracil endocyclic nitrogen atoms may be bonded to groups of formula (III) in the compounds of the invention:

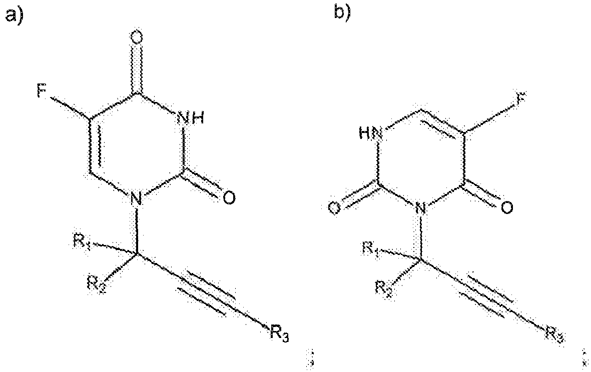

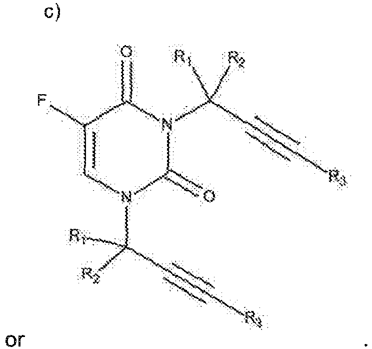

Thus, where more than one group of formula (III) is bonded to a group of formula (II) in the compounds of the invention, the methods of preparing heterocyclic compounds described above may comprise cleavage of more than one of said bonds. Typically, all bonds between the first group of formula (II) and the second group of formula (III) are cleaved in the methods of the invention.

Typically, wherein the first compound comprised a drug residue according to formula (II) bonded to a group according to formula (III), the first compound exhibits an activity of less than 20 times the activity of the corresponding free drug compound, such as less than 30 times, 40 times, 50 times, 60 times, 70 times, 80 times, 90 times, 100 times, 200 times, 300 times, 400 times, 500 times, 600 times, preferably less than 700 times and more preferably less than 800 times the activity of the corresponding free drug compound. Such activity may be inferred by the corresponding IC₅₀ values, such as determined by competitive inhibition assay, or by EC50 values determined by from biological activity assays such as described in the Examples.

### R-group definitions

### R₁, R₂ and R₃

In the compounds and methods of the present invention described above, R₁, R₂ and R₃ are selected independently from the group consisting of H, C₁₋₄alkyl, C₂₋₄alkenyl, and phenyl.

Where more than one group of formula (III) is present in the compounds of the invention as described above, each respective R₁, R₂ and R₃ group is selected independently. Thus, each of the respective R₁ groups, R₂ groups and R₃ groups in any one compound may be the same or different.

In some embodiments, R¹ and R² are both H. In some embodiments, R³ is H.

For example, each group of formula (III) may be selected independently from the group consisting of:

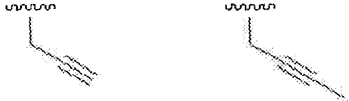

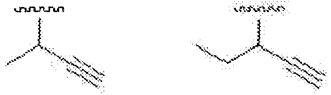

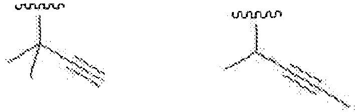

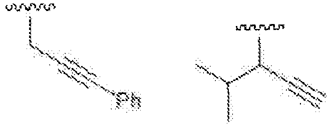

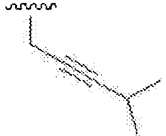

### R₄, R₅, R₆ and R₇

R₄, R₅, R₆ and R₇ are independently selected from the group consisting of H, OH, CN, NO₂, N₃, halo, optionally substituted amino, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted C₂₋₁₀heteroalkynyl, optionally substituted C₆₋₁₄aryl and optionally substituted C₅₋₁₄heteroaryl; optionally wherein any of R₄, R₅, R₆ and R₇ are taken together with an adjacent group and the carbon atoms to which they are attached to form a cyclic group, such as an optionally substituted C₃₋₁₀heterocycloalkyl, optionally substituted C₃₋₁₀heterocycloalkenyl, optionally substituted C₆₋₁₄aryl or optionally substituted C₅₋₁₄heteroaryl group, preferably a phenyl or pyridyl group.

Thus, in embodiments, at least one of R₄, R₅, R₆ and R₇ is taken together with an adjacent group, and the carbon atoms to which they are attached to form a cyclic group. In alternative embodiments, none of R₄, R₅, R₆ and R₇ is taken together with an adjacent group and the carbon atoms to which they are attached to form said cyclic group.

In embodiments, R₄, R₅, R₆ and R₇ are independently selected from the group consisting of H, OH, CN, NO₂, halo, optionally substituted amino, optionally substituted C₁₋₁₀alkyl, optionally substituted C₂₋₁₀alkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted C₂₋₁₀heteroalkynyl, optionally substituted C₆₋₁₄aryl and optionally substituted C₅₋₁₄heteroaryl. Suitably, R₄, R₅, R₆ and R₇ are independently selected from the group consisting of H, OH, CN, NO₂, halo, amino, C₁₋₁₀alkyl, optionally substituted C₂₋₁₀alkynyl, optionally substituted C₆₋₁₄aryl and optionally substituted C₅₋₁₄heteroaryl. Preferably, R₄, R₅, R₆ and R₇ are independently selected from the group consisting of H, halo, amino, C₁₋₁₀alkyl, C₁₋₁₀haloalkyl, optionally substituted C₂₋₁₀alkynyl, and optionally substituted C₃₋₁₀heterocycloalkyl.

Where R₄, R₅, R₆ or R₇ are optionally substituted, the optional substituents may be as defined in the optional substituents section below. In embodiments, the optional R₄, R₅, R₆ / R₇ substituents are selected from halogen, trihalomethyl, trihaloethyl, OH, NH₂, N₃, -NO₂, -CN, -CO₂H, -CO₂C₁₋₆alkyl, -C(=O)H, -C(=O)C₁₋₆alkyl, =O, -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, -N(C₁₋₆alkyl)C(=S)C₁₋₆alkyl, -C₁₋₆alkyl, -C₁₋₆heteroalkyl, -C₃₋₆cycloalkyl, -C₃₋₆heterocycloalkyl, -C₂₋₆alkenyl, -C₂₋₆heteroalkenyl, -C₃₋₆cycloalkenyl, -C₃₋₆heterocycloalkenyl, -C₂₋₆alkynyl, -C₂₋₆heteroalkynyl, -Z^{u}-C₁₋₆alkyl, -Z^{u}-C₃₋₆cycloalkyl, -Z^{u}-C₂₋₆alkenyl, -Z^{u}-C₃₋₆cycloalkenyl and -Z^{u}-C₂₋₆alkynyl, wherein Z^{u} is independently O, S, NH or N(C₁₋₆alkyl). Preferably, the optional R₄, R₅, R₆ / R₇ substituents are selected from halogen, OH, NH₂, -NO₂, -CO₂H, -CO₂C₁₋₆alkyl, =O, -C₂₋₆alkynyl, and -Z^{u}-C₂₋₆alkynyl, wherein Z^{u} is independently O, S, NH or N(C₁₋₆alkyl), such as wherein the optional substituents are selected from OH, N₃ and C₁₋₁₀heteroalkyl.

### R_{6'} and R_{7'}

In the methods and compounds of the present invention, R_{6'} and R_{7'} are each independently selected from the group consisting of H, optionally substituted C₁₋₁₀alkyl, optionally substituted C₂₋₁₀alkenyl, optionally substituted C₂₋₁₀alkynyl, optionally substituted C₂₋₁₀heteroalkyl, optionally substituted C₂₋₁₀heteroalkenyl, and optionally substituted C₂₋₁₀heteroalkynyl. Suitably, each R_{6'} and R_{7'} is independently selected from the group consisting of H, optionally substituted C₁₋₄alkyl, optionally substituted C₂₋₄alkenyl and optionally substituted C₂₋₄alkynyl. Preferably, each R_{6'} and R_{7'} is H.

Where R_{6'} and R_{7'} are optionally substituted, the optional substituents may be as defined in the optional substituents section below. In embodiments, the optional R_{6'} / R_{7'} substituents are selected from halogen, trihalomethyl, trihaloethyl, OH, NH₂, -NO₂, -CN, -CO₂H, -CO₂C₁-₆alkyl, -C(=O)H, -C(=O)C₁₋₆alkyl, =O, -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, -N(C₁₋₆alkyl)C(=S)C₁₋₆alkyl, -C₁₋₆alkyl, -C₁₋₆heteroalkyl, -C₃₋₆cycloalkyl, -C₃₋₆heterocycloalkyl, -C₂₋₆alkenyl, -C₂₋₆heteroalkenyl, -C₃₋₆Cycloalkenyl, -C₃₋₆heterocycloalkenyl, -C₂₋₆alkynyl, -C₂₋₆heteroalkynyl, -Z^{u}-C₁₋₆alkyl, -Z^{u}-C₃₋₆cycloalkyl, -Z^{u}-C₂₋₆alkenyl, -Z^{u}-C₃₋₆cycloalkenyl and -Z^{u}-C₂₋₆alkynyl, wherein Z^{u} is independently O, S, NH or N(C₁₋₆alkyl). Preferably, the optional R_{6'} and R_{7'} substituents are selected from halogen, OH, NH₂, -NO₂, -CO₂H, -CO₂C₁₋₆alkyl, =O, -C₂₋₆alkynyl, and -Z^{u}-C₂₋₆alkynyl, wherein Z^{u} is independently O, S, NH or N(C₁₋₆alkyl).

### R₈

In the methods and compounds of the present invention, R₈ is independently selected from the group consisting of H, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkeny), optionally substituted C₂₋₁₀heteroalkynyl, optionally substituted C₆₋₁₄aryl and optionally substituted C₅₋₁₄heteroaryl. Typically, R₈ is independently selected from the group consisting of H, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl and optionally substituted C₆₋₁₄aryl. In embodiments, each R₈ is independently selected from the group consisting of H, optionally substituted C₁₋₄alkyl, optionally substituted C₂₋₄alkenyl, optionally substituted C₂₋₄alkynyl and optionally substituted phenyl. Preferably, each R₈ is selected from H and a group according to formula (III) as defined above, e.g. H.

Where R⁸ is an optionally substituted group, the optional substituents may be as defined for the optional substituents section below. In embodiments, the optional R₈ substituents are selected from halogen, trihalomethyl, trihaloethyl, OH, NH₂, -NO₂, -CN, -CO₂H, -CO₂C₁₋₆alkyl, -C(=O)H, -C(=O)C₁₋₆alkyl, =O, -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, -N(C₁₋₆alkyl)C(=S)C₁₋₆alkyl, -C₁₋₆alkyl, -C₁₋₆heteroalkyl, -C₃₋₆cycloalkyl, -C₃₋₆heterocycloalkyl, -C₂₋₆alkenyl, -C₂₋₆heteroalkenyl, -C₃₋₆cycloalkenyl, -C₃₋₆heterocycloalkenyl, -C₂₋₆alkynyl, -C₂₋₆heteroalkynyl, -Z^{u}-C₁₋₆alkyl, -Z^{u}-C₃₋₆cycloalkyl, -Z^{u}-C₂₋₆alkenyl, -Z^{u}-C₃₋₆cycloalkenyl and -Z^{u}-C₂₋₆alkynyl, wherein Z^{u} is independently O, S, NH or N(C₁₋₆alkyl). Preferably, the optional R₈ substituents are selected from halogen, OH, NH₂, -NO₂, -CO₂H, -CO₂C₁₋₆alkyl, =O, -C₂₋₆alkynyl, and -Z^{u}-C₂₋₆alkynyl, wherein Z^{u} is independently O, S, NH or N(C₁₋₆alkyl).

### Palladium

Suitable palladium sources for use in the present methods will be known to the skilled person. Pd(0) is particularly preferred for biological applications due to its relative biological inertness (for instance, metallic palladium has shown the safest toxicity profile among all palladium species (Environmental Health Criteria 226: Palladium. World Health Organization, Geneva, 2002), having been widely used in dentistry as part of metal alloys for dental restoration (Rushforth, R. Platinum Metals Rev. 48, 30-31 (2004)) and having remarkable catalytic properties.

As such, for biological applications, e.g. in *in vivo* application, the methods may include a method in which the compound is administered to a patient in which the palladium is present and in a manner that allows contact between the compound and palladium so that the heterocyclic compound or salt thereof as described above is generated in the body. Methods of administration of compounds of the invention and palladium are discussed further below.

For instance, palladium may be provided by any convenient means, e.g. as a fluid solution containing the palladium, or as a colloidal solution containing palladium nanoparticles. Suitable ligand systems for use in forming a fluid solution or for chelating the palladium to a solid phase medium such as a particle / implant will be apparent to the skilled person.

In embodiments, the palladium is conjugated to another molecule. Suitably the palladium may be conjugated to a peptide, polynucleic acid (polynucleotide), or fluorogenic tag, preferably a peptide or polynucleic acid. For instance, the palladium may be conjugated to an antibody or aptamer. For example, by conjugating the palladium to an antibody or aptamer, the palladium may be delivered to a specific target site in the body (by virtue of the specific interaction between target antigen and the antibody or aptamer and target site in the body) ready for performing the bond cleavage reaction according to the method of the present invention.

In preferred embodiments, the palladium is provided in the form of an implant, which may be located at a therapeutically important location in the body, e.g. at, in, adjacent or near a tissue requiring treatment with the therapeutically active form of the drug, such as at, in, adjacent or near a tumour. Advantageously, if the palladium is provided as an extracellular implant, once the relevant condition has been treated (e.g. once a cancer tumour has shrunk to a safe healthy-to-tumoral tissue ratio), the palladium may be safely removed by surgery (e.g. along with any residual tumour in the case of cancer treatment).

Palladium bonded in solid phase may take a number of physical forms. For instance, the palladium may be provided as a palladium implant (i.e. for administration to a patient). Such implants may have a range of physical forms, the intention being that the implant retains the palladium substantially at or near the site of administration/implantation thereby providing a localised concentration of palladium and preventing unwanted high levels of palladium circulating throughout the body. Examples of a palladium implant include a material coated or impregnated by palladium or by a palladium containing compound, such as a palladium-containing alloy. The material may be a solid (e.g. a porous solid) or semi-solid, e.g. a gel, and may be in the form of a bolus. The implant should allow for contact of prodrug present in the tissue or associated vasculature with the palladium present in the implant.

The implant material may be selected to allow the coated or impregnated palladium to be released from the material when administered to or implanted in the subject. Release kinetics may be altered by altering the structure, e.g. porosity, of the material.

The material provides a scaffold or matrix support for the palladium. The material may be suitable for implantation in tissue, or may be suitable for administration to the body (e.g. as microcapsules in solution).

Preferably, the implant material should be biocompatible, e.g. non-toxic and of low immunogenicity (most preferably non-immunogenic). The biomaterial may be biodegradable such that the biomaterial degrades over time. Alternatively a non-biodegradable biomaterial may be used, allowing surgical removal of the implant as required.

Suitable materials may be soft and/or flexible, e.g. hydrogels, fibrin web or mesh, wafers or collagen sponges. A "hydrogel" is a substance formed when an organic polymer, which can be natural or synthetic, is set or solidified to create a three-dimensional open-lattice structure that entraps molecules of water or other solutions to form a gel. Solidification can occur by aggregation, coagulation, hydrophobic interactions or cross-linking.

Alternatively suitable materials may be relatively rigid structures, e.g. formed from solid materials such as plastics, resins or biologically inert metals such as titanium.

The implant material may have a porous matrix structure which may be provided by a cross-linked polymer.

Matrix structures may be formed by crosslinking fibres, e.g. fibrin or collagen, or of liquid films of sodium alginate, chitosan, or other polysaccharides with suitable crosslinkers, e.g. calcium salts, polyacrylic acid, heparin. Alternatively scaffolds may be formed as a gel, fabricated by collagen or alginates, crosslinked using well established methods known to those skilled in the art.

Suitable polymer materials for matrix formation include, but are not limited by, biodegradable/bioresorbable polymers which may be chosen from the group of: agarose, collagen, fibrin, chitosan, polycaprolactone, poly(DL-lactide-co-caprolactone), poly(L-lactide-co-caprolactone-co-glycolide), polyglycolide, polylactide, polyhydroxyalcanoates, co-polymers thereof; or non-biodegradable polymers which may be chosen from the group of: polystyrene, polyethylene glycol, cellulose acetate; cellulose butyrate, alginate, polysulfone, polyurethane, polyacrylonitrile, sulfonated polysulfone, polyamide, polyacrylonitrile, polymethylmethacrylate, co-polymers thereof. Preferably the non-biodegradable polymer is polystyrene, polyethylene glycol, or a polystyrene-polyethylene glycol copolymer.

Collagen is a promising material for matrix construction owing to its biocompatibility and favourable property of supporting cell attachment and function (U.S. Pat. No. 5,019,087; Tanaka, S.; Takigawa, T.; Ichihara, S. & Nakamura, T. Mechanical properties of the bioabsorbable polyglycolic acid-collagen nerve guide tube Polymer Engineering & Science 2006, 46, 1461-1467). Clinically acceptable collagen sponges are one example of a matrix and are well known in the art (e.g. from Integra Life Sciences). Fibrin scaffolds (e.g. fibrin glue) provide an alternative matrix material. Fibrin glue enjoys widespread clinical application as a wound sealant, a reservoir to deliver growth factors and as an aid in the placement and securing of biological implants (Rajesh Vasita, Dhirendra S Katti. Growth factor delivery systems for tissue engineering: a materials perspective. Expert Reviews in Medical Devices. 2006; 3(1): 29-47; Wong C, Inman E, Spaethe R, Helgerson S. Thromb.Haemost. 2003 89(3): 573-582; Pandit AS, Wilson DJ, Feldman DS. Fibrin scaffold as an effective vehicle for the delivery of acidic growth factor (FGF-1). J. Biomaterials Applications. 2000; 14(3); 229-242; DeBlois Cote MF. Doillon CJ. Heparin-fibroblast growth factor fibrin complex: in vitro and in vivo applications to collagen based materials. Biomaterials. 1994; 15(9): 665-672.).

Other suitable materials include ceramic or metal (e.g. titanium), hydroxyapatite, tricalcium phosphate, demineralised bone matrix (DBM), autografts (i.e. grafts derived from the patient's tissue), or allografts (grafts derived from the tissue of an animal that is not the patient). Implant materials may be synthetic (e.g. metal, fibrin, ceramic) or biological (e.g. carrier materials made from animal tissue, e.g. non-human mammals (e.g. cow, pig), or human).

One form of commercially available palladium implant is a palladium seed implant, such as the TheraSeed™ (Theragenics Corporation, Buford, Georgia, USA), which is used as a brachytherapy biocompatible device but could be adapted to the purpose of this invention (using it in a non-radioactive form).

In a preferred embodiment, the polymer material is polyethylene glycol (PEG)-polystyrene graft co-polymer in which the PEG chains have been terminally functionalized with an amino group (e.g. NovaSyn® TG amino resin). This polymer has been previously functionalized with Pd⁰ nanoparticles by: (i) mixing with Pd(OAc)₂, (ii) in situ reduction to Pd⁰ and (iii) intensive cross-linking of the polymer surface with activated diacyl compounds to physically trap the Pd⁰ nanoparticles in the polymer (Bradley, et al. J. Am. Chem. Soc. 128, 6276-6277 (2006)). The Pd⁰ functionalized polymer demonstrated high catalytic activity in water and remarkable reusability properties (over 10 catalytic cycles without reducing performance).

In embodiments, the palladium may include palladium nanoparticles, such as described in Nature Protocols, 7, 1207-1218 (2012), Pd⁰-functionalized polystyrene microspheres, such as described in Bradley, M. et al., Nat. Chem. 2011, 3, 239-243,, Pd⁰-functionalized polyethylene glycol polyacrylamide copolymer (PEGA) resins, and PEG-polystyrene graft co-polymer in which the PEG chains have been terminally functionalized with an amino group (e.g. NovaSyn® TG amino resin, which is a 3000-4000 M.W.) such as described in Bradley, et al. J. Am. Chem. Soc. 128, 6276-6277 (2006). Preferably, the palladium is provided as a palladium functionalized PEG-polystyrene composite resin.

### Compounds of the present invention

### Second aspect

In a second aspect of the invention is provided a compound or salt thereof comprising a group defined according to formula (III) bonded at the position indicated by an asterisk to an endocyclic nitrogen atom of a heterocyclic compound or salt thereof, wherein the endocyclic nitrogen is adjacent to a ring carbonyl group 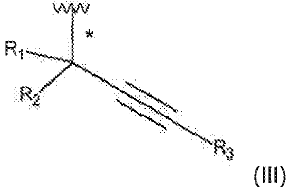 wherein
R₁, R₂ and R₃ are selected independently from the group consisting of H, C₁₋₄alkyl, C₂₋₄alkenyl, and phenyl,
wherein the heterocyclic compound or salt thereof is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine.

### Heterocyclic compound or salt thereof

As described in the above aspect, the group of formula (III) is bonded to a ring atom of a heterocyclic compound or salt thereof. In this context, the bond to the heterocyclic compound or salt thus forms a notional heterocyclic radical bonded to a notional radical of formula (III). For instance, it is intended that the group of formula (III) may notionally (i.e. hypothetically) replace (i.e. substitute) the ring N-H bond of the heterocyclic compound. The term "notional replacement" is not intended to require that the compound or salt thereof according to the above aspect has been synthetically prepared by replacement of a ring N-H bond with bond between the endocyclic nitrogen and the group of formula (III), i.e. the compound may be prepared in any suitable way.

The heterocyclic compound or salt thereof according to the above aspect may suitably be defined as above for the corresponding heterocyclic compounds or salts thereof described in the first aspect and embodiments of the methods of the invention. Accordingly, where the heterocyclic compound or salt thereof is a drug compound, the compound or salt of the invention is suitably a prodrug.

### Group defined according to formula (III)

In the above aspect, the group according to formula (III) as well as the corresponding R-groups R₁-R₃ may be defined as for the corresponding groups in any one of the aspect and embodiments described above, e.g. in relation to the methods of the invention.

Typically, wherein the compound of the invention comprises a group according to formula (III) bonded at the position indicated by an asterisk to an endocyclic nitrogen atom of a drug compound or salt thereof wherein the endocyclic nitrogen is adjacent to a ring carbonyl group, the compound of the invention exhibits an activity of less than 20 times the activity of the corresponding free drug compound, such as less than 30 times, 40 times, 50 times, 60 times, 70 times, 80 times, 90 times, 100 times, 200 times, 300 times, 400 times, 500 times, 600 times, preferably less than 700 times and more preferably less than 800 times the activity of the corresponding free drug compound. Such activity may be inferred by the corresponding IC₅₀ values, such as determined by competitive inhibition assay, or by EC50 values determined by biological activity assays such as described in the Examples.

### Third aspect

In a third aspect, the invention provides a compound or salt thereof comprising a first group defined according to formula (II) 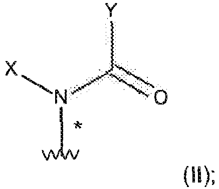 bonded to a second group defined according to formula (III) at the positions indicated by asterisks 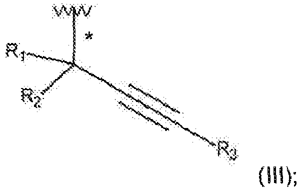 wherein
X and Y taken together with the endocyclic nitrogen atom and ring carbonyl group to which they are attached form a heterocyclic group;
R₁, R₂ and R₃ are selected independently from the group consisting of H, C₁₋₄alkyl, C₂₋₄alkenyl, and phenyl,
wherein the heterocyclic compound or salt thereof is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine.

As described above for the methods of the invention, suitably the bond between the heterocyclic compound or salt thereof according to the second aspect above, or the group defined according to formula (II) according to the third aspect; and the group as defined according to formula (III) is cleavable upon treatment with palladium. For example, as described in the examples, cleavage of the group of formula (III) proceeded to completion in 24 h when a compound of the invention (500 µM) was dissolved in phosphate buffered saline ("PBS") (0.5 ml) with 0.5 mg of Pd⁰ resin and shaken at 1400 rpm and 37 °C in a Thermomixer. In embodiments, compounds of the invention are provided wherein the cleavage of the group of formula (III) proceeds to at least 50% completion by 72 h according to the above reaction conditions, such as at least 60%, 70%, 80%, 90%, 95% and preferably at least 98% completion by 72 h, preferably by 48 h, more preferably by 24 h.

Furthermore, the covalent bond between either the heterocyclic compound or salt thereof according to the second aspect and embodiments above, or the group defined according to formula (II) according to the third aspect; and the group as defined according to formula (III) is not readily cleaved by natural metabolic pathways and thus the present compounds of the invention are useful substrates for bioorthogonal reaction processes.

Consequently, such compounds thus provide useful candidates as prodrugs or biomolecular probes (for instance for the detection of palladium in cells).

The Examples show that the compounds of the invention can be deprotected in a controlled and efficient manner in ambient biological conditions using a biocompatible palladium catalyst to generate free active drug *in situ* that exhibits the desired biological activity. Moreover, the data show that compounds of the invention are suitably non-toxic and do not interfere with the active drug pathway, thus providing ideal drug precursors. The examples also show that the reaction is catalytic and is understood to proceed largely heterogeneously. The palladium is therefore not used up in the process and a potentially unlimited number of dosage cycles may therefore be repeated using only a single palladium implant.

Suitably, the by-product produced in the cleavage of the second group of formula (III) is biocompatible. For instance, cleavage of a propargyl group as illustrated in the examples provides 1-hydroxyacetone (also known as acetol) as the by-product (see Figure 12), which is a known lipid metabolite.

The precise spatial control of prodrug deprotection provided by palladium implants, along with lack of toxicity of the prodrug compounds means that prodrugs of the invention can be deprotected specifically at the disease site, which should thus reduce general systemic concentration of the free drug. This is especially desirable in cancer treatments where side-effects resulting from the drug acting non-specifically on other organs in the body can be severe. This may also in turn allow prodrugs of the invention to be administered in higher doses, providing higher concentrations of drug at the disease site than would have been tolerated through general systemic administration of the active drug due to risk of the side-effects mentioned above.

### The group according to formula (II)

In the third aspect and embodiments thereof described above, the group according to formula (II), including the respective R₄-R₈ groups, etc may be defined according to any corresponding definition above for the first aspect and embodiments (in relation to the methods of the invention). Thus, the group according to formula (II) may be a drug residue of formula (II), *etc.*

### The group according to formula (III)

In the third aspect and embodiments thereof described above, the group according to formula (III), as well as the corresponding R-groups R₁-R₃ may be defined according to any corresponding definition above for the first aspect and embodiments (in relation to the methods of the invention).

Typically, wherein compound comprises a group according to formula (II) bonded to a group according to formula (III) at the position indicated by an asterisk wherein the group according to formula (II) is a drug residue, the compound of the invention exhibits an activity of less than 20 times the activity of the corresponding free drug compound, such as less than 30 times, 40 times, 50 times, 60 times, 70 times, 80 times, 90 times, 100 times, 200 times, 300 times, 400 times, 500 times, 600 times, preferably less than 700 times, more preferably less than 800 times the activity of the corresponding free drug compound. Such activity may be inferred by the corresponding IC₅₀ values, such as determined by competitive inhibition assay, or by EC50 values determined by biological activity assays such as described in the Examples.

### Further compounds of the invention

In a fourth aspect, the invention provides a compound selected from the group consisting of: 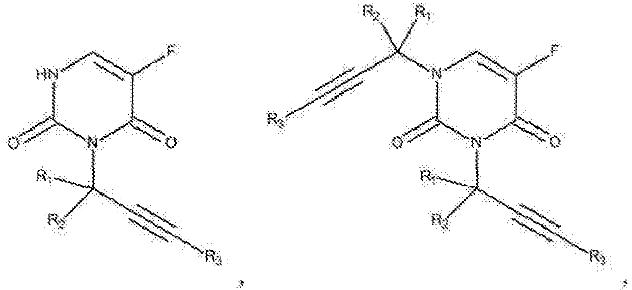 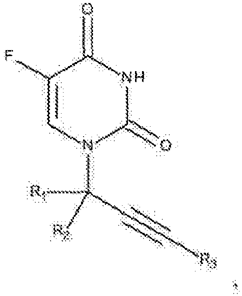 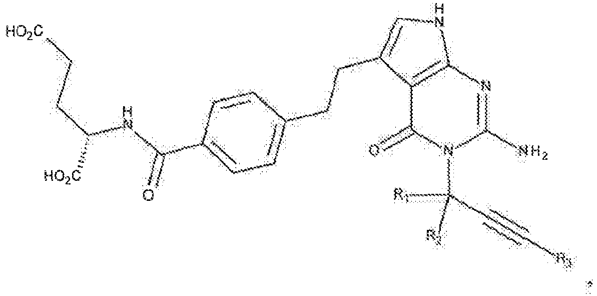 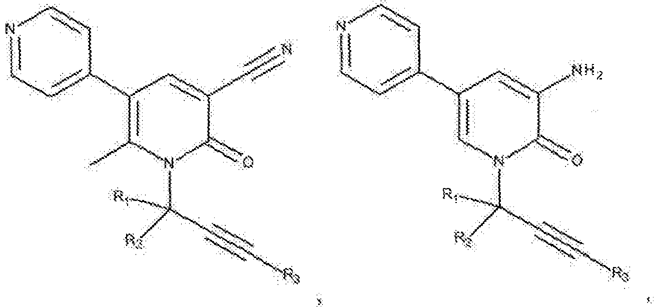 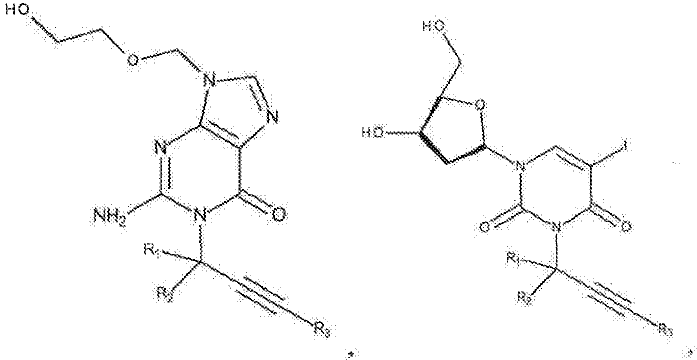 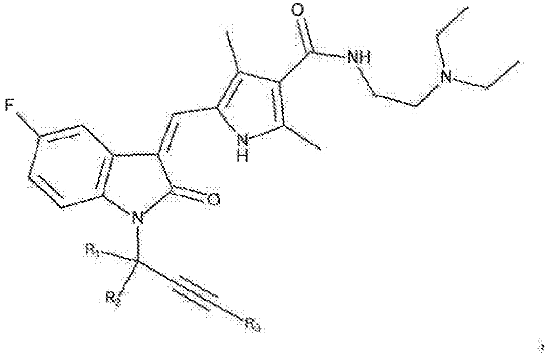 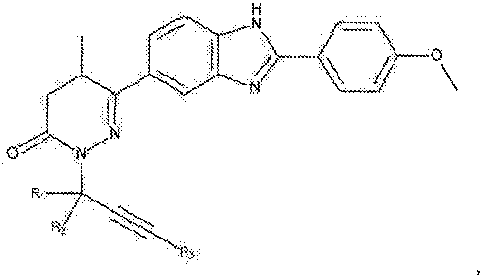 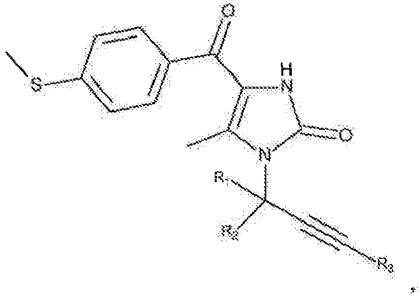 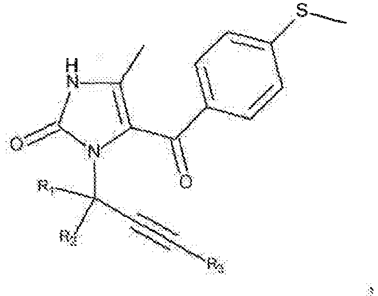 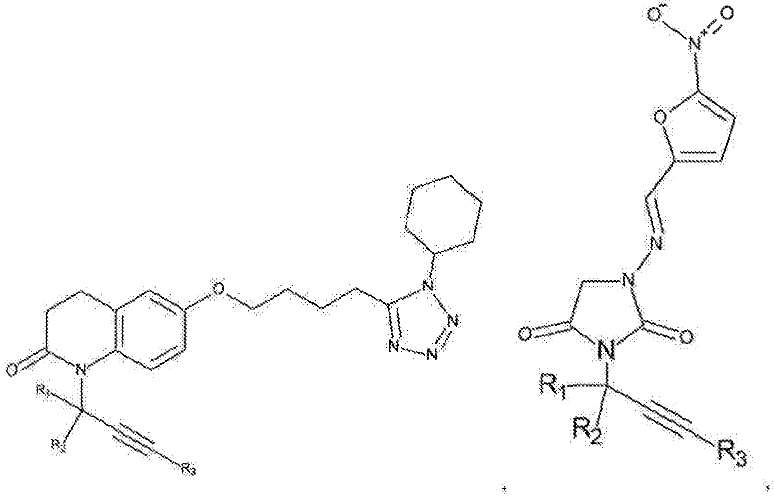 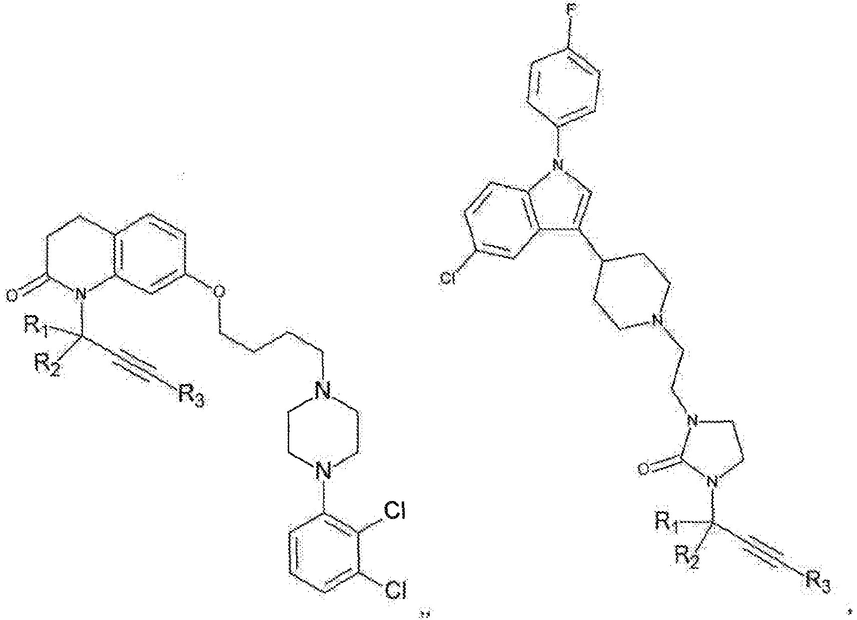 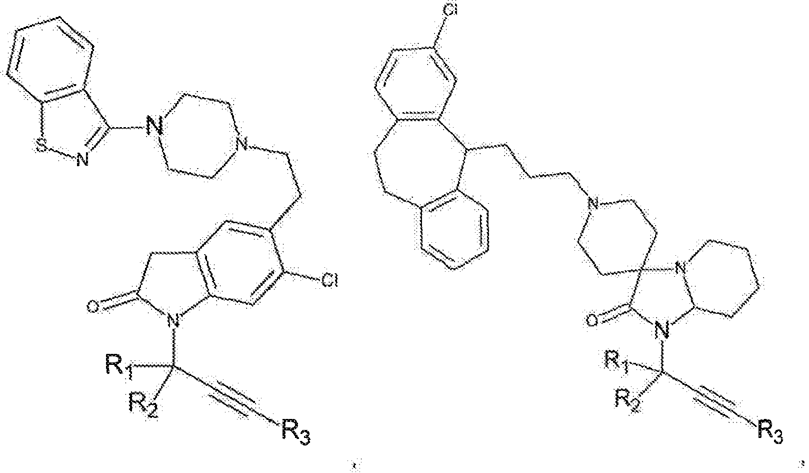 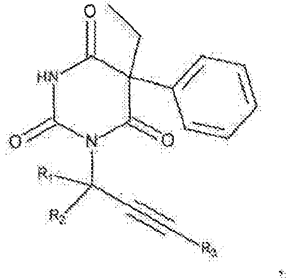 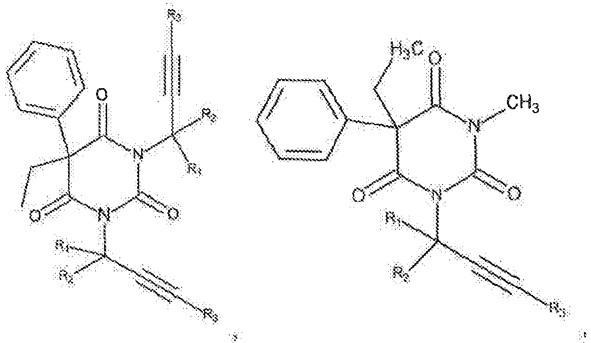 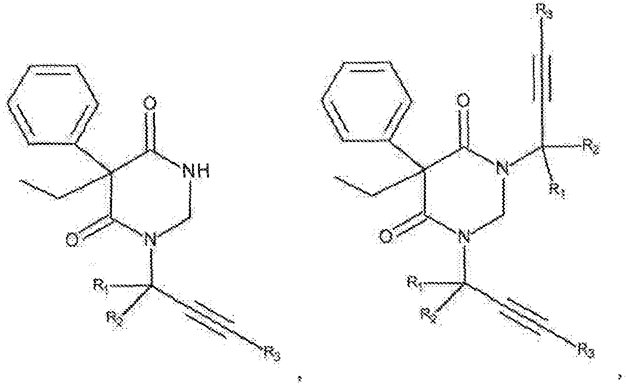 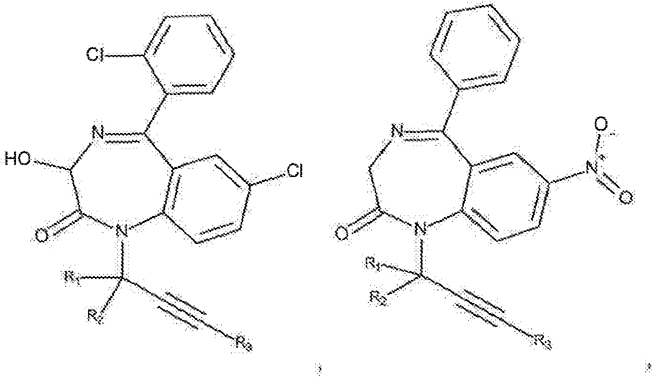 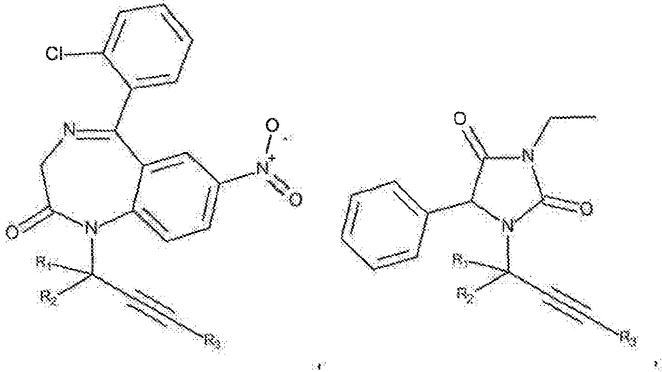 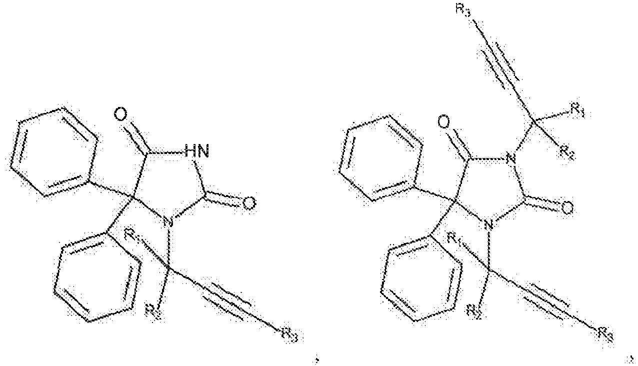 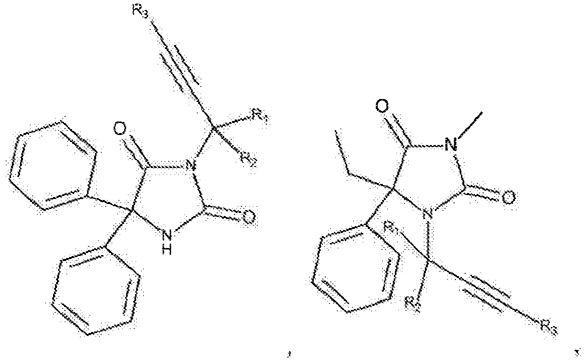 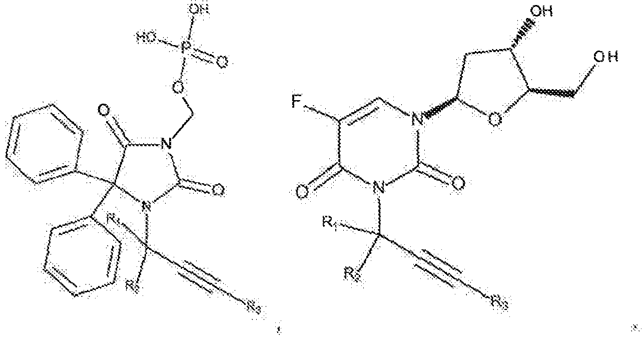 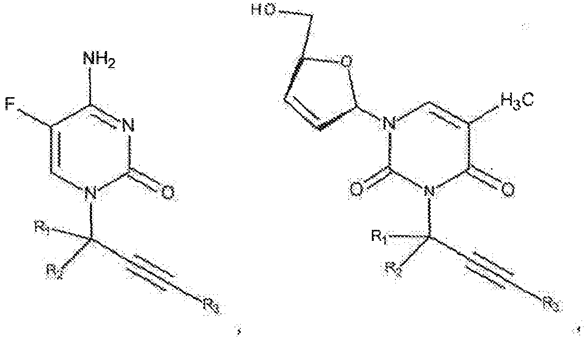 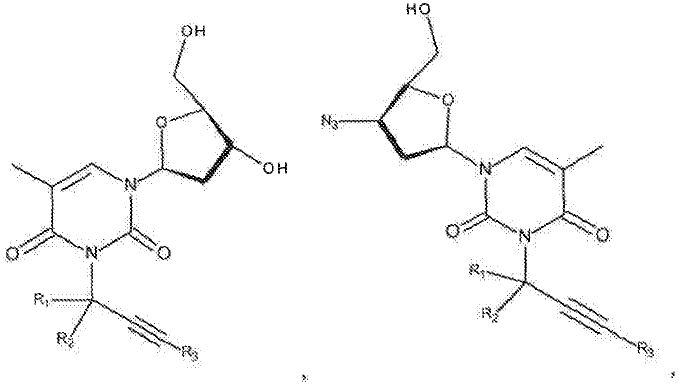 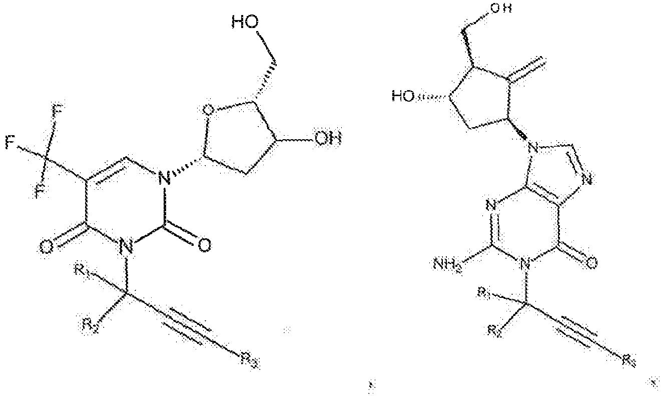 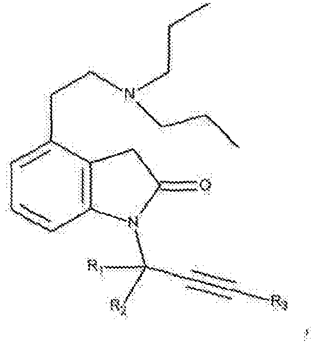 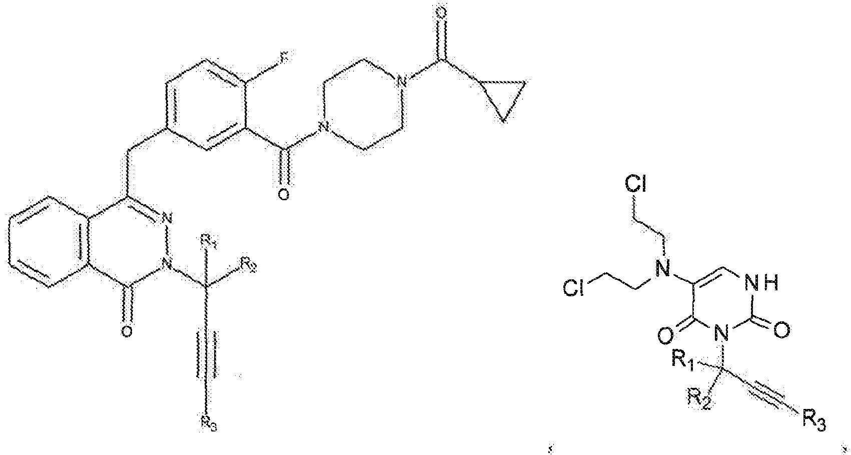 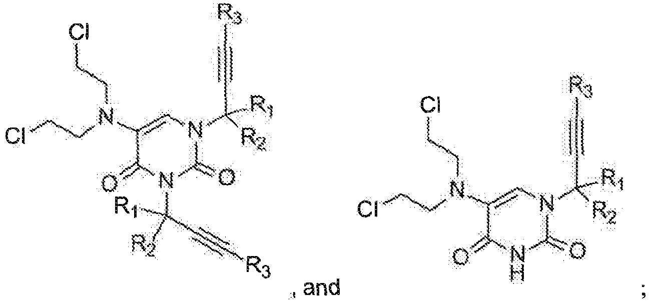 or salt thereof, wherein R₁-R₃ are as defined according to any aspect or embodiment above.

Preferably the compounds or salts of the invention described in the above aspects and embodiments may be provided in isolated form, typically in solid form. Suitably, the isolated compound or salt may be produced in substantially pure form. In embodiments, "substantially pure" means that at least 80mol% of the total content of a sample of the compound of the invention consists of a compound of the invention, such as at least 85 mol%, 90 mol%, 91 mol%, 92 mol%, 93 mol%, 94 mol%, 95 mol%, 96 mol%, 97 mol%, 98 mol% or more preferably at least 99mol%. In preferred embodiments, "substantially pure" means that at least 80wt% of the total content of a sample of the compound of the invention consists of a compound of the invention, such as at least 85 wt%, 90 wt%, 91 wt%, 92 wt%, 93 wt%, 94 wt%, 95 wt%, 96 wt%, 97 wt%, 98 wt% or more preferably at least 99 wt%.

In embodiments, the isolated compound or salt is in solid form. Preferably, the isolated solid form comprises at least 80 mol% of a compound or salt of the invention; more preferably 85 mol%, 90 mol%, 91 mol% 92 mol%, 93 mol%, 94 mol%, 95 mol%, 96 mol%, 97 mol%, 98 mol% or most preferably at least 99 mol%.

### Further aspects

A further aspect disclosed herein is a method of preparing a compound of formula (V) comprising: 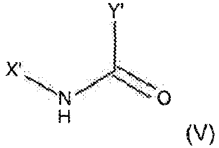
a) providing a compound comprising a first group as defined according to formula (VI) 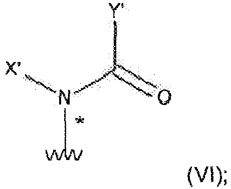 bonded to a second group as defined according to formula (III) at the position marked by asterisks 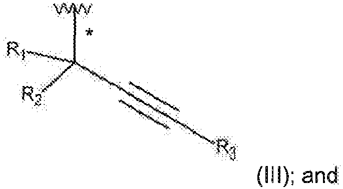
b) cleaving the bond between the first and second groups by reacting the first compound with palladium,
   wherein
   X' and Y' are independently H or a carbon-containing group; or
   X' and Y' taken together with the nitrogen atom and carbonyl group to which they are attached form a heterocyclic group; and
   R₁, R₂ and R₃ are selected independently from the group consisting of H, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted C₆₋₁₄aryl and optionally substituted C₅₋₁₄heteroaryl.

Accordingly, the above method provides a useful amide protecting group strategy which offers a useful tool for a chemist seeking alternative orthogonal amide protecting groups. The deprotection is extremely atom efficient and can be effected by palladium in the presence of, e.g. benzyl groups (see examples).

Suitably, when X' and Y' are independently H or a carbon-containing group, X' and Y' may be independently defined according to R₁-R₃ above according to any embodiment above. When X' and Y' are taken together with the nitrogen and carbonyl group to which they are attached to form an optionally substituted heterocyclic group, said group may be defined according to any definition above for the group according to formula (II).

The compounds of the invention may be manufactured by any suitable method that would be apparent to a skilled person, such as detailed in the examples. For instance, in a further aspect, the present invention provides a process for preparing a compound according any of the second, third, fourth or further aspects and embodiments above, comprising reacting a heterocyclic compound or salt thereof as defined in any of the first aspect and embodiments thereof (e.g. wherein the heterocyclic compound is a drug compound) with a compound of formula (IIIa): 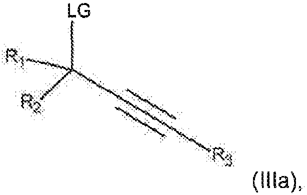 wherein LG is a leaving group and R₁-R₃ are as defined according to any aspect or embodiment above.

For instance, the heterocyclic compound (e.g. drug compound) or salt thereof may be a compound of formula (I): 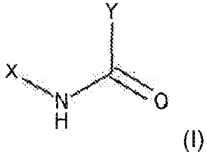 or salt thereof, wherein X and Y are as defined according to any aspect and embodiment above.

Thus, compounds of the invention may be formed by nucleophilic substitution of a leaving group from the group of formula (IIIa) by an endocyclic nitrogen adjacent to a ring carbonyl group. Suitable solvents and conditions will be apparent to the skilled person and exemplary syntheses are provided in the examples. Accordingly, the LG group may be any leaving group that can be displaced by the endocyclic nitrogen. Preferably, the LG group is a halo group, more preferably Br.

### Further embodiments of compounds and methods of the invention

### General

Various embodiments of the compounds of the invention are described in this application. The skilled person will recognise that features specified in each of these embodiments may be combined with other features specified in other aspects and embodiments to provide further embodiments of the invention.

As described above, the above methods disclosed herein involve cleavage of the covalent bond between a first group defined according to formula (II) and a second group according to formula (III) using palladium. As illustrated in Figure 4, when palladium(0) is used to cleave the covalent bond, the bond cleavage results in formation of a heterocyclic compound and a hydroxyketone by-product (e.g. 1-hydroxyketone (acetol) in the case of the process exemplified in Figure 4). Thus, the methods described above in the first aspect are also methods for preparing a hydroxyketone of formula (VII): 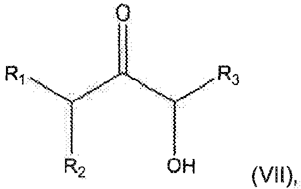 wherein R₁-R₃ are as defined above.

In other words, disclosed herein is also a method of preparing a hydroxyketone according to formula (VII) comprising 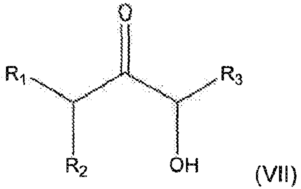
a) providing a compound comprising a first group as defined according to formula (VI) 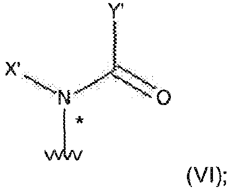 bonded to a second group as defined according to formula (III) at the position marked by asterisks 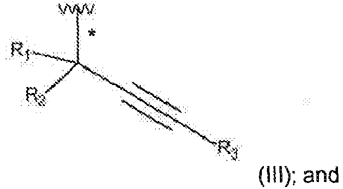
b) cleaving the bond between the first and second groups by reacting the first compound with palladium (0),
   wherein
   X' and Y' are independently H or a carbon-containing group; or
   X' and Y' taken together with the nitrogen atom and carbonyl group to which they are attached form a heterocyclic group; and
   R₁, R₂ and R₃ are selected independently from the group consisting of H, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted C₆₋₁₄aryl and optionally substituted C₅₋₁₄heteroaryl.

Suitably, the method of preparing a hydroxyketone according to formula (VII) may thus comprise:
a) providing a first compound comprising a first group defined according to formula (II): 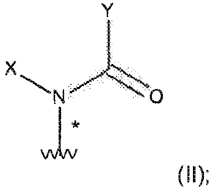 bonded to a second group defined according to formula (III) at the positions indicated by asterisks 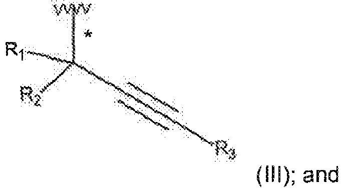
b) cleaving the bond between the first and second groups by reacting the first compound with palladium
   wherein
   X and Y taken together with the endocyclic nitrogen atom and ring carbonyl group to which they are attached form a heterocyclic group; and
   R₁, R₂ and R₃ are selected independently from the group consisting of H, optionally substituted C₁₋₁₀alkyl, optionally substituted C₃₋₁₀cycloalkyl, optionally substituted C₂₋₁₀alkenyl, optionally substituted C₃₋₁₀cycloalkenyl, optionally substituted C₂₋₁₀alkynyl, optionally substituted C₂₋₁₀heteroalkyl, optionally substituted C₃₋₁₀heterocycloalkyl, optionally substituted C₂₋₁₀heteroalkenyl, optionally substituted C₃₋₁₀heterocycloalkenyl, optionally substituted C₂₋₁₀heteroalkynyl, optionally substituted C₆₋₁₄aryl and optionally substituted C₅₋₁₄heteroaryl.

### Chemical Groups

### Halo

The term "halogen" (or "halo") includes fluorine, chlorine, bromine and iodine.

*Alkyl, alkylene, alkenyl, alkynyl, cycloalkyl etc.*

The terms "alkyl", "alkylene", "alkenyl" or "alkynyl" are used herein to refer to both straight and branched chain acyclic forms. Cyclic analogues thereof are referred to as cycloalkyl, etc.

The term "alkyl" includes monovalent, straight or branched, saturated, acyclic hydrocarbyl groups. In one embodiment alkyl is C₁₋₁₀alkyl, in another embodiment C₁₋₆alkyl, in another embodiment C₁₋₄alkyl, such as methyl, ethyl, n-propyl, i-propyl or t-butyl groups.

The term "cycloalkyl" includes monovalent, saturated, cyclic hydrocarbyl groups. In some embodiments the cycloalkyl is C₃₋₁₀cycloalkyl, in other embodiments C₃₋₆cycloalkyl, such as cyclopentyl and cyclohexyl.

The term "alkoxy" means alkyl-O-.

The term "alkylamino" means alkyl-NH-.

The term "alkylthio" means alkyl-S(O)ₜ-, wherein t is defined below.

The term "alkenyl" includes monovalent, straight or branched, unsaturated, acyclic hydrocarbyl groups having at least one carbon-carbon double bond and, in one embodiment, no carbon-carbon triple bonds. In one embodiment alkenyl is C₂₋₁₀alkenyl, in another embodiment C₂₋₆alkenyl, in another embodiment C₂₋₄alkenyl.

The term "cycloalkenyl" includes monovalent, partially unsaturated, cyclic hydrocarbyl groups having at least one carbon-carbon double bond and, in one embodiment, no carbon-carbon triple bonds. In one embodiment cycloalkenyl is C₃₋₁₀cycloalkenyl, in another embodiment C₅₋₁₀cycloalkenyl, *e.g.* cyclohexenyl or benzocyclohexyl.

The term "alkynyl" includes monovalent, straight or branched, unsaturated, acyclic hydrocarbyl groups having at least one carbon-carbon triple bond and, in one embodiment, no carbon-carbon double bonds. In one embodiment, alkynyl is C₂₋₁₀alkynyl, in another embodiment C₂₋₆alkynyl, in another embodiment C₂₋₄alkynyl.

The term "alkylene" includes divalent, straight or branched, saturated, acyclic hydrocarbyl groups. In one embodiment alkylene is C₁₋₁₀alkylene, in another embodiment C₁₋₆alkylene, in another embodiment C₁₋₄alkylene, such as methylene, ethylene, n-propylene, i-propylene or t-butylene groups.

The term "alkenylene" includes divalent, straight or branched, unsaturated, acyclic hydrocarbyl groups having at least one carbon-carbon double bond and, in some embodiments, no carbon-carbon triple bonds. In some embodiments alkenylene is C₂₋₁₀alkenylene, in other embodiments C₂₋₆alkenylene, such as C₂₋₄alkenylene.

The term "cyclic group" includes carbocyclic and heterocyclic groups, such as cycloalkyl, cycloalkenyl, aryl, heterocycloalkyl, heterocycloalkenyl and heteroaryl groups as defined below.

### Heterocyclic compound

The term "heterocyclic compound" refers to a compound comprising a heterocyclic group.

The term "heterocyclic group" refers to group a saturated, partially unsaturated or unsaturated (e.g. aromatic) monocyclic or bicyclic group containing one or more (for example 1, 2, 3, 4 or 5) ring heteroatoms selected from O, S(O)ₜ or N and includes unsubstituted groups and groups substituted with one or more substituents (for example 1, 2, 3, 4 or 5 substituents), optionally wherein the one or more substituents are taken together to form a further ring system. Unless stated otherwise herein, where a heterocyclic group is bonded to another group, the heterocyclic group may be C-linked or N-linked, *i.e.* it may be linked to the remainder of the molecule through a ring carbon atom or through a ring nitrogen atom (i.e. an endocyclic nitrogen atom). The term heterocyclic group thus includes optionally substituted heterocycloalkyl, heterocycloalkenyl and heteroaryl groups as defined below.

### Heteroalkyl etc.

The term "heteroalkyl" includes alkyl groups in which up to three carbon atoms, in one embodiment up to two carbon atoms, in another embodiment one carbon atom, are each replaced independently by O, S(O)ₜ or N, provided at least one of the alkyl carbon atoms remains. The heteroalkyl group may be C-linked or hetero-linked, *i.e.* it may be linked to the remainder of the molecule through a carbon atom or through O, S(O)ₜ or N, wherein t is defined below.

The term "heterocycloalkyl" includes cycloalkyl groups in which up to three carbon atoms, in one embodiment up to two carbon atoms, in another embodiment one carbon atom, are each replaced independently by O, S(O)ₜ or N, provided at least one of the cycloalkyl carbon atoms remains. Examples of heterocycloalkyl groups include oxiranyl, thiaranyl, aziridinyl, oxetanyl, thiatanyl, azetidinyl, tetrahydrofuranyl, tetrahydrothiophenyl, pyrrolidinyl, tetrahydropyranyl, tetrahydrothiopyranyl, piperidinyl, 1,4-dioxanyl, 1,4-oxathianyl, morpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl and 1,4-diazepanyl. The heterocycloalkyl group may be C-linked or N-linked, *i.e.* it may be linked to the remainder of the molecule through a carbon atom or through a nitrogen atom.

The term "heteroalkenyl" includes alkenyl groups in which up to three carbon atoms, in one embodiment up to two carbon atoms, in another embodiment one carbon atom, are each replaced independently by O, S(O)ₜ or N, provided at least one of the alkenyl carbon atoms remains. The heteroalkenyl group may be C-linked or hetero-linked, *i.e.* it may be linked to the remainder of the molecule through a carbon atom or through O, S(O)ₜ or N.

The term "heterocycloalkenyl" includes cycloalkenyl groups in which up to three carbon atoms, in one embodiment up to two carbon atoms, in another embodiment one carbon atom, are each replaced independently by O, S(O)ₜ or N, provided at least one of the cycloalkenyl carbon atoms remains. Examples of heterocycloalkenyl groups include 3,4-dihydro-2H-pyranyl, 5-6-dihydro-2H-pyranyl, 2H-pyranyl, 1,2,3,4-tetrahydropyridinyl and 1,2,5,6-tetrahydropyridinyl. The heterocycloalkenyl group may be C-linked or N-linked, *i.e.* it may be linked to the remainder of the molecule through a carbon atom or through a nitrogen atom.

The term "heteroalkynyl" includes alkynyl groups in which up to three carbon atoms, in one embodiment up to two carbon atoms, in another embodiment one carbon atom, are each replaced independently by O, S(O)ₜ or N, provided at least one of the alkynyl carbon atoms remains. The heteroalkynyl group may be C-linked or hetero-linked, *i.e.* it may be linked to the remainder of the molecule through a carbon atom or through O, S(O)ₜ or N.

The term "heteroalkylene" includes alkylene groups in which up to three carbon atoms, in one embodiment up to two carbon atoms, in another embodiment one carbon atom, are each replaced independently by O, S(O)ₜ or N, provided at least one of the alkylene carbon atoms remains.

The term "heteroalkenylene" includes alkenylene groups in which up to three carbon atoms, in one embodiment up to two carbon atoms, in another embodiment one carbon atom, are each replaced independently by O, S(O)ₜ or N, provided at least one of the alkenylene carbon atoms remains.

### Aryl

The term "aryl" includes monovalent, aromatic, cyclic hydrocarbyl groups, such as phenyl or naphthyl (*e.g.* 1-naphthyl or 2-naphthyl). In general, the aryl groups may be monocyclic or polycyclic fused ring aromatic groups. Preferred aryl refers to C₆-C₁₄aryl.

Other examples of aryl groups are monovalent derivatives of aceanthrylene, acenaphthylene, acephenanthrylene, anthracene, azulene, chrysene, coronene, fluoranthene, fluorene, as-indacene, s-indacene, indene, naphthalene, ovalene, perylene, phenalene, phenanthrene, picene, pleiadene, pyrene, pyranthrene and rubicene.

The term "arylalkyl" means alkyl substituted with an aryl group, *e.g.* benzyl.

### Heteroaryl

The term "heteroaryl" includes aryl groups in which one or more carbon atoms are each replaced by heteroatoms independently selected from O, S, N and NR^{N}, where R^{N} is defined below (and in one embodiment is H or alkyl (*e.g.* C₁₋₆alkyl)).

In general, the heteroaryl groups may be monocyclic or polycyclic (*e.g.* bicyclic) fused ring heteroaromatic groups. Typically, heteroaryl groups contain 5-14 ring members (preferably 5-10 members) wherein 1, 2, 3 or 4 ring members are independently selected from O, S, N and NR^{N}. In one embodiment, a heteroaryl group may be 5, 6, 9 or 10 membered, *e.g.* 5-membered monocyclic, 6-membered monocyclic, 9-membered fused-ring bicyclic or 10-membered fused-ring bicyclic.

Monocyclic heteroaromatic groups include heteroaromatic groups containing 5-6 ring members wherein 1, 2, 3 or 4 ring members are independently selected from O, S, N or NR^{N}.

In one embodiment, 5-membered monocyclic heteroaryl groups contain 1 ring member which is an -NR^{N}- group, an -O- atom or an -S- atom and, optionally, 1-3 ring members (*e.g.* 1 or 2 ring members) which are =N- atoms (where the remainder of the 5 ring members are carbon atoms).

Examples of 5-membered monocyclic heteroaryl groups are pyrrolyl, furanyl, thiophenyl, pyrazolyl, imidazolyl, isoxazolyl, oxazolyl, isothiazolyl, thiazolyl, 1,2,3 triazolyl, 1,2,4 triazolyl, 1,2,3 oxadiazolyl, 1,2,4 oxadiazolyl, 1,2,5 oxadiazolyl, 1,3,4 oxadiazolyl, 1,3,4 thiadiazolyl, pyridyl, pyrimidinyl, pyridazinyl, pyrazinyl, 1,3,5 triazinyl, 1,2,4 triazinyl, 1,2,3 triazinyl and tetrazolyl.

Examples of 6-membered monocyclic heteroaryl groups are pyridinyl, pyridazinyl, pyrimidinyl and pyrazinyl.

In one embodiment, 6-membered monocyclic heteroaryl groups contain 1 or 2 ring members which are =N- atoms (where the remainder of the 6 ring members are carbon atoms).

Bicyclic heteroaromatic groups include fused-ring heteroaromatic groups containing 9-14 ring members wherein 1, 2, 3, 4 or more ring members are independently selected from O, S, N or NR^{N}.

In one embodiment, 9-membered bicyclic heteroaryl groups contain 1 ring member which is an -NR^{N}- group, an -O- atom or an -S- atom and, optionally, 1-3 ring members (*e.g.* 1 or 2 ring members) which are =N- atoms (where the remainder of the 9 ring members are carbon atoms).

Examples of 9-membered fused-ring bicyclic heteroaryl groups are benzofuranyl, benzothiophenyl, indolyl, benzimidazolyl, indazolyl, benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,2-a]pyridinyl, pyrrolo[1,2-b]pyridazinyl and imidazo[1,2-c]pyrimidinyl.

In one embodiment, 10-membered bicyclic heteroaryl groups contain 1-3 ring members which are =N- atoms (where the remainder of the 10 ring members are carbon atoms). Examples of 10-membered fused-ring bicyclic heteroaryl groups are quinolinyl, isoquinolinyl, cinnolinyl, quinazolinyl, quinoxalinyl, phthalazinyl, 1,6-naphthyridinyl, 1,7-naphthyridinyl, 1,8-naphthyridinyl, 1,5-naphthyridinyl, 2,6-naphthyridinyl, 2,7-naphthyridinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heteroarylalkyl" means alkyl substituted with a heteroaryl group.

The term "nucleobase" refers to a compound containing a base according to any nucleoside, such as an adenine, guanine, cytosine, thymine and uracil.

The term analog or derivative refers to compounds that have a close structural and, preferably, functional similarity to a given reference compound.

### General

Unless indicated explicitly otherwise, where combinations of groups are referred to herein as one moiety, e.g. arylalkyl, the last mentioned group contains the atom by which the moiety is attached to the rest of the molecule.

Where reference is made to a carbon atom of an alkyl group or other group being replaced by O, S(O)ₜ or N, what is intended is that: 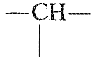 is replaced by 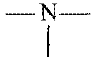
-CH= is replaced by -N=;
=C-H is replaced by =N; or
-CH₂- is replaced by -O-, -S(O)ᵣ- or -NR^{N}-.

By way of clarification, in relation to the above mentioned heteroatom containing groups (such as heteroalkyl etc.), where a numerical of carbon atoms is given, for instance C₃₋₆heteroalkyl, what is intended is a group based on C₃₋₆alkyl in which one of more of the 3-6 chain carbon atoms is replaced by O, S(O)ₜ or N. Accordingly, a C₃₋₆heteroalkyl group, for example, will contain less than 3-6 chain carbon atoms.

Where mentioned above, R^{N} is H, alkyl, cycloalkyl, aryl, heteroaryl, -C(O)-alkyl, -C(O)-aryl, -C(O)-heteroaryl, -S(O)ₜ-alkyl, -S(O)ₜ-aryl or -S(O)ₜ-heteroaryl. R^{N} may, in particular, be H, alkyl (e.g. C₁₋₆alkyl) or cycloalkyl (e.g. C₃₋₅cycloalkyl).

Where mentioned above, t is independently 0, 1 or 2, for example 2. Typically, t is 0.

Where a group has at least 2 positions which may be substituted, the group may be substituted by both ends of an alkylene or heteroalkylene chain to form a cyclic moiety.

### Substituents

Optionally substituted groups of the compounds of the invention (e.g. heterocyclic groups, alkyl, cycloalkyl, alkoxy, alkenyl, cycloalkenyl, alkynyl, alkylene, alkenylene, heteroalkyl, heterocycloalkyl, heteroalkenyl, heterocycloalkenyl, heteroalkynyl, heteroalkylene, heteroalkenylene, aryl, arylalkyl, arylheteroalkyl, heteroaryl, heteroarylalkyl or heteroarylheteroalkyl groups etc.) may be substituted or unsubstituted, in one embodiment unsubstituted. Typically, substitution involves the notional replacement of a hydrogen atom with a substituent group, or two hydrogen atoms in the case of substitution by =O.

Where substituted, there will generally be 1 to 3 substituents unless otherwise stated herein, in one embodiment 1 or 2 substituents, for example 1 substituent.

The optional substituent(s) may be selected independently from the groups consisting of halogen, trihalomethyl, trihaloethyl, OH, NH₂, -NO₂, -CN, -N⁺(C₁₋₆alkyl)₂O⁻, -CO₂H, -CO₂C₁₋₆alkyl, -SO₃H, -SOC₄₋₆alkyl, -SO₂C₁₋₆alkyl, -SO₃C₁₋₆alkyl, -OC(=O)OC₁₋₆alkyl, -C(=O)H, -C(=O)C₁₋₆alkyl, -OC(=O)C₁₋₆alkyl, =O, -N(C₁₋₆alkyl)₂, -C(=O)NH₂, -C(=O)N(C₁₋₆alkyl)₂, -N(C₁₋₆alkyl)C(=O)O(C₁₋₆alkyl), -N(C₁₋₆alkyl)C(=O)N(C₁₋₆alkyl)₂, -OC(=O)N(C₁₋₆alkyl)₂, -N(C₁₋₆alkyl)C(=O)C₁₋₆alkyl, -C(=S)N(C₁₋₆alkyl)₂, -N(C₁₋₆alkyl)C(=S)C₁₋₆alkyl, -SO₂N(C₁₋₆alkyl)₂, -N(C₁₋₆alkyl)SO₂C₁₋₆alkyl, -N(C₁₋₆alkyl)C(=S)N(C₁₋₆alkyl)₂, -N(C₁₋₆alkyl)SO₂N(C₁₋₆alkyl)₂, -C₁₋₆alkyl, -C₁₋₆heteroalkyl, -C₃₋₆cycloalkyl, -C₃₋₆heterocycloalkyl, -C₂₋₆alkenyl, -C₂₋₆heteroalkenyl, -C₃₋₆cycloalkenyl, -C₃₋₆heterocycloalkenyl, -C₂₋₆alkynyl, -C₂₋₆heteroalkynyl, -Z^{u}-C₁₋₆alkyl, -Z^{u}-C₃₋₆cycloalkyl, -Z^{u}-C₂₋₆alkenyl, -Z^{u}-C₃₋₆cycloalkenyl and -Z^{u}-C₂₋₆alkynyl, wherein
Z^{u} is independently O, S, NH or N(C₁₋₆alkyl).

In another embodiment, the optional substituent(s) is/are independently OH, NH₂, halogen, trihalomethyl, trihaloethyl, -NO₂, -CN, -N⁺(C₁₋₆alkyl)₂O⁻, -CO₂H, -SO₃H, -SOC₁₋₆alkyl, -SO₂C₁₋₆alkyl, -C(=O)H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl or -Z^{u}-C₃₋₆cycloalkyl, wherein Z^{u} is defined above.

In another embodiment, the optional substituent(s) is/are independently OH, NH₂, halogen, trihalomethyl, -NO₂, -CN, -CO₂H, -C(=O)C₁₋₆alkyl, =O, -N(C₁₋₆alkyl)₂, -C(=O)NH₂, -C₁₋₆alkyl, -C₃₋₆cycloalkyl, -C₃₋₆heterocycloalkyl, -Z^{u}C₁₋₆alkyl or - Z^{u}-C₃₋₆cycloalkyl, wherein Z^{u} is defined above.

In another embodiment, the optional substituent(s) is/are independently halogen, OH, NH₂, -NO₂, -CN, -CO₂H, =O, -N(C₁₋₆alkyl)₂, -C₁₋₆alkyl, -C₃₋₆cycloalkyl or -C₃₋₆heterocycloalkyl.

In another embodiment, the optional substituent(s) is/are independently halogen, OH, NH₂, =O, -C₁₋₆alkyl, -C₃₋₆cycloalkyl or -C₃₋₆heterocycloalkyl.

### Compounds of the invention and derivatives thereof

As used herein, the terms "compounds of the invention" and "compound of formula (I)" etc. include pharmaceutically acceptable derivatives thereof and polymorphs, geometrical, optical, enantiomeric, diastereomeric isomers or tautomeric forms and isotopically labelled variants thereof.

### Pharmaceutically acceptable derivatives

The term "pharmaceutically acceptable derivative" includes any pharmaceutically acceptable salt, solvate, hydrate or prodrug of a compound of the invention. In one embodiment, the pharmaceutically acceptable derivatives are pharmaceutically acceptable salts, solvates or hydrates of a compound of the invention, particularly pharmaceutically acceptable salts.

### Pharmaceutically acceptable salts

Salts of the compounds of the invention may be formed where acidic or basic groups are present. In typical embodiments the salts are pharmaceutically acceptable salts.

Compounds of the invention which contain basic, e.g. amino, groups are capable of forming salts, such as pharmaceutically acceptable salts, with acids. In embodiments, pharmaceutically acceptable acid addition salts of the compounds of the invention include salts of inorganic acids such as hydrohalic acids (e.g. hydrochloric, hydrobromic and hydroiodic acid), sulfuric acid, nitric acid and phosphoric acids. In embodiments, pharmaceutically acceptable acid addition salts of the compounds of the invention include those of organic acids such as aliphatic, aromatic, carboxylic and sulfonic classes of organic acids, examples of which include: aliphatic monocarboxylic acids such as formic acid, acetic acid, propionic acid and butyric acid; aliphatic hydroxy acids such as lactic acid, citric acid, tartaric acid and malic acid; dicarboxylic acids such as maleic acid and succinic acid; aromatic carboxylic acids such as benzoic acid, p-chlorobenzoic acid, phenylacetic acid, diphenylacetic acid and triphenylacetic acid; aromatic hydroxyl acids such as o-hydroxybenzoic acid, p-hydroxybenzoic acid, 1-hydroxynaphthalene-2-carboxylic acid and 3-hydroxynaphthalene-2-carboxylic acid; and sulfonic acids such as methanesulfonic acid, ethanesulfonic acid and benzenesulfonic acid. Other pharmaceutically acceptable acid addition salts of the compounds of the invention include those of glycolic acid, glucuronic acid, furoic acid, glutamic acid, anthranilic acid, salicylic acid, mandelic acid, embonic (pamoic) acid, pantothenic acid, stearic acid, sulfanilic acid, algenic acid and galacturonic acid. Wherein the compound of the invention comprises a plurality of basic groups, multiple centres may be protonated to provide multiple salts, e.g. di- or tri-salts of compounds of the invention. For example, a hydrohalic acid salt of a compound of the invention as described herein may be a monohydrohalide, dihydrohalide or trihydrohalide, *etc.* In one embodiment, the salts include, but are not limited to those resulting from addition of any of the acids disclosed above. In one embodiment of the compound of the invention, two basic groups form acid addition salts. In a further embodiment, the two addition salt counterions are the same species, e.g. dihydrochloride, dihydrosulphide etc. Typically, the pharmaceutically acceptable salt is a hydrochloride salt, such as a dihydrochloride salt.

Compounds of the invention which contain acidic, e.g. carboxyl, groups are capable of forming pharmaceutically acceptable salts with bases. Pharmaceutically acceptable basic salts of the compounds of the invention include, but are not limited to, metal salts such as alkali metal or alkaline earth metal salts (*e.g.* sodium, potassium, magnesium or calcium salts) and zinc or aluminium salts, and salts formed with ammonia, organic amines (e.g. ammonium, mono-, di-, tri- and tetraalkylammonium salts), or heterocyclic bases such as ethanolamines (*e.g.* diethanolamine), benzylamines, N-methyl-glucamine, and amino acids (*e.g.* lysine). In typical embodiments, the base addition salt is selected from sodium, potassium and ammonium, mono-, di-, tri- and tetraalkylammonium salts. In one embodiment, pharmaceutically acceptable basic salts of the compounds of the invention include, but are not limited to, salts formed with ammonia or pharmaceutically acceptable organic amines or heterocyclic bases such as ethanolamines (*e.g.* diethanolamine), benzylamines, N-methyl-glucamine, and amino acids (*e.g.* lysine).

Hemisalts of acids and bases may also be formed, *e.g.* hemisulphate salts.

Pharmaceutically acceptable salts of compounds of the invention may be prepared by methods well-known in the art.

For a review of pharmaceutically acceptable salts, see Stahl and Wermuth, Handbook of Pharmaceutical Salts: Properties, Selection and Use (Wiley-VCH, Weinheim, Germany, 2002).

### Solvates & hydrates

The compounds of the invention may exist in both unsolvated and solvated forms. The term "solvate" includes molecular complexes comprising a compound of the invention and one or more pharmaceutically acceptable solvent molecules such as water or C₁₋₆ alcohols, *e.g.* ethanol. The term "hydrate" means a "solvate" where the solvent is water.

### Prodrugs

The compounds as disclosed herein act as bioorthogonal prodrugs which may be cleaved in the presence of palladium. However, the compounds may be used with conventional prodrug strategies and thus may further include pro-moieties which are, when administered *in vivo,* converted into compounds of the invention (e.g. compounds of formula (I)) under biological conditions. Tegafur is for example a known prodrug of 5-FU. Thus, the disclosure provides compounds wherein the heterocyclic compound is tegafur, i.e. wherein the heterocyclic group according to formula (II) is a tegafur residue.

Suitable pro-moieties for use alongside the groups of formula (III) in the compounds of the invention are metabolized *in vivo* to form a compound of the invention comprising the group of formula (III) or a heterocyclic compound as produced when the group of formula (III) is cleaved from the group of formula (II) in the presence of palladium. The design of prodrugs is well-known in the art, as discussed in Bundgaard, Design of Prodrugs 1985 (Elsevier), The Practice of Medicinal Chemistry 2003, 2nd Ed, 561-585 and Leinweber, Drug Metab. Res. 1987, 18: 379.

Examples of prodrugs of compounds of the invention are esters and amides of the compounds of the invention (e.g. esters and amides of compounds of formula (I)). For example, where the compound of the invention contains a carboxylic acid group (-COOH), the hydrogen atom of the carboxylic acid group may be replaced in order to form an ester (*e.g.* the hydrogen atom may be replaced by C₁₋₆alkyl). Where the compound of the invention contains an alcohol group (-OH), the hydrogen atom of the alcohol group may be replaced in order to form an ester (*e*.*g*. the hydrogen atom may be replaced by -C(O)C₁₋₆alkyl. Where the compound of the invention contains a primary or secondary amino group, one or more hydrogen atoms of the amino group may be replaced in order to form an amide (*e.g.* one or more hydrogen atoms may be replaced by -C(O)C₁₋₆alkyl).

### Amorphous & crystalline forms

The compounds of the invention may exist in solid states from amorphous through to crystalline forms. All such solid forms are included within the invention.

### Isomeric forms

Compounds of the invention may exist in one or more geometrical, optical, enantiomeric, diastereomeric and tautomeric forms, including but not limited to *cis*- and *trans*-forms, *E*- and *Z-*forms, *R-, S-* and meso-forms, keto- and enol-forms. All such isomeric forms are included within the invention. The isomeric forms may be in isomerically pure or enriched form, as well as in mixtures of isomers (*e.g.* racemic or diastereomeric mixtures).

Accordingly, the invention provides:
- stereoisomeric mixtures of compounds of the invention;
- a diastereomerically enriched or diastereomerically pure isomer of a compound of the invention; or
- an enantiomerically enriched or enantiomerically pure isomer of a compound of the invention.

Where appropriate, isomers can be separated from their mixtures by the application or adaptation of known methods (*e.g.* chromatographic techniques, resolution techniques and recrystallization techniques). Where appropriate, isomers can be prepared by the application or adaptation of known methods (*e.g.* asymmetric synthesis).

### Isotopic labeling

The invention includes pharmaceutically acceptable isotopically-labelled compounds of the invention wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled compounds of formula (I), for example, those incorporating a radioactive isotope, are useful in drug and/or substrate tissue distribution studies. The radioactive isotopes ³H and ¹⁴C are particularly useful for this purpose in view of their ease of incorporation and ready means of detection.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled compounds of the invention can generally be prepared by conventional techniques known to those skilled in the art or by processes analogous to those described herein using an appropriate isotopically-labelled reagent in place of the non-labelled reagent previously employed.

### Treatment of Diseases and Conditions

Compounds of the invention have been found by the inventors to be useful prodrugs, for instance in aspects and embodiments described herein wherein the group of formula (III) is bonded to the endocyclic nitrogen atom of a drug compound, or in compounds wherein the group of formula (II) is a drug residue, the compounds are prodrugs because the group of formula (III) may be cleaved using palladium *in vivo* to reveal the drug compound *in vivo.* Suitably, the drug compound is produced in therapeutically effective amounts.

Typically, the compound or salt is provided as pharmaceutical composition comprising a compound or salt (e.g. pharmaceutically acceptable salt) thereof according to the invention in combination with a pharmaceutically acceptable excipient (such as described below in "*Administration and Formulation*"), which may be suitably used in the methods of treatment discussed above.

In some embodiments of the present invention a compound according to the present invention is provided for use in a method of treatment, wherein the method of treatment involves administration of the compound to the patient in the form of a prodrug intended to be converted into an active drug compound in the body. The conversion from prodrug to drug is intended to be a reaction capable of being catalysed by palladium, particularly palladium(0). As such, methods of treatment and therapeutic uses may include a method in which the compound is administered to a patient in which palladium is present and in a manner that allows contact between the compound and palladium so that the therapeutically active form of the drug is generated in the body.

The invention thus provides a compound or salt according to any of the second, third or fourth aspects or embodiments thereof described above, or a pharmaceutical composition thereof for use in a method of treatment, wherein the method comprises co-administering the compound or composition and the palladium to a subject. Suitable compounds for use in said methods of treatment, as discussed above, are prodrugs intended to be converted into an active drug compound in the body as described above, i.e. compounds of the above aspects and embodiments thereof wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The invention provides the use of a compound or salt according to any of the second, third or fourth aspects or embodiments thereof described above, or a suitable pharmaceutical composition in the manufacture of a medicament for use in a method of treatment, wherein the method comprises co-administering the compound or composition and palladium to a subject. Suitable compounds and salts for use in said methods of treatment are prodrugs intended to be converted into an active drug compound in the body as described above, i.e. compounds and salts of the above aspects and embodiments thereof wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The disclosure thus provides a method of treatment comprising co-administering a compound or salt according to any of the second, third or fourth aspects or embodiments thereof described above, or a suitable pharmaceutical composition thereof and palladium to a subject. The compound, salt or composition is administered in an amount that is suitable for generating a therapeutic amount of drug in the subject. Suitable compounds and salts for use in said methods of treatment are prodrugs intended to be converted into an active drug compound in the body as described above, i.e. compounds and salts of the above aspects and embodiments thereof wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The invention also provides a compound or salt according to any of the second to fourth aspects and embodiments thereof described above, or a pharmaceutical composition thereof for use in a method of treatment, wherein the method comprises administration of the compound or composition to a subject to which palladium has already been administered, optionally wherein the palladium has been administered as an extracellular palladium implant. Suitable compounds and salts for use in said methods of treatment are prodrugs intended to be converted into an active drug compound in the body as described above, i.e. compounds and salts of the above aspects and embodiments thereof wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The invention thus provides the use of a compound or salt according to any of the second to fourth aspects and embodiments thereof described above, or a pharmaceutical composition thereof in the manufacture of a medicament for use in a method of treatment, wherein the method comprises administration of the compound or composition to a subject to which palladium has already been administered, optionally wherein the palladium has been administered as an extracellular palladium implant. Suitable compounds and salts for use in said methods of treatment are prodrugs intended to be converted into an active drug compound in the body as described above, i.e. compounds and salts of the above aspects and embodiments thereof wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The disclosure thus provides a method of treatment comprising administering a compound or salt according to any of the second to fourth aspects and embodiments described above, or a pharmaceutical composition thereof to a subject to which palladium has already been administered, optionally wherein the palladium has been administered as an extracellular palladium implant. The compound, salt or composition is administered in an amount that is suitable for generating a therapeutic amount of drug in the subject. Suitable compounds and salts for use in said methods of treatment are prodrugs intended to be converted into an active drug compound in the body as described above, i.e. compounds and salts of the above aspects and embodiments thereof wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The invention also provides a palladium implant for use in a method of treatment, wherein the method comprises co-administering a compound or salt thereof as defined according to any of the second to fourth aspects and embodiments thereof described above, or a pharmaceutical composition thereof, and the palladium implant to the subject. Suitable compounds and salts for use in said methods of treatment are prodrugs intended to be converted into an active drug compound in the body as described above, i.e. compounds and salts of the above aspects wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The invention thus provides the use of a palladium implant in the manufacture of a combined preparation for use in a method of treatment, wherein the method comprises co-administering a compound or salt thereof as defined according to any of the second to fourth aspects and embodiments thereof described above, or a pharmaceutical composition thereof and the palladium implant to the subject. Suitable compounds and salts for use in said methods of treatment are prodrugs intended to be converted into an active drug compound in the body as described above, i.e. compounds and salts of the above aspects wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The disclosure thus provides a method of treatment comprising administration of a palladium implant to a subject and separately or simultaneously administering a compound or salt thereof as defined according to any of the second to fourth aspects and embodiments thereof described above, or a pharmaceutical composition thereof to the subject. Suitable compounds and salts for use in said methods of treatment are prodrugs intended to be converted into an active drug compound in the body as described above, i.e. compounds and salts of the above aspects wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The invention also provides a palladium implant for use in a method of treatment, wherein the method comprises administration of a compound or salt thereof according to any of the second to fourth aspects and embodiments thereof described above, or a pharmaceutical composition thereof, wherein the subject has been pre-implanted with the palladium implant prior to administration of the compound, salt or composition. Suitable compounds and salts for use in said methods of treatment are prodrugs intended to be converted into an active drug compound in the body, i.e. compounds and salts of the above aspects wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The invention also provides the use of a palladium implant in the manufacture of a combined preparation for use in a method of treatment, wherein the method comprises administration of a compound or salt thereof according to any of the second to fourth aspects and embodiments described above, or a pharmaceutical composition thereof wherein the subject has been pre-implanted with the palladium implant prior to administration of the compound, salt or composition. Suitable compounds and salts for use in said methods of treatment are prodrugs intended to be converted into an active drug compound in the body, i.e. compounds and salts of the above aspects wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

The disclosure also provides a method of treatment comprising implanting a palladium implant in a patient prior to administration of a compound or salt thereof as described in any of the second to fourth aspects and embodiments, or a pharmaceutical composition thereof. Suitable compounds and salts for use in said methods of treatment are prodrugs intended to be converted into an active drug compound in the body, i.e. compounds and salts of the above aspects wherein the group of formula (III) is bonded to an endocyclic nitrogen atom adjacent to a ring carbonyl of a drug compound, or in other words, wherein the group of formula (II) is a drug residue (e.g. compounds as described in the fourth aspect).

Preferably, the method of treatment in the above methods and therapeutic uses is a method of treating cancer, Parkinson's disease, a viral infection, heart disease, convulsions, psychosis, a bacterial infection or a fungus infection. In particular embodiments, the cancer is selected from pancreatic and / or colorectal cancer, for instance, wherein the colorectal cancer includes cancerous HCT116 cells and / or wherein the pancreatic cancer includes cancerous BXPc-3 cells.

Said co-administration may involve the simultaneous, sequential or separate administration of the compound of the invention and the palladium. Typically, however, the palladium is administered (e.g. implanted) prior to, or at the same time as, administering the compound of the invention. Preferably, the palladium is administered before the compound of the invention is administered. How long before may depend on the method by which the palladium is administered.

### Administration of palladium

Palladium may be administered to the patient by any convenient means, e.g. by injection of a fluid solution containing palladium, or a colloidal solution containing palladium nanoparticles. The mode of administration may depend on the form in which the palladium is provided. As described above, the palladium may be conjugated to another molecule, such as a peptide, polynucleic acid, or fluorogenic tag. In such embodiments, it is desirable for the palladium to be free to move systemically around the body and so the palladium is preferably administered in the form of a solution or suspension, such as a colloidal solution containing palladium nanoparticles.

In preferred embodiments the palladium is provided in the form of an implant, which may be located at a therapeutically important location in the body, e.g. at, in, adjacent or near a tissue requiring treatment with the therapeutically active form of the drug, such as at, in, adjacent or near a tumour.

In some embodiments, the palladium may be administered by non-surgical means, such as injection or ingestion. In other embodiments, the palladium may be administered by or during a surgical process.

Administration of the palladium will preferably occur before administration of the prodrug so that the prodrug may be converted to the therapeutically active agent in the body.

As described above under the heading "palladium" in the section referring to methods of the invention, a palladium implant may have a range of physical forms, the intention being that the implant retains the palladium substantially at or near the site of administration/implantation thereby providing a localised concentration of palladium and preventing unwanted high levels of palladium circulating throughout the body. Examples of a palladium implant include a material coated or impregnated in palladium or in a palladium containing compound, such as a palladium-containing alloy. The material may be a solid (e.g. a porous solid) or semi-solid, e.g. a gel, and may be in the form of a bolus. The implant should allow for contact of prodrug present in the tissue or associated vasculature with the palladium present in the implant.

The implant material may be selected to allow the coated or impregnated palladium to be released from the material when administered to or implanted in the subject. Release kinetics may be altered by altering the structure, e.g. porosity, of the material.

The material provides a scaffold or matrix support for the palladium. The material may be suitable for implantation in tissue, or may be suitable for administration to the body (e.g. as microcapsules in solution).

Preferably, the implant material should be biocompatible, e.g. non-toxic and of low immunogenicity (most preferably non-immunogenic). The biomaterial may be biodegradable such that the biomaterial degrades over time. Alternatively a non-biodegradable biomaterial may be used, allowing surgical removal of the implant as required.

Suitable materials and constructions for suitable palladium implants are described above under the heading "palladium" in the section referring to methods of the invention.

### Therapeutic definitions

As used herein, "treatment" includes curative and prophylactic treatment. As used herein, a "patient" means an animal, preferably a mammal, preferably a human, in need of treatment.

The amount of the compound of the invention administered should be a therapeutically effective amount where the compound or derivative is used for the treatment of a disease or condition and a prophylactically effective amount where the compound or derivative is used for the prevention of a disease or condition.

The term "therapeutically effective amount" used herein refers to the amount of compound needed to treat or ameliorate a targeted disease or condition. The term "prophylactically effective amount" used herein refers to the amount of compound needed to prevent a targeted disease or condition. The exact dosage will generally be dependent on the patient's status at the time of administration. Factors that may be taken into consideration when determining dosage include the severity of the disease state in the patient, the general health of the patient, the age, weight, gender, diet, time, frequency and route of administration, drug combinations, reaction sensitivities and the patient's tolerance or response to therapy. The precise amount can be determined by routine experimentation, but may ultimately lie with the judgement of the clinician. Generally, an effective dose will be from 0.01 mg/kg/day (mass of drug compared to mass of patient) to 1000 mg/kg/day, *e.g.* 1 mg/kg/day to 100 mg/kg/day. Compositions may be administered individually to a patient or may be administered in combination with other agents, drugs or hormones.

### Administration & Formulation

### General

For pharmaceutical use, the compounds of the invention may be administered as a medicament by enteral or parenteral routes, including intravenous, intramuscular, subcutaneous, transdermal, airway (aerosol), oral, intranasal, rectal, vaginal, urethral and topical (including buccal and sublingual) administration. The compounds of the invention should be assessed for their biopharmaceutical properties, such as solubility and solution stability (across pH), permeability, *etc.,* in order to select the most appropriate dosage form and route of administration for treatment of the proposed indication.

The compounds of the invention may be administered as crystalline or amorphous products. The compounds of the invention may be administered alone or in combination with one or more other compounds of the invention or in combination with one or more other drugs (or as any combination thereof). Generally, they will be administered as a formulation, i.e. composition in association with one or more pharmaceutically acceptable excipients. The term "excipient" includes any ingredient other than the compound(s) of the invention which may impart either a functional (*e.g.* drug release rate controlling) and/or a non-functional (*e.g.* processing aid or diluent) characteristic to the formulations. The choice of excipient will to a large extent depend on factors such as the particular mode of administration, the effect of the excipient on solubility and stability and the nature of the dosage form.

Typical pharmaceutically acceptable excipients include:
- diluents, *e.g.* lactose, dextrose, sucrose, mannitol, sorbitol, cellulose and/or glycine;
- lubricants, *e.g.* silica, talcum, stearic acid, its magnesium or calcium salt and/or polyethyleneglycol;
- binders, *e.g.* magnesium aluminum silicate, starch paste, gelatin, tragacanth, methylcellulose, sodium carboxymethylcellulose and/or polyvinylpyrrolidone;
- disintegrants, *e.g.* starches, agar, alginic acid or its sodium salt, or effervescent mixtures; and/or
- absorbants, colorants, flavors and/or sweeteners.

A thorough discussion of pharmaceutically acceptable excipients is available in Gennaro, Remington: The Science and Practice of Pharmacy 2000, 20th edition (ISBN: 0683306472).

Accordingly, in one embodiment, the present invention provides a pharmaceutical composition comprising a heterocyclic compound of the invention and a pharmaceutically acceptable excipient.

### Oral administration

The compounds of the invention may be administered orally. Oral administration may involve swallowing, so that the compound enters the gastrointestinal tract, and/or buccal, lingual, or sublingual administration by which the compound enters the blood stream directly from the mouth.

Formulations suitable for oral administration include solid plugs, solid microparticulates, semi-solid and liquid (including multiple phases or dispersed systems) such as tablets; soft or hard capsules containing multi- or nano-particulates, liquids (*e.g.* aqueous solutions), emulsions or powders; lozenges (including liquid-filled); chews; gels; fast dispersing dosage forms; films; ovules; sprays; and buccal/mucoadhesive patches.

Formulations suitable for oral administration may also be designed to deliver the compounds of the invention in an immediate release manner or in a rate-sustaining manner, wherein the release profile can be delayed, pulsed, controlled, sustained, or delayed and sustained or modified in such a manner which optimises the therapeutic efficacy of the said compounds. Means to deliver compounds in a rate-sustaining manner are known in the art and include slow release polymers that can be formulated with the said compounds to control their release.

Examples of rate-sustaining polymers include degradable and non-degradable polymers that can be used to release the said compounds by diffusion or a combination of diffusion and polymer erosion. Examples of rate-sustaining polymers include hydroxypropyl methylcellulose, hydroxypropyl cellulose, methyl cellulose, ethyl cellulose, sodium carboxymethyl cellulose, polyvinyl alcohol, polyvinyl pyrrolidone, xanthum gum, polymethacrylates, polyethylene oxide and polyethylene glycol.

Liquid (including multiple phases and dispersed systems) formulations include emulsions, suspensions, solutions, syrups and elixirs. Such formulations may be presented as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Liang and Chen, Expert Opinion in Therapeutic Patents 2001, 11(6): 981-986.

The formulation of tablets is discussed in H. Lieberman and L. Lachman, Pharmaceutical Dosage Forms: Tablets 1980, vol. 1 (Marcel Dekker, New York).

### Parenteral administration

The compounds of the invention can be administered parenterally. The compounds of the invention may be administered directly into the blood stream, into subcutaneous tissue, into muscle, or into an internal organ. Suitable means for administration include intravenous, intraarterial, intrathecal, intraventricular, intraurethral, intrasternal, intracranial, intramuscular, intrasynovial and subcutaneous. Suitable devices for administration include needle (including microneedle) injectors, needle-free injectors and infusion techniques.

Parenteral formulations are typically aqueous or oily solutions. Where the solution is aqueous, excipients such as sugars (including but not restricted to glucose, mannitol, sorbitol, *etc*.) salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water (WFI).

Parenteral formulations may include implants derived from degradable polymers such as polyesters (*i.e*. polylactic acid, polylactide, polylactide-co-glycolide, polycaprolactone, polyhydroxybutyrate), polyorthoesters and polyanhydrides. These formulations may be administered via surgical incision into the subcutaneous tissue, muscular tissue or directly into specific organs.

The preparation of parenteral formulations under sterile conditions, for example, by lyophilization, may readily be accomplished using standard pharmaceutical techniques well known to those skilled in the art.

The solubility of compounds of formula (I) used in the preparation of parenteral solutions may be increased by the use of appropriate formulation techniques, such as the incorporation of co-solvents and/or solubility-enhancing agents such as surfactants, micelle structures and cyclodextrins.

### Inhalation & intranasal administration

The compounds of the invention can be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler, as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane, or as nasal drops. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenization or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as *I*-leucine, mannitol or magnesium stearate. The lactose may be anhydrous or in the form of the monohydrate, preferably the latter. Other suitable excipients include dextran, glucose, maltose, sorbitol, xylitol, fructose, sucrose and trehalose.

Formulations for inhaled/intranasal administration may be formulated to be immediate and/or modified release using, for example, poly(lactic*-co*-glycolic acid) (PGLA). Modified release formulations include delayed-, sustained-, pulsed-, controlled-, targeted and programmed release.

### Transdermal administration

Suitable formulations for transdermal application include a therapeutically effective amount of a compound of the invention with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

### Combination Therapy

The compounds, salts and compositions of the invention may be administered alone or may be administered in combination with another therapeutic agent (i.e. a different agent to the compound of the invention). Preferably, the compound of the invention and the other therapeutic agent are administered in a therapeutically effective amount.

The compounds, salts and compositions of the present invention may be administered either simultaneously with, or before or after, the other therapeutic agent. The compound or salt of the present invention may be administered separately, by the same or different route of administration, or together in the same pharmaceutical composition.

In one embodiment, the invention provides a product comprising a compound of the invention and another therapeutic agent as a combined preparation for simultaneous, separate or sequential use in therapy. The combined preparation may suitably be co-administered with palladium to promote deprotection of the group of formula (III) in compounds of the invention. For instance, the combination therapy may be administered to a patient in which palladium has already been administered, for instance in the form of a palladium implant. Products provided as a combined preparation include a composition comprising the compound of the invention and the other therapeutic agent together in the same pharmaceutical composition, or the compound of the invention and the other therapeutic agent in separate form, e.g. in the form of a kit. The other therapeutic agent may suitably be another compound, e.g. prodrug of the invention.

In one embodiment, the invention provides a pharmaceutical composition comprising a compound of the invention and another therapeutic agent. Optionally, the pharmaceutical composition may comprise a pharmaceutically acceptable excipient, as described above in "*Administration and Formulation*"*.* Typically the composition is a solid composition.

In one embodiment, the invention provides a kit comprising two or more separate pharmaceutical compositions, at least one of which contains a compound of the invention. In one embodiment, the kit comprises means for separately retaining said compositions, such as a container, divided bottle or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, the compound of the invention and the other therapeutic agent may be manufactured and/or formulated by the same or different manufacturers. Moreover, the compound of the invention and the other therapeutic may be brought together into a combination therapy: (i) prior to release of the combination product to physicians (*e.g.* in the case of a kit comprising the compound of the invention and the other therapeutic agent); (ii) by the physician themselves (or under the guidance of the physician) shortly before administration; (iii) in the patient themselves, *e.g.* during sequential administration of the compound of the invention and the other therapeutic agent.

Accordingly, the invention provides a compound of the invention (i.e. a prodrug) for use in a method of treating a disease or condition, wherein the medicament may optionally be prepared for administration with another therapeutic agent. The invention also provides the use of another therapeutic agent in the manufacture of medicament for treating a disease or condition, wherein the medicament is prepared for administration with the compound of the invention.

In an embodiment, the other therapeutic agent is selected from:
(i) blood pressure lowering therapies, comprising, for example, a) Angiotensin-converting enzyme (ACE) inhibitors, such as benazepril, captopril, cilazapril, enalapril, fosinopril, lisinopril, perindopril, quinapril, ramipril and trandolapril; b) Angiotensin Receptor Blockers, such as candesartan, eprosartan, irbesartan, losartan, olmesartan, telmisartan and valsartan; c) Calcium-channel blockers, such as amlodipine, diltiazem, felodipine, isradipine, lacidipine, lercanidipine, nicardipine, nifedipine, nisoldipine and verapamil; d) Diuretics, such as bendroflumethiazide (bendrofluazide), chlorothiazide, chlorthalidone, cyclopenthiazide, furosemide, hydrochlorothiazide indapamide, metolazone and torsemide; e) Beta-blockers, such as acebutolol, atenolol, betaxolol, bisoprolol, metoprolol, nadolol, oxprenolol, pindolol, propranolol, sotalol and timolol; f) methyldopa or alpha blockers; g) endothelin receptor antagonists such as bosentan, darusentan, enrasentan, tezosentan, atrasentan, ambrisentan sitaxsentan; h) smooth muscle relaxants such as PDE5 inhibitors (indirect-acting), minoxidil and diazoxide (direct-acting); i) alpha receptor blockers, such as doxazosin, terazosin, alfuzosin, tamsulosin; and j) central alpha agonists, such as clonidine.
(iii) angina therapies, comprising, for example, the above vasodilators and a) Isosorbide dinitrate (ISDN), as found in Angitac, Sorbid, Isoket, Sorbitrate, Sorbichew, Isordil and Cedocard; and b) Isosorbide mononitrate (ISMN), as found in Isotrate, Chemydur, Imdur, Isib, Isotard, MCR, Modisal, Monomax, Monosorb, Imazin, Elantan, Ismo, Monit and Mono-Cedocard; and
(iv) cholesterol lowering therapies, comprising, for example, a) statins, such as atorvastatin, fluvastatin, lovastatin, pravastatin, rosuvastatin and simvastatin; b) Anion-exchange resins such as colestyramine (cholestyramine) and colestipol; c) Fibrates, such as bezafibrate, ciprofibrate, fenofibrate and gemfibrozil; d) cholesteryl ester transfer protein inhibitors, such as torcetrapib; and d) others, such as Nicotinic acid, Ezetimibe, cholesterol absorption inhibitors and Fish oils.

In another embodiment, the other therapeutic agent is a chemotherapeutic agent selected from the group consisting of:
(i) alkylating agents, comprising, for example, busulfan, cisplatin, carboplatin, chlorambucil, cyclophosphamide, ifosfamide, dacarbazine (DTIC), mechlorethamine (nitrogen mustard), melphalan and temozolomide;
(ii) nitrosoureas, comprising, for example, carmustine (BCNU) and lomustine (CCNU);
(iii) antimetabolites, comprising, for example, 5-fluorouracil, capecitabine, 6-mercaptopurine, methotrexate, gemcitabine, cytarabine (ara-C), fludarabine and pemetrexed;
(iv) anthracyclines and related drugs, comprising, for example, daunorubicin, doxorubicin (Adriamycin), epirubicin, idarubicin and mitoxantrone;
(v) topoisomerase II inhibitors, comprising, for example, topotecan, irinotecan, etoposide (VP-16) and teniposide;
(vi) mitotic inhibitors, comprising, for example, taxanes (paclitaxel, docetaxel) and the vinca alkaloids (vinblastine, vincristine and vinorelbine); and
(vii) corticosteroid hormones, comprising, for example, prednisone and dexamethasone.

The chemotherapeutics may also be selected from other known chemotherapeutics, e.g. L-asparaginase, dactinomycin, thalidomide, tretinoin, imatinib (Gleevec), gefitinib (Iressa), erlotinib (Tarceva), rituximab (Rituxan), bevacizumab (Avastin), anti-estrogens (tamoxifen, fulvestrant), aromatase inhibitors (anastrozole, exemestane, letrozole), progestins (megestrol acetate), anti-androgens (bicalutamide, flutamide) and LHRH agonists (leuprolide, goserelin).

It is particularly contemplated that the chemotherapeutic agent can be, for example, a microtubule poison, a DNA alkylating agent, etc. Suitable microtubule poisons include, but are not limited to, paclitaxel. Suitable DNA alkylating agents include, e.g., carboplatin, etc.

In another embodiment, the other therapeutic agent is selected from:
(i) antidepressants, comprising, for example, amitriptyline, amoxapine, bupropion, citalopram, clomipramine, desipramine, doxepin duloxetine, elzasonan, escitalopram, fluvoxamine, fluoxetine, gepirone, imipramine, ipsapirone, maprotiline, mirtazapine, nortriptyline, nefazodone, paroxetine, phenelzine, protriptyline, reboxetine, sertraline, sibutramine, thionisoxetine, tranylcypromaine, trazodone, trimipramine and venlafaxine;
(ii) atypical antipsychotics, comprising, for example, quetiapine and lithium;
(iii) antipsychotics, comprising, for example, amisulpride, aripiprazole, asenapine, benzisoxidil, bifeprunox, carbamazepine, clozapine, chlorpromazine, debenzapine, divalproex, duloxetine, eszopiclone, haloperidol, iloperidone, lamotrigine, loxapine, mesoridazine, olanzapine, paliperidone, perlapine, perphenazine, phenothiazine, phenylbutlypiperidine, pimozide, prochlorperazine, risperidone, sertindole, sulpiride, suproclone, suriclone, thioridazine, trifluoperazine, trimetozine, valproate, valproic acid, zopiclone, zotepine and ziprasidone;
(iv) anxiolytics, comprising, for example, alnespirone, azapirones,benzodiazepines, barbiturates such as adinazolam, alprazolam, balezepam, bentazepam, bromazepam, brotizolam, buspirone, clonazepam, clorazepate, chlordiazepoxide, cyprazepam, diazepam, diphenhydramine, estazolam, fenobam, flunitrazepam, flurazepam, fosazepam, lorazepam, lormetazepam, meprobamate, midazolam, nitrazepam, oxazepam, prazepam, quazepam, reclazepam, tracazolate, trepipam, temazepam, triazolam, uldazepam, zolazepam and equivalents and pharmaceutically active isomer(s) and metabolite(s) thereof;
(v) anticonvulsants, comprising, for example, carbamazepine, topiramate, valproate, lamotrigine and gabapentin;
(vi) Alzheimer's therapies, comprising, for example, donepezil, memantine and tacrine;
(vii) Parkinson's therapies, comprising, for example, deprenyl, L-dopa, Requip (ropinirole), Mirapex, MAOB inhibitors such as selegiline and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase;
(viii) migraine therapies, comprising, for example, almotriptan, amantadine, bromocriptine, butalbital, cabergoline, dichloralphenazone, eletriptan, frovatriptan, lisuride, naratriptan, pergolide, pramipexole, rizatriptan, ropinirole, sumatriptan, zolmitriptan and zomitriptan;
(ix) stroke therapies, comprising, for example, abciximab, activase, (NXY-059), citicoline, crobenetine, desmoteplase, repinotan and traxoprodil;
(x) urinary incontinence therapies, comprising, for example, darifenacin, falvoxate, oxybutynin, propiverine, robalzotan, solifenacin, trypium and tolterodine;
(xi) neuropathic pain therapies, comprising, for example, gabapentin, lidoderm and pregablin;
(xii) nociceptive pain therapies, comprising, for example, celecoxib, etoricoxib, lumiracoxib, rofecoxib, valdecoxib, diclofenac, loxoprofen, naproxen and paracetamol; and
(xiii) insomnia therapies, comprising, for example, allobarbital, alonimid, amobarbital, benzoctamine, butabarbital, capuride, chloral, cloperidone, clorethate, dexclamol, eszopiclone, ethchlorvynol, etomidate, glutethimide, halazepam, hydroxyzine, mecloqualone, melatonin, mephobarbital, methaqualone, midaflur, nisobamate, pentobarbital, phenobarbital, propofol, roletamide, triclofos3secobarbital, zaleplon and Zolpidem.

### GENERAL

The term "comprising" encompasses "including" as well as "consisting" e.g. a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x is optional and means, for example, x±10 %.

### DESCRIPTION OF FIGURES

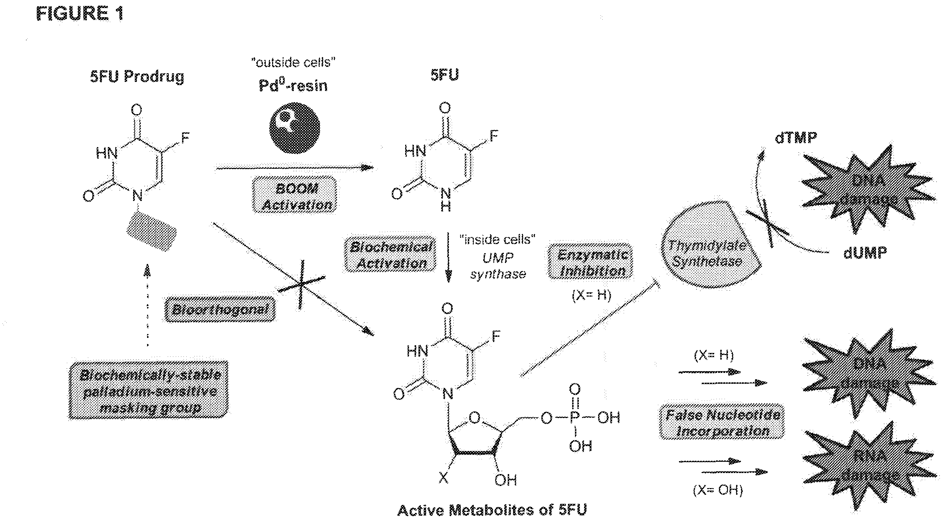

**Figure 1** depicts a figurative illustration of a BOOM prodrug deprotection strategy of the present invention showing in situ deprotection of a 5FU prodrug by an extracellular Pd resin implant to generate active 5FU which acts on the respective biological pathways leading to cytotoxic anti-cancer effects.

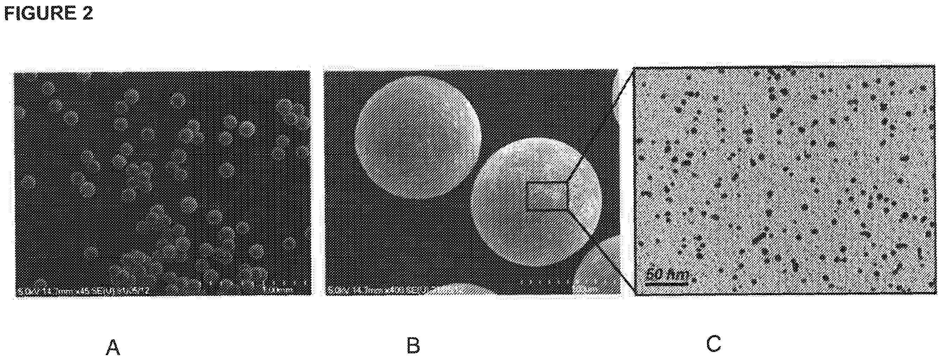

**Figure 2** shows images of Pd⁰-functionalized PEG-PS resins: Figure 2A shows an SEM image at x45 magnification; Figure 2B shows an SEM image at x400 magnification; and Figure 2C shows a TEM image of a Pd⁰-resin cross-section at x1,000 magnification.
**Figure 3** shows LCMS data for Pd⁰-mediated conversion of prodrugs into 5FU performed in 0.1% (v/v) DMSO in PBS and incubated for 24h at 37 °C (Thermomixer, shaker speed: 1,400 rpm). Figures 3A-C show the LCMS data for the conversion of Pro-5FU to 5FU at 0 h, 7 h and 24 h, respectively. Figure 3D shows LCMS analysis of commercial 5FU. Figures E and F show the LCMS analysis of crude reaction mixture at 48 h for All-5FU and Bn-5FU respectively showing low levels of 5FU in the reaction mixture.
**Figure 4** illustrates the proposed BOOM reaction pathway for the reaction of Pro-5FU with Pd(0) to form 5FU via an allenyl-palladium intermediate.

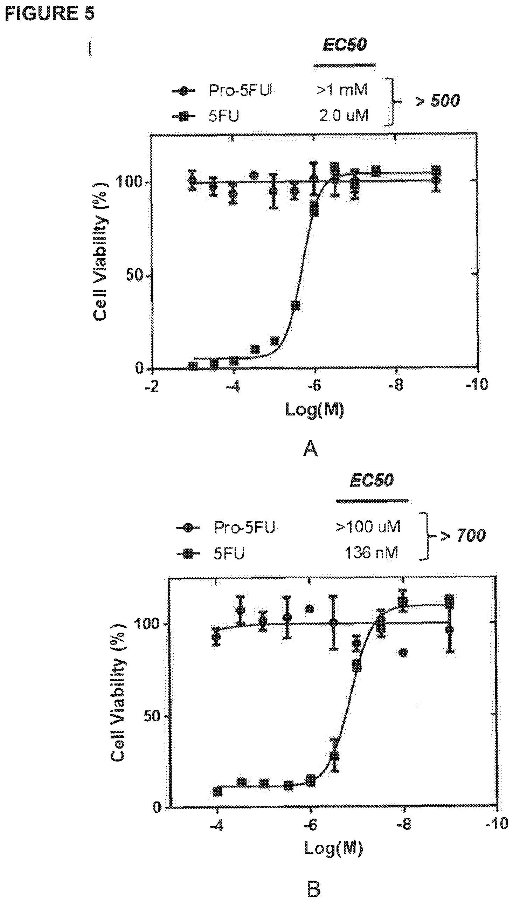

**Figure 5** shows the relationship between drug/prodrug concentrations for 5FU and Pro-5FU and cell viability (%) for both colorectal HCT116 cells (Figure 5A) and pancreatic BxPC-3 cells (Figure 5B) and the respective calculated EC₅₀ values, showing relative toxicity for 5FU and Pro-5FU in these cell lines.

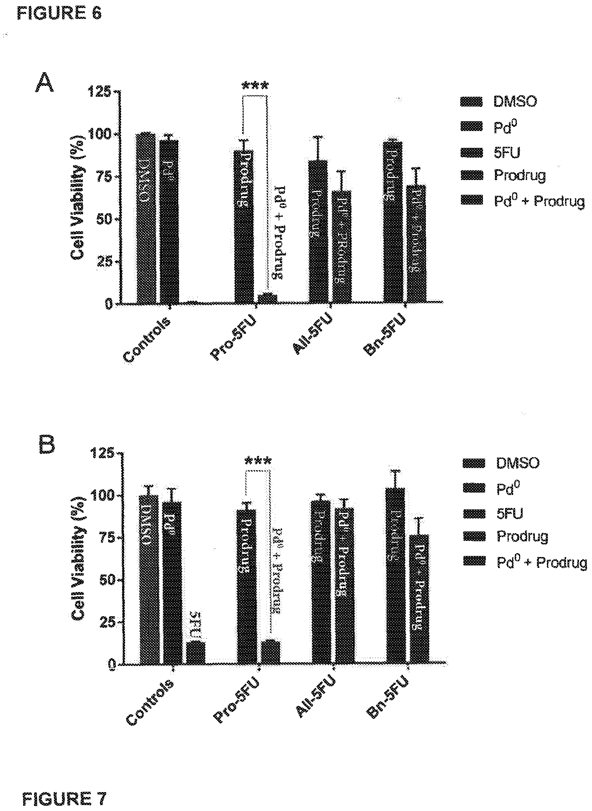

**Figure 6** shows the results of a BOOM conversion study showing relative toxicities (as indicated by % cell viability) against HCT116 cells (Figure 6A) and pancreatic BxPC-3 cells (Figure 6B) of prodrug-palladium combinations for Pro-5FU compared to All-5FU and Bn-5FU. Cell viabilities for each prodrug provided as a combination with palladium is presented in the left of each set of two bars, Data for prodrug in the absence of palladium catalyst is also provided (the right bar of each set of two bars) for comparison along with negative controls (from left to right: DMSO, Pd(0) and 5FU). Cells were incubated in tissue culture media containing 0.1% (v/v) DMSO and: Pd⁰-resins (1 mg / mL, negative control); 100 µM of each prodrug (negative control); and Pd⁰-resins (1 mg / mL) + 100 µM of each prodrug (BOOM reaction assay). Cells incubated in 0.1% (v/v) DMSO in media were used as untreated cell control.

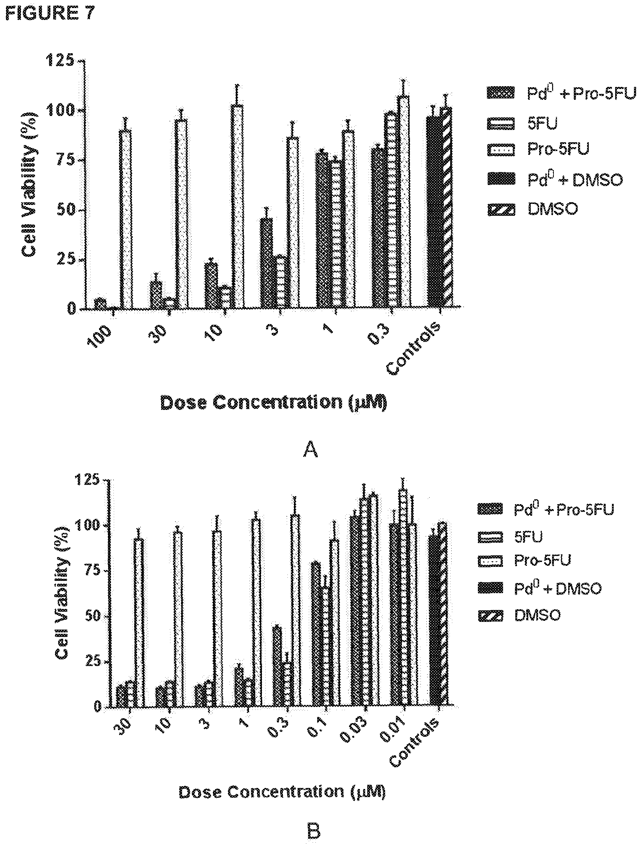

**Figure 7** shows the dose dependent toxicology data (bar graph) indicated by % cell viability (colorectal HCT116 cells in Figure 7A and pancreatic BXPc-3 cells in Figure 7B) for conversion of Pro-5FU into 5FU using extracellular palladium resins. Following 5 days treatment, cells were incubated with PrestoBlue™ Cell Viability Reagent (Life Technologies) for 45 min. Fluorescence intensity values were related to the untreated cells (100% cell viability). Data are provided for cells incubated in tissue culture media containing 0.1% (v/v) DMSO and: Pd⁰-resins (1 mg / mL, negative control); 0.01-100 µM of Pro-5FU (negative control); 0.01-100 µM of 5FU (positive control); and Pd⁰-resin (1 mg / mL) + Pro-5FU (BOOM reaction assay). Cells incubated in 0.1% (v/v) DMSO in media were used as untreated cell control.

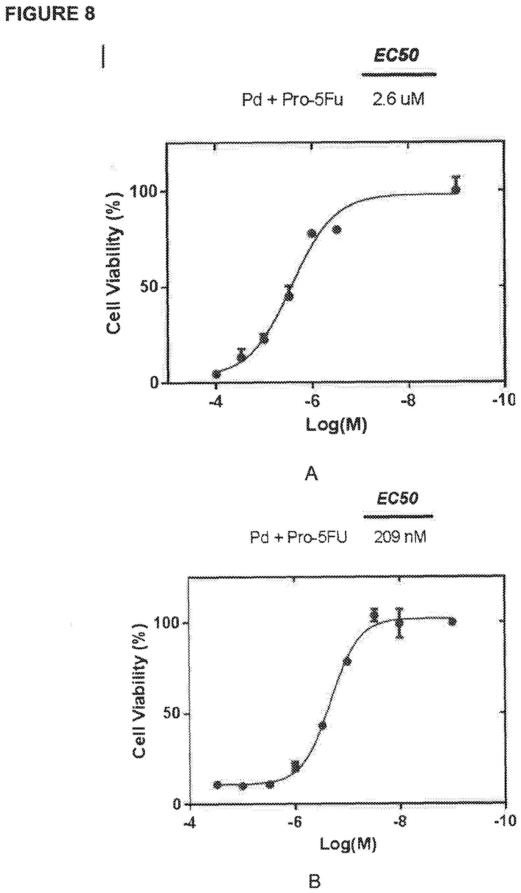

**Figure 8** shows the dose / toxigenic response data (line graph) showing % cell viability against concentration for Pro-5FU / Pd⁰-resin combinations (colorectal HCT116 cells in Figure 8A and pancreatic BXPc-3 cells in Figure 8B). Increasing doses of Pro-5FU (0.01, 0.03, 0.1, 0.3, 1, 3, 10, 30 µM for both cell lines, and additional 100 µM for colorectal HCT116) and a constant dose of Pd⁰-resin (1 mg / mL) were incubated with cells for 5 days and cell viability measured to determine the corresponding EC₅₀ values as above.

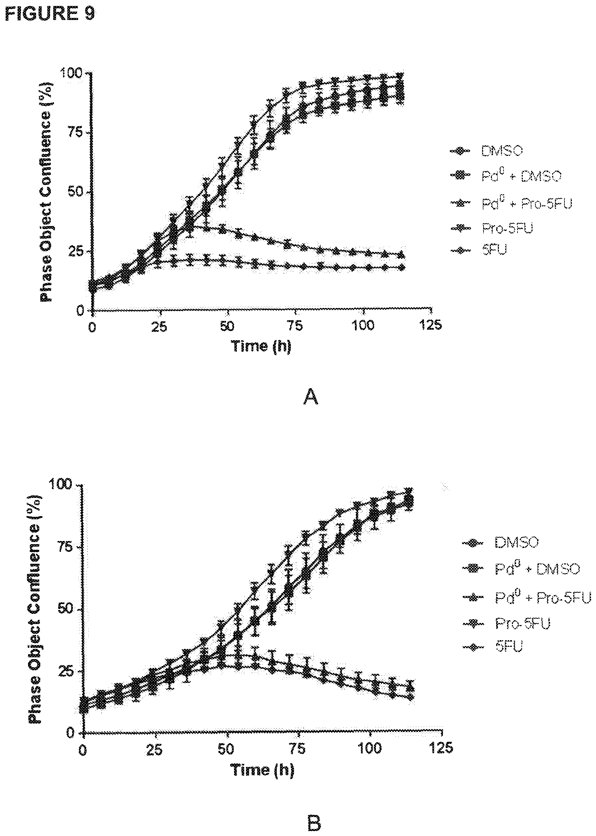

**Figure 9** shows real-time cell confluence study data for Pro-5FU and 5FU (HCT116 cells in Figure 9A and BXPc-3 cells in Figure 9B) plotting cell proliferation (indicated by % phase object confluence) against time. Cell proliferation was monitored for 5 days (120 h) using the high-content live-cell imaging system Incucyte™ (Essen BioScience) placed in an incubator (5% CO₂, 37 °C). Pro-5FU and 5FU were used at 100 µM and 10 µM for HCT116 and BXPc-3 cells, respectively.

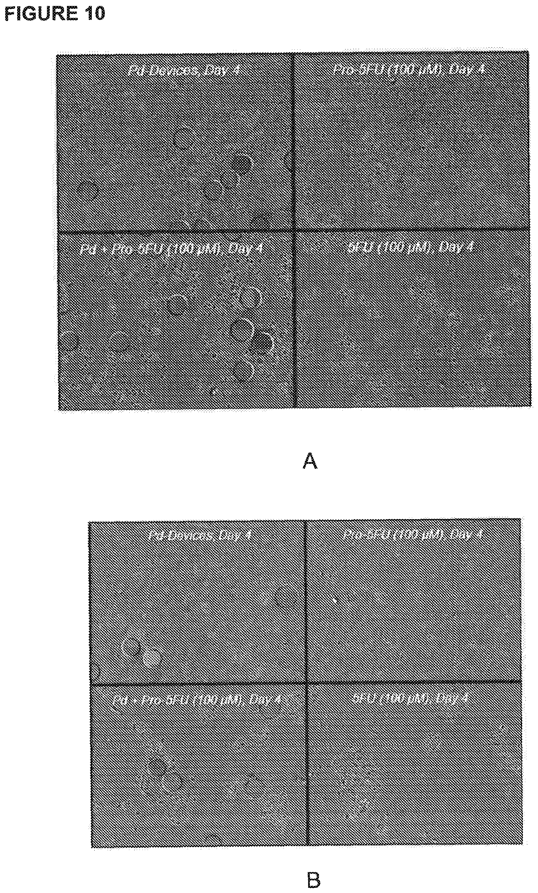

**Figure 10** shows the phase contrast images of cells in cell confluence study in Figure 9 after 4 days of treatment (96 h) for HCT116 cells (Figure 10A) and BXPc-3 cells (Figure 10B).

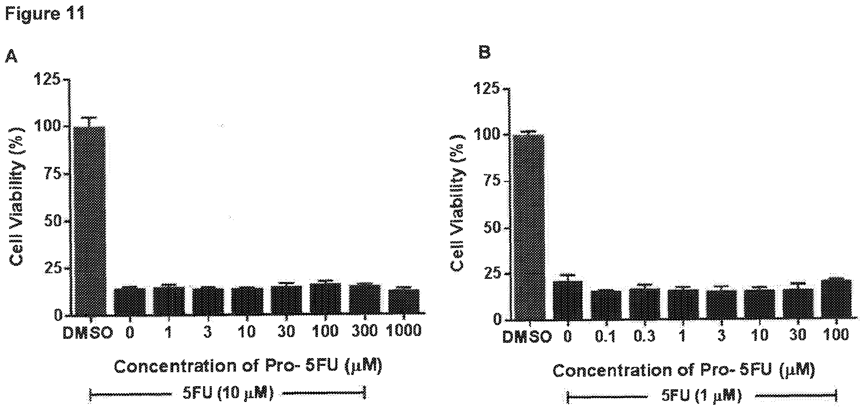

**Figure 11** shows toxicological data (% cell viability) for 5FU with increasing doses of Pro-5FU (0.1-1,000 µM) against colorectal HCT116 cells (Figure 11A) and pancreatic BxPC-3 cells (Figure 11B). The data show that Pro-5FU has no effect on cytotoxicity of 5FU.
**Figure 12** shows a proposed pallado-cyclic intermediate formed by the coordination of the alkyne group and the proximal carbonyl group in the reaction between N-propargyl protected 5-FU (i.e. "Pro-5FU") and palladium according to a method of the invention.
**Figure 13** shows the various canonical forms and respective theoretical pKa values based on delocalisation of a negative charge at the N3 position versus delocalisation of a negative charge at the N1 position.

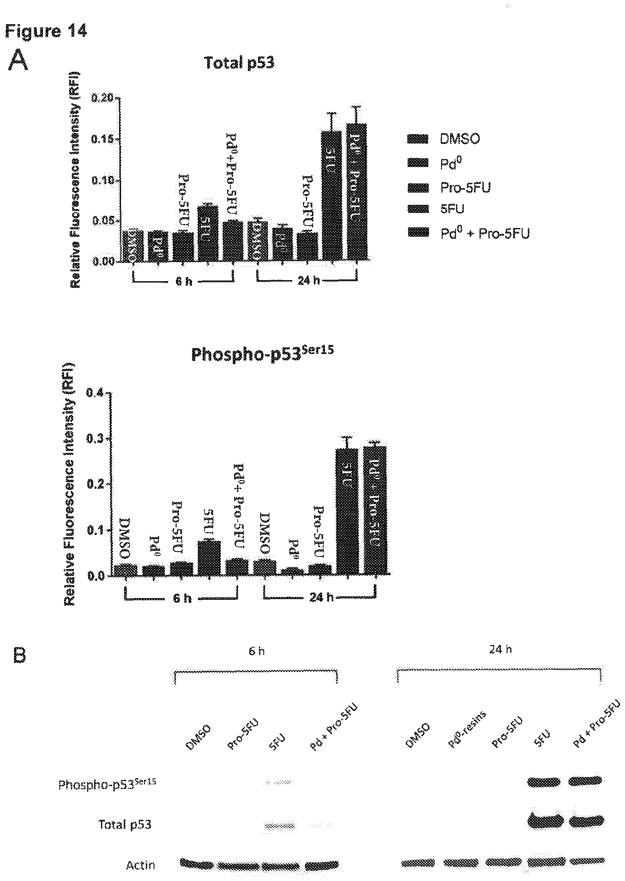

**Figure 14** shows most significant post-translational modifications identified from the study of colorectal HCT116 cells. Figure 14A shows Zeptosens Reverse Protein Microarray analysis. Bar graphs represent the median relative fluorescence intensity (RFI) values and standard deviation calculated across a 4-fold protein concentration series extracted from each sample. Data represents the relative abundance of total p53 protein (left) and phosphorylated p53 (right) across negative control (DMSO, Pd⁰+DMSO, Pro-5FU) samples, positive control (5FU) sample and Pd⁰ + Pro-5FU combination sample at 6 and 24 h treatment exposures. Figure 14B shows Western Blot analysis of p53 response in colorectal HCT116 cells. Relative abundance of phosphorylated p53 and total p53 protein across negative controls (DMSO, Pd⁰+DMSO, Pro-5FU), positive control (5FU) and Pd⁰ + Pro-5FU combination at 6 and 24 h treatment exposures.

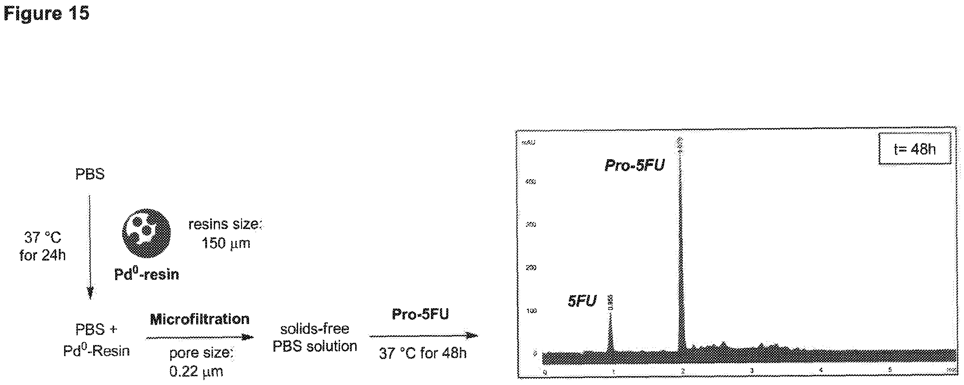

**Figure 15** shows the HPLC method and data from a heterogeneous catalysis study as described in the examples.

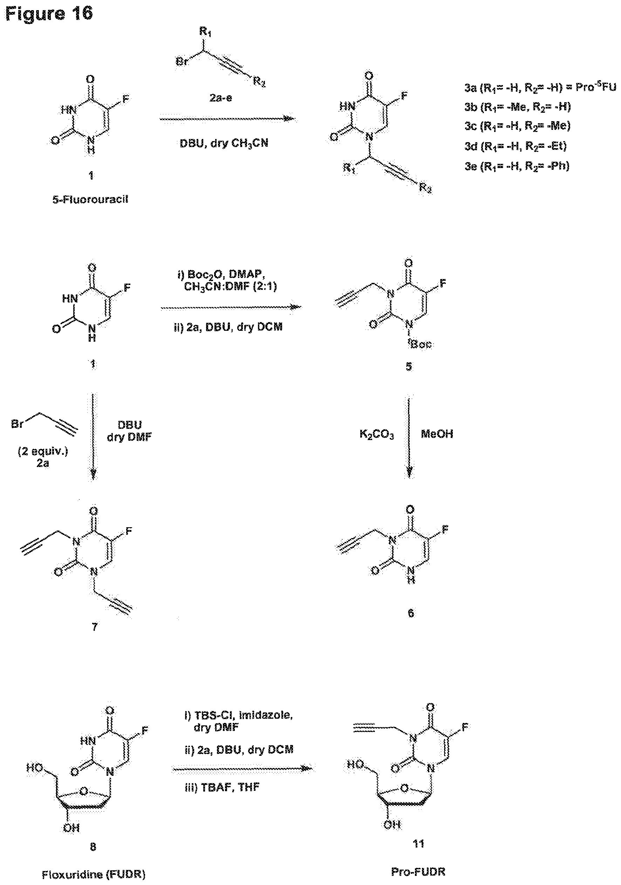

**Figure 16** shows the synthesis schemes for compounds **3a-e** (upper panel), compounds **6,7** (middle panel) and compound **11** (lower panel).

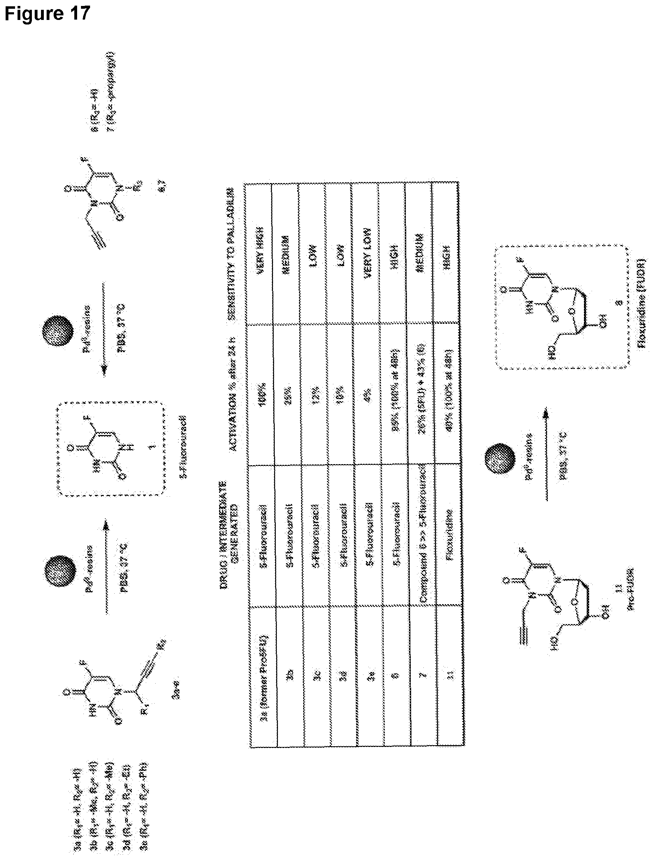

**Figure 17** illustrates the palladium-mediated reactions of the drug precursors with Pd(0) under biocompatible conditions to form either 5FU, **1,** or floxuridine, **8.** In the table it is compiled the conversion percentages and resulting products after incubating each of the drug precursors (100 µM) with 1mg/mL of Pd⁰-resins in PBS for 24 h at 37 °C (Thermomixer, shaker speed: 1,400 rpm). Values were calculated by HPLC using an UV detector.

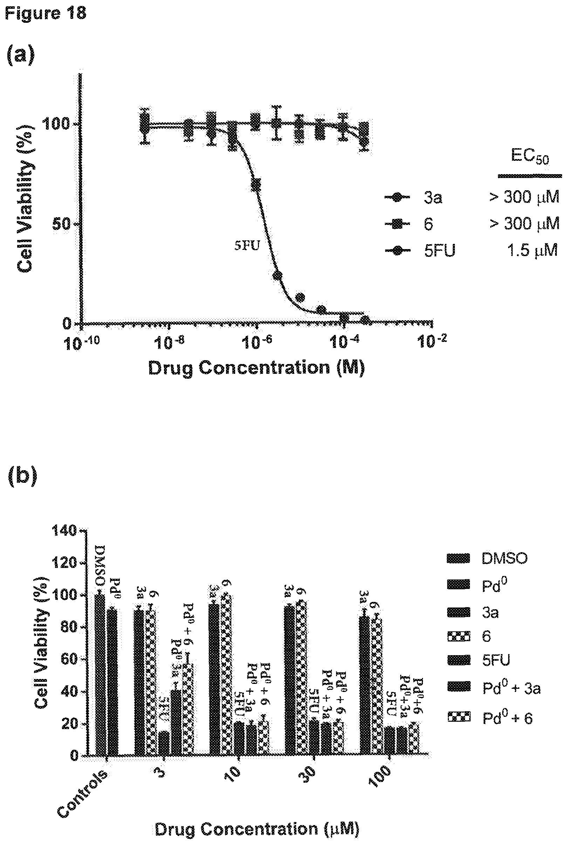

**Figure 18** **Panel (a)** shows dose response toxicological data (% cell viability; dose range 0.1-300 µM) induced by the native drug 5FU in comparison with compounds **3a** (also known as Pro-5FU) and compound **6** against colorectal HCT116 cells and the respective calculated EC₅₀ values. The data show that **3a** (Pro-5FU) and **6** have no effect on HCT116 cell viability. **Panel (b)** shows the dose dependent toxicology data (bar graph) indicated by % cell viability against colorectal HCT116 for conversion of compound **6** into 5FU using extracellular palladium resins. Following 5 days treatment, cells were incubated with PrestoBlue™ Cell Viability Reagent (Life Technologies) for 45 min. Fluorescence intensity values were related to the untreated cells (100% cell viability). Data are provided for cells incubated in tissue culture media containing 0.1% (v/v) DMSO and: Pd⁰-resins (1 mg / mL, negative control); 3-100 µM of **6** (negative control); 3-100 µM of 5FU (positive control); and Pd⁰-resin (1 mg / mL) + **6** (BOOM reaction assay). Cells incubated in 0.1% (v/v) DMSO in media were used as untreated cell control.

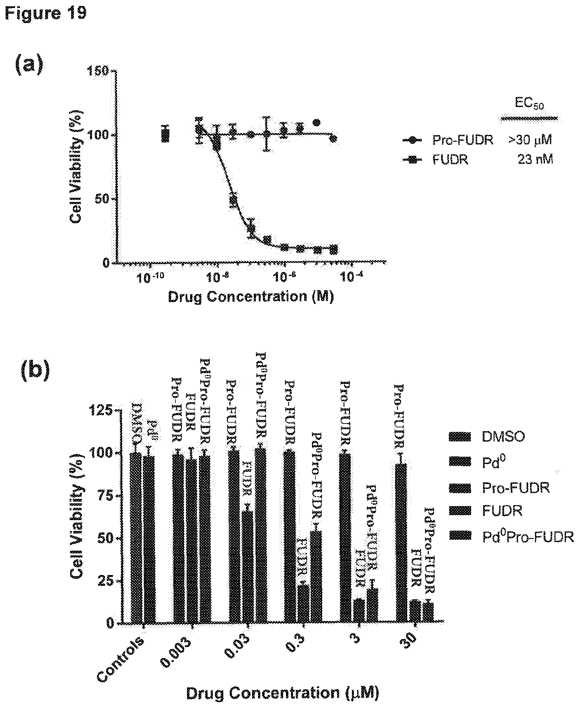

**Figure 19****. Panel (a)** shows dose response toxicological data (% cell viability; dose range 0.003-30 µM) induced by the native drug floxuridine **8** (FUDR) in comparison with compounds **11** (Pro-FUDR) against colorectal HCT116 cells and the respective calculated EC₅₀ values. The data show that **11** (Pro-FUDR) has over 1000 times less cytotoxic effect on HCT116 cell viability than floxuridine **8** (FUDR). **Panel (b)** shows the dose dependent toxicology data (bar graph) indicated by % cell viability against colorectal HCT116 for conversion of compound **11** into floxuridine **8** using extracellular palladium resins. Following 5 days treatment, cells were incubated with PrestoBlue™ Cell Viability Reagent (Life Technologies) for 45 min. Fluorescence intensity values were related to the untreated cells (100% cell viability). Data are provided for cells incubated in tissue culture media containing 0.1% (v/v) DMSO and: Pd⁰-resins (1 mg / mL, negative control); 0.003-30 µM of **11** (Pro-FUDR, negative control); 0.003-30 µM of floxuridine **8** (FUDR, positive control); and Pd⁰-resin (1 mg / mL) + **11** (Pro-FUDR, BOOM reaction assay). Cells incubated in 0.1% (v/v) DMSO in media were used as untreated cell control.

The invention is described in more detail by way of example only with reference to the following Examples.

### GENERAL METHODS

### Materials synthesis and characterization

Chemicals and solvents were obtained from Fisher Scientific, Sigma-Aldrich or VWR International Ltd. NMR spectra were recorded at ambient temperature on a 500 MHz Bruker Avance III spectrometer. Chemical shifts are reported in parts per million (ppm) relative to the solvent peak. Rf values were determined on Merck TLC Silica gel 60 F254 plates under a 254 nm UV source. Purification of compounds was achieved through manual column chromatography using commercially available silica gel.

### Preparation of palladium functionalized resins

As reported previously (Nature Protocols, 7, 1207-1218 (2012) and Nature Protocols, 7, 1207-1218 (2012), palladium nanoparticles are highly active, safe for biological applications, and can be efficiently trapped in / on amino-functionalized polystyrene matrix by Pd²⁺ coordination, reduction to generate Pd⁰ nanoparticles, and intensive surface cross-linking to physically capture the nanoparticles.

Following the procedure developed by Bradley (J. Am. Chem. Soc. 128, 6276-6277 (2006)) with minor modifications, Pd⁰-functionalized PEG-polystyrene resins were prepared. NovaSyn TG amino resin HL (1.00 g, 0.44 mmol NH₂ / g) and palladium acetate (296.3 mg, 1.32 mmol) were added into a 25 mL Biotage® microwave with toluene (10 mL) and heated to 80 °C for 10 min. The mixture was then stirred at room temperature for 2 h and the resins subsequently filtered and washed with dichloromethane (5 x 20 ml) and methanol (5 x 20 ml). Resins were dispersed in 10 % hydrazine monohydrate in methanol (10 mL) and stirred at room temperature for 1 h. The resins were then filtered and washed with dichloromethane (5 x 20 mL) and methanol (5 x 20 ml). Resins were added to a solution of Fmoc-Glu(OH)-OH (216 mg, 0.59 mmol), Oxyma (166 mg, 1.18 mmol), DIC (149 mg, 1.18 mmol) and DCM : DMF (2:1, 9 mL) and shaken in a Thermomixer for 2 h at room temperature. The resins were filtered and washed with dichloromethane (5 x 20 mL), methanol (5 x 20 mL) and H₂O (5 x 20 mL) and dried in a pressurized 40 °C oven for 3 days. Complete coupling was verified by the ninhydrin test.

The Pd⁰-functionalized PEG-polystyrene resins were of 0.15mm in average diameter (Figs 2A and 2B). TEM images showed dark nanoparticles (5 nM) regularly distributed across the resins (Fig 2c), while the presence of elemental Pd⁰ was confirmed by powder x-ray diffraction. Palladium was quantified by inductively coupled plasma - optical emission spectrometry (ICP-OES), indicating that Pd⁰ content was 28.3 g / Kg of Pd⁰-resins (i.e. 2.83 % w/w). Although total Pd⁰ content is 2.8 % w/w, the proportion of it that is accessible / reactive to prodrugs is expected to be lower.

### EXAMPLES

### General method for synthesis of 5-FU prodrugs from 5-FU

DBU (276 µl, 1.85 mmol, 1.2 equiv.) and 5-FU (200 mg, 1.54 mmol, 1 equiv.) were dissolved in dry DMF (2 ml) under N₂ atmosphere and cooled to 4 °C. Either allyl, propargyl or benzyl bromide (1.54 mmol, 1 equiv.) were dissolved in dry DMF (0.5 ml). The solution was added dropwise to the mixture and the resulting mixture stirred at room temperature overnight. Solvents were then removed under reduced pressure and the crude purified via flash chromatography (3 % MeOH in DCM).

### Example 1 - Pro-5FU

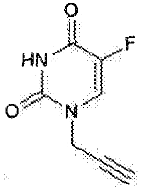

The synthetic method described above using propargyl bromide gave a colourless solid, 104 mg (40 % yield); Rf 0.35 (6% MeOH in DCM); ¹H NMR (500 MHz, DMSO) δ 11.91 (br s, 1H, N*H*), 8.13 (d, *J* = 5, 1H, ArH), 4.46 (d, *J* = 2.5, 2H, N-C*H*₂-C), 3.44 (t, *J* = 2.5, 1H, C-CH); ¹³C NMR (126 MHz, DMSO) δ 157.35 (d, *J_{C-F}* = 25.9, C), 149.11 (C), 139.80 (d, *J_{C-F}* = 230.4, C), 128.95 (d, *J_{C-F}* = 33.9, CH), 78.40 (C), 76.15 (CH), 37.00 (CH₂); MS (ESI) m/z 167.2 [M-H]⁻; HRMS (FAB) *m*/*z* calc. for C₇H₅O₂N₂F [M+H]⁺: 168.0332, found: 168.0330.

### Reference example 1 - All-5FU

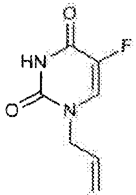

The synthetic method described above using allyl bromide gave a colourless solid, 80 mg (31 % yield); Rf= 0.5 (6% MeOH in DCM); ¹H NMR (500 MHz, DMSO) δ 11.80 (br s, 1H, N*H*), 8.01 (d, *J* = 6.7, 1H, ArH), 5.88 (ddt, *J* = 17.0, 10.5, 5.3, 1H, N-CH₂-C*H*), 5.19 (m, 2H, CH₂-CH=C*H*₂), 4.24 (d, *J* = 5.3, 2H, N-C*H*₂-CH); ¹³C NMR (126 MHz, DMSO) δ 157.45 (d, *J_{C-F}* = 25.7, C), 149.46 (C), 139.68 (d, *J_{C-F}* = 229.3, C), 132.63 (CH), 129.79 (d, *J_{C-F}* = 33.2, CH), 117.64 (CH₂), 49.34 (CH₂); MS (ESI) m/z 169.2 [M-H]⁻; HRMS (FAB) *m*/*z* calc. for C₇H₇O₂N₂F [M+H]⁺: 170.0486, found: 170.0489.

### Reference example 2 - Bn-5FU

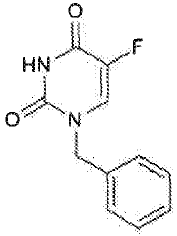

The synthetic method described above using benzyl bromide gave a pale yellow solid, 133 mg (38 % yield); Rf 0.44 (6% MeOH in DCM); ¹H NMR (500 MHz, DMSO) δ 11.86 (br s, 1H, NH), 8.22 (d, *J* = 6.7, 1H, Ar*H*), 7.39 - 7.28 (m, 5H, ArH), 4.83 (s, 2H, N-C*H*₂-Ph); ¹³C NMR (126 MHz, DMSO) δ 157.42 (d, *J_{C-F}* = 25.6, C), 149.68 (C), 139.62 (d, *J_{C-F}* = 227.9, C), 136.52 (C), 130.08 (d, *J_{C-F}* = 33.4, CH), 128.67 (CH), 127.75 (CH), 127.49 (CH), 50.63 (CH₂); MS (ESI) m/z 219.2 [M-H]⁻; HRMS (FAB) *m*/*z* calc. for C₁₁H₉O₂N₂F [M+H]⁺: 220.0643, found: 220.0643.

### Synthesis of N1-functionalized 5FU derivatives 3b-e

5-Fluoruouracil (100 mg, 0.77 mmol) and DBU (115 µl, 0.77 mmol) were dissolved in acetonitrile (2 ml), and the mixture was cooled down to 4 °C in an ice bath. The corresponding alkyl bromide (0.77 mmol) was added dropwise and the reaction mixture allowed to warm up to room temperature. The mixture was stirred overnight, the solvents removed *in vacuo* and the resulting crude purified via flash chromatography (eluent: 1.5 % MeOH in DCM), to yield compounds **3b-e** as pure white solids.

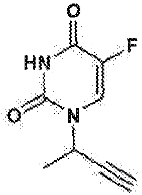

**1-(1-butyn-3-yl)-5-fluorouracil (3b).** 75 mg, 54 % yield. Rf = 0.55 (10 % MeOH in DCM). ¹H NMR (500 MHz, DMSO) δ 11.89 (s, 1H), 8.15 (d, *J* = 6.8, 1H), 5.40 - 5.30 (m, 1H), 3.61 (d, *J* = 2.4, 1H), 1.47 (d, *J* = 7.0, 3H). ¹³C NMR (126 MHz, DMSO) δ 157.01 (d, *J* = 26.0, C), 148.69, 140.19 (d, *J* = 231.4, C), 125.97 (d, *J* = 33.8, CH), 81.34, 76.53 (CH), 42.92 (CH), 20.47 (CH₃). MS (ESI) m/z 181.0 [M-H]⁻.

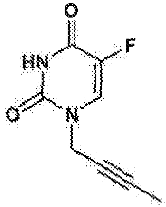

**1-(2-butyn-1-yl)-5-fluorouracil (3c).** 63 mg, 45 % yield. R_{f} = 0.53 (10 % MeOH in DCM). ¹H NMR (500 MHz, DMSO) δ 11.86 (s, 1H), 8.11 (d, *J* = 6.7, 1H), 4.41 (q, *J* = 2.3, 2H), 1.82 (t, *J* = 2.4, 3H). ¹³C NMR (126 MHz, DMSO) δ 157.30 (d, *J* = 259, C), 149.04, 139.71 (d, *J* = 230.2, C), 128.93 (d, *J* = 33.8, CH), 81.61, 73.39, 37.30 (CH₂), 3.07 (CH₃). MS (ESI) m/z 181.0 [M-H]⁻.

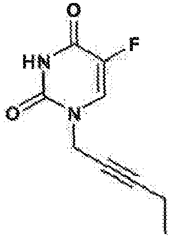

**1-(2-pentyn-1-yl)-5-fluorouracil (3d).** 56 mg, 37 % yield. R_{f} = 0.53 (10 % MeOH in DCM). ¹H NMR (500 MHz, DMSO) δ 11.86 (s, 1H), 8.10 (d, *J* = 6.6, 1H), 4.43 (t, *J* = 2.2, 2H), 2.21 (qt, *J* = 7.5, 2.2, 2H), 1.06 (t, *J* = 7.5, 3H). ¹³C NMR (126 MHz, DMSO) δ 157.31 (d, *J* = 25.9, C), 149.03, 139.71 (d, *J* = 230.2, C), 128.87 (d, *J* = 33.7, CH), 87.05, 73.53, 37.27 (CH₂), 13.45 (CH₃), 11.60 (CH₂). MS (ESI) m/z 195.0 [M-H]⁻.

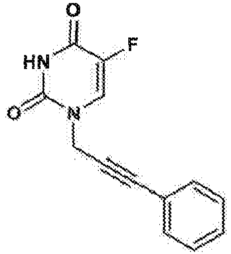

**1-(3-phenyl-1-propargyl)-5-fluorouracil (3e).** 66 mg, 35 % yield. R_{f} = 0.66 (10 % MeOH in DCM). ¹H NMR (500 MHz, DMSO) δ 11.92 (s, 1H), 8.22 (d, *J* = 6.6, 1H), 7.49 - 7.36 (m, 5H), 4.73 (s, 2H). ¹³C NMR (126 MHz, DMSO) δ 157.35 (d, *J* = 25.9, C), 149.12, 139.83 (d, *J* = 230.4, C), 131.53 (CH), 129.01 (d, *J* = 34.0, CH), 129.04 (CH), 128.69 (CH), 121.60, 84.38, 83.79, 37.64 (CH₂). MS (ESI) m/z 243.0 [M-H]⁻.

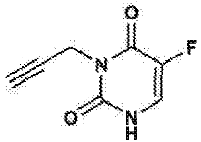

### Synthesis of N3-functionalized 5FU derivative 6

5-Fluoruouracil (100 mg, 0.77 mmol) was dissolved in a 2:1 mixture of acetonitrile and DMF (3 ml). Boc₂O (252 mg, 1.16 mmol) and DMAP (19 mg, 0.15 mmol) were subsequently added to the mixture and stirred overnight at room temperature. the solvents removed *in vacuo* and the resulting crude purified via flash chromatography (eluent: hexane/ EtOAc 3:1), to yield compound **4** as a white solid (70 mg, 40 %). ¹H NMR (500 MHz, DMSO) δ 10.60 (s, 1H), 7.64 (d, *J* = 4.7, 1H), 1.62 (s, 9H). ¹³C NMR (126 MHz, DMSO) δ 159.21 (d, *J* = 24.4, C), 150.70, 139.59 (d, *J* = 224.8, C), 123.02 (d, *J* = 31.6, CH), 61.45, 29.35 (CH₃). Compound **4** (56 mg, 0.24 mmol), propargyl bromide (31 µl, 0.29 mmol) and DBU (55 µl, 0.36 mmol) were dissolved in dry DCM (2 ml), and the mixture stirred at room temperature for 4 h. The solvents were removed *in vacuo* and the reaction crude purified via flash chromatography (eluent: hexane / EtOAc 5:1), to yield compound **5** as an colourless oil (43 mg, 67 %). ¹H NMR (500 MHz, CDCl₃) δ 7.40 (d, *J* = 4.6, 1H), 4.45 (d *J* = 2, 2H), 2.49 (t, *J* = 2.6, 1H), 1.68 (s, 9H). ¹³C NMR (126 MHz, CDCl₃) δ 159.08 (d, *J* = 24.3, C), 150.31, 140.51 (d, *J* = 233.9, C), 123.13 (d, *J* = 33.9, CH), 76.22, 75.88 (CH), 64.18, 38.01 (CH₂), 29.76 (CH₃). Compound **5** (28 mg, 0.1 mmol) and K₂CO₃ (7 mg, 0.05 mmol) were dissolved in MeOH (2 ml), and the mixture stirred at room temperature for 3 h. The mixture was stirred overnight, the solvents removed *in vacuo* and the resulting crude purified via flash chromatography (eluent: 3 % MeOH in DCM), to yield compounds **7** as a colourless solid (12 mg, 71 %). ¹H NMR (500 MHz, MeOD) δ 7.61 (d, *J* = 5.2, 1H), 4.64 (dd, *J* = 2.5, 0.5, 2H), 2.57 (t, *J* = 2.5, 1H). ¹³C NMR (126 MHz, MeOD) δ 158.82 (d, *J* = 25.8, C), 151.06, 141.35 (d, *J* = 229.9, C), 125.88 (d, *J* = 32.1, CH), 78.65, 71.94 (CH), 31.06 (CH₂). MS (ESI) m/z 167.0 [M-H]⁻.

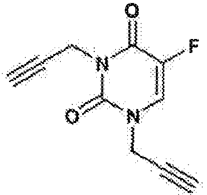

### Synthesis of 1,3-dipropargyl-5-fluorouracil (7)

5-Fluoruouracil (100 mg, 0.8 mmol) and DBU (345 µl, 2.3 mmol) were dissolved in dry DMF (2 ml) under a nitrogen atmosphere, and the mixture was cooled down to 4 °C in an ice bath. Propargyl bromide (170 µl, 1.6 mmol) was added dropwise and the reaction mixture allowed to warm up to room temperature. The mixture was stirred overnight, the solvents removed *in vacuo* and the resulting crude purified via flash chromatography (eluent: 1.5 % MeOH in DCM), to yield compounds **7** as a colourless solid (136 mg, 86 %). ¹H NMR (500 MHz, CDCl₃) δ 7.60 (d, *J* = 5.3, 1H), 4.72 (d, *J* = 2.4, 2H), 4.61 (d, *J* = 2.6, 2H), 2.56 (t, *J* = 2.6, 1H), 2.20 (t, *J* = 2.5, 1H). ¹³C NMR (126 MHz, CDCl₃) δ 156.19 (d, *J* = 26.0, C), 148.92, 140.26 (d, *J* = 237.3, C), 125.33 (d, *J* = 33.7, CH), 77.24, 76.74 (CH), 75.47, 71.51 (CH), 38.18 (CH₂), 31.27 (CH₂). MS (ESI) m/z 435.2 [2M+Na]⁺.

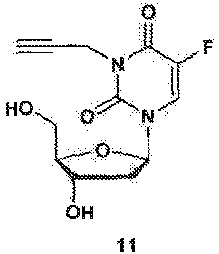

### Synthesis of 3-propargyl-floxuridine (11)

To a solution of floxuridine **8** (300 mg, 1.22 mmol) in dry DMF (8 ml) was added imidazole (605 mg, 8.89 mmol) and stirred at room temperature for 5 min. TBS-Cl (643 mg, 4.27 mmol) was then added to the mixture and the reaction stirred at room temperature for 2 h. The mixture was then concentrated *in vacuo,* dissolved in EtOAc (20 ml) and washed with H₂O (20 ml). The aqueous layer was then washed two more times with EtOAc. The organic layer were collected, washed with brine (60 ml), and dried over anhydrous MgSO₄. The product was then purified by column chromatography with EtOAc/hexane (2:1, v/v, DCM (R_{f} 0.74, EtOAc 2: 1 Hexane) to yield TBS-protected compound **9** as a white solid 552 mg (91%). ¹H NMR (500 MHz, CDCl3) δ 9.31 (d, *J* = 3.9, 1H, NH), 8.04 (d, *J* = 6.3, 1H, ArH), 6.29 (td, *J* = 6.3, 1.6, 1H, ArH), 4.44 - 4.38 (m, 1H, ArH), 3.96 - 3.90 (m, 2H, C*H*₂- OH), 3.77 (t, *J* = 6.0, 1H), 2.32 (ddd, *J* = 13.3, 6.1, 3.8, 1H), 2.09 - 2.01 (m, 1H), 0.94 - 0.91 (m, 9H), 0.90 - 0.87 (m, 9H), 0.12 (t, *J* = 3.2, 6H), 0.07 (t, *J* = 2.8, 6H). ¹³C NMR (126 MHz, CDCl3) δ 157.05 (d, *J_{CCF}* = 26.9, C), 148.98 (C), 140.63 (d, *J_{CF}* = 236.6, C), 124.43 (d, *J_{CCF}* = 34.0, CH), 88.23 (CH), 85.67 (CH), 71.67 (CH), 62.82 (CH₂), 41.95 (CH₂), 26.02 (CH₃), 25.85 (CH₃), 18.55 (C), 18.12 (C), -4.61 (d_{SiCH3}, *J* = 31.1, CH₃), -5.45 (d, *J*_{*SiCH*3} = 3.2, CH₃). LC-MS (m/z): 475.4 [M+H]⁺. Spectra is in accordance with published results (J. Med. Chem. 2004, 47, 1840-1846). Compound **9** (150 mg, 0.32 mmol) and DBU (168 µl, 1.12 mmol) were dissolved in dry DCM (3 ml) and stirred at room temperature for 5 min. Propargyl bromide (120 µl, 0.93 mmol) was added dropwise to the solution the reaction stirred at room temperature for 30 mim. Subsequently, additional 17 ml of DCM was added and the mixture was washed with H₂O (20 ml). The aqueous layer was then washed twice more with DCM (20 ml). The organic layers were collected, washed with brine (60 ml, x2) and dried over anhydrous MgSO₄ The crude product containing compound **10** was concentrated *in vacuo* and used in the next reaction without further purification. Compound **10** was dissolved in THF (2.5 ml) with TBAF solution (1 M in THF, 804 µl). The reaction was stirred at room temperature for 1 h. The mixture was then concentrated *in vacuo,* dissolved in a 3:1 chloroform - IPA mixture (20 ml) and washed with H₂O (20 ml). The aqueous layer was then washed twice a 3:1 chloroform - IPA mixture (20 ml). The organic layers were collected and dried over anhydrous MgSO₄. The resulting crude was purified by column chromatography (6 % MeOH in DCM) to yield compound **11** as a colourless gummy solid (52 mg, 58 %). R_{f} 0.47 (10 % MeOH in DCM). ¹H NMR (500 MHz, DMSO) δ 8.36 (d, *J* = 7.0, 1H, Ar*H*), 6.18 (td, *J* = 6.4, 1.5, 1H, Ar*H*), 5.28 (d, *J* = 4.3, 1 H, CH₂-O*H*), 5.19 (t, *J* = 4.9, 1 H, CH-O*H*), 4.54 (t, *J* = 2.0, 2H, C*H*₂-C≡CH), 4.25 (dt, *J* = 8.7, 4.3, 1H, Ar*H*), 3.81 (q, *J* = 3.3, 1H, ArH), 3.61 (qdd, *J* = 11.9, 4.9, 3.5, 2H, C*H*₂-OH), 3.18 (t, *J* = 2.4, 1H, C=CH), 2.19 - 2.12 (m, 2H, Ar*H*₂). ¹³C NMR (126 MHz, DMSO) δ 155.63 (d, *J_{CCF}*= 26.4, C), 148.28 (C), 139.29 (d, *J_{CF}* = 228.6, C), 124.04 (d, *J_{CCF}* = 34.6, CH), 87.72 (CH), 85.68 (CH), 78.41 (C), 73.52 (CH), 69.93 (CH), 60.86 (CH₂), 39.94 (CH₂), 30.49 (CH₂). LC-MS (m/z): 319.0 [M+Cl]⁻.

The specific compounds of the invention disclosed above for the fourth aspect of the invention may also be prepared in an analogous way.

### EXPERIMENTAL DATA

### Cell-free palladium mediated deprotection of prodrugs

To recreate a biocompatible scenario, prodrug-into-drug conversion was carried out at 37 °C in an isotonic solution with a physiologic pH. All-5FU, Pro-5FU and Bn-5FU (500 µM) were dissolved in phosphate buffered saline ("PBS") (0.5 ml) with 0.5 mg of Pd⁰ resin and shaken at 1400 rpm and 37 °C in a Thermomixer. Reaction crudes were monitored at 0 h, 7h, 24 h and 48 h using analytical HPLC (Agilent) or LCMS system (Agilent). Eluent A: water and formic acid (0.1%); eluent B: acetonitrile, formic acid (0.1%); A/B = 95 : 5 to 5 : 95 in 3 min, isocratic 1 min, 5 : 95 to 95 : 5 in 1 min, isocratic 1 min with the UV detector at 280nm.

Figures 3A-C show the LCMS chromatographs for the reaction Pro-5FU deprotection profile at sample times of 0 h, 7 h and 24 h. As seen in Figure 3C, Pro-5FU completely disappeared from the crude mixture after 24 h, with 5FU being the major reaction product. MS identified nontoxic 1-hydroxyacetone as the reaction byproduct, consistent with the production of an allenyl-palladium intermediate (see Figure 4 and, e.g. Rambabua, D. et al. Tetrahedron Lett. 54, 1169 (2013)).

For All-5FU and Bn-5FU, the prodrug (100 µM) and Pd⁰-resin (1 mg / mL, [Pd⁰] ∼ 266 µM) were dispersed in 0.1% (v/v) DMSO in PBS and incubated for 48h at 37 °C. Reaction crude was monitored by HPLC using the UV detector at 280nm. As indicated by the production of relatively low levels of 5FU after 48 h (see figure 4D for HPLC chromatograph for commercial 5FU), the respective allylic (Figure 3E) and benzylic (Figure 3F) pro-moieties showed significantly higher stability under the deprotection conditions compared to the propargyl group of Pro-5FU.

Figure 17 shows the deprotection of prodrugs of 5-fluorouaracil and floxuridine (FUDR). The data demonstrate *N*-depropargylation at both the 1- and 3-positions of the pyrimidine dione. The reactions of compounds 3 demonstrate *N*-depropargylation at the 1-position, while the reaction of compound 6 demonstrates *N*-depropargylation at the 3-position. The reaction of compound 7 shows *N*-depropargylation at both positions. These data also show how activation may be altered with differing steric bulk on the propargyl group.

*N*-depropargylation of Pro-FUDR was also effected (lower reaction scheme), floxuridine (FUDR) being a preferred heterocyclic compound of the invention. It is important to note that while FUDR is activated at a slightly slower rate than 5-FU, because FUDR is around 50 times more potent than 5-FU the effect is stronger.

### Confirmation of heterogeneous catalytic mechanism

*N*-depropargylation of Pro-5FU using sub-stoichiometric amounts of Pd⁰ confirmed the catalytic nature of the reaction with the reaction proceeding to completion in 72 h. To verify that Pro-5FU dealkylation is mediated by heterogeneous catalysis, Pd⁰-resins (2 mg) were incubated in PBS (2 mL) at 37 °C for 24 h, micro-filtered (Millipore microfilter, 0.22 µm) to eliminate solid contents and Pro-5FU added to the mixture (final concentration = 100 µM) for additional 48 h incubation. HPLC (UV detector at 280nm) detected unreacted Pro-5FU as major mixture component and minor quantities of 5FU (see Figure 15), indicating minimal escape of Pd⁰ into the solution.

### Biological activity

Colorectal HCT116 and pancreatic BxPC-3 cells were chosen as models for the antiproliferative studies because these are primary malignancies against which 5-FU is currently prescribed.

### Prodrug safety studies

The toxicities of 5FU and its prodrugs were compared by performing dose-response studies with 5FU and Pro-5FU (0.01 µM to 1mM). Doses of Pro-5FU and 5FU (0.01, 0.03, 0.1, 0.3, 1, 3, 10, 30 and 100 µM, for both cell lines, and additional doses of 300 µM and 1,000 µM for colorectal HCT116 cells) were incubated with cells for 5 days and cell viability measured to determine the corresponding EC₅₀ values. Cell viability was analysed by fluoresce intensity (λₑₓ=540 nm; λₑₘ=590 nm) after 45 min incubation with PrestoBiue™ Reagent (Life Technologies).

The cell viability data for HCT116 and BxPC-3 cells are provided in Figures 5A and 5B, respectively. EC₅₀ values calculated for 5FU were 2 µM and 0.136 µM, respectively. On the other hand, none of the prodrugs displayed a cytotoxic effect at the concentrations used, with Pro-5FU for example showing greater than 500 fold reduction in effect for HCT116 cells (i.e. EC₅₀ (Pro-5FU) / EC₅₀ (5FU) > 500) and greater than 700 fold reduction for BxPC-3 cells. These results illustrate the high efficacy and biochemical stability of the propargyl group in the cellular environment.

### Generation of drug from prodrug in cell culture and cytotoxic effects

The toxigenic effect as a result of in situ generation of 5FU in cell culture was determined by incubating colorectal HCT116 cells or pancreatic BXPc-3 cells in tissue culture media containing 0.1% (v/v) DMSO and a) Pd⁰-resin (1 mg / mL, negative control); b) prodrug (negative control); or c) Pd⁰-resin (1 mg / mL) + prodrug (reaction assay). Cells incubated in 0.1% (v/v) DMSO in media was used as an untreated cell reference standard (100% viability). A PrestoBlue® cell viability assay as described above was carried out and fluorescent intensities compared to the untreated cell control. Prodrug concentrations were 100 µM for HCT116 cells, and 30 µM for BxPC-3 cells.

Consistent with the outcome of the cell-free reactions above, the combination of Pro-5FU + Pd⁰-resins showed a strong toxigenic effect in both HCT116 and BxPC-3 cell lines (Figures 6A and 6B respectively), indicating the generation of 5FU in levels significantly greater than 2 µM (i.e. the calculated EC₅₀ value of 5FU in HCT116 cells). Bn-5FU combined with Pd⁰-resins showed only low levels of toxicity in both cell lines, which suggests the generation of low levels of 5FU, consistent with the cell-free experiments performed above. No appreciable cytotoxicity was observed for All-5FU. As shown in formulae 6A and 6B, the asterisks are used to indicate the relative level of the p-value (i.e. statistical significance) between the corresponding data. In these figures, "***" means p< 0.001. Further dose response toxicological data are shown in Figures 18 and 19.

### Dose response cell viability assay

To show extracellular efficacy of the palladium-mediated dealkylation of Pro-5FU, a range of concentrations of Pro-5FU and Pd⁰-resins were incubated independently (negative controls) and in combination (BOOM conversion assay) at varying doses to study of proliferation HCT116 and BXPc-3 cells in comparison to unmodified 5FU (positive control). A dose response study was performed for each cell line keeping the quantity of Pd⁰ resin constant (1 mg / mL).

HCT116 and BXPc-3 cells were plated in Dulbecco's Modified Eagle Media (DMEM) and Roswell Park Memorial Institute (RPMI) respectively, supplemented with serum (10 % FBS) and L-glutamine (2 mM). Cells were seeded in a 96 well plate format with a density of either 30,000 cells / mL and incubated for 48 h at 37 °C and 5% CO₂ before treatment. Each well was then replaced with fresh media containing: Pd⁰-resins (1 mg/ml) (negative control); prodrug (0.01 µM to 1 mM) in DMSO (0.1 % v/v) (negative control); 5FU (0.01 µM to 1 mM) in DMSO (0.1 % v/v) (positive control); or a combination of Pd⁰ resin + prodrug (0.01 µM to 1 mM in 0.1 % v/v DMSO). Cells incubated in 0.1% (v/v) DMSO in media were used as untreated cell reference standard (i.e. 100% cell viability). Cells were incubated in the fresh media for 5 days. PrestoBlue cell viability reagent (Life Technologies) (10 % v/v) was then added to each well and the plate incubated for 45 min. Fluorescence intensity values (detected using a PerkinElmer EnVision 2101 multilabel reader with excitation filter at 540 nm and emissions filter at 590 nm) were determined relative to the untreated cell control.

As shown in Figures 7A-B, the Pro-5FU / catalyst system showed significant cytotoxic effects at each concentration tested and calculated EC₅₀ values (see Figures 8A-B) similar to those calculated above for free 5FU:

| Cell line | EC₅₀ 5FU | EC₅₀ Pro-5FU/Catalyst |
|---|---|---|
| HCT116 | 2 µM | 2.6 µM |
| BXPc-3 | 136 nM | 209 nM |

### Real-time confluence study

To study the phenotypic effect of the prodrug / catalyst system compared to 5FU, cell proliferation was monitored for 5 days (120 h) using the high-content live-cell imaging system Incucyte™ (Essen BioScience) placed in an incubator (5% CO₂, 37 °C). Cell confluence analysis and supplementary movies 1 and 2 were carried out using the Incucyte software. Experiments (performed as described above for the dose response cell viability assay) were imaged by time-lapse contrast phase microscopy and cell growth represented as a function of total cell confluence.

An evident variance in the comparative level of cytotoxicity was however observed at several doses (Figures 8A-B). This is probably due to the time lag required for the reaction to take place (from hours up to 1 day) and reach cytotoxic levels of drug. This difference was insignificant in BxPC-3 cells at Pro-5FU doses higher than 1 µM, indicating that cytotoxic levels for this sensitive cell line are generated rapidly.

As observed from the growth vs time data in Figures 8A-B (Drug / prodrug concentrations of 100 µM for HCT116 cells, and 30 µM for BxPC-3 cells), cells treated with 5FU decreased rapidly after a few hours. The growth curve of HCT116 cells incubated with the Pd⁰-resin + Pro-5FU combination (Figure 9A) showed two distinct phases: a regular increment for 24 h followed by a drastic fall to reach comparable cytotoxic levels than 5FU at day 5, which is in accordance with the time delay required to generate cytotoxic levels of the drug for this cell line (i.e. up to 1 day). Due to the higher sensitivity of BxPC-3 cells to 5FU, both drug and the prodrug / catalyst combination experiments showed comparable bell-shaped population curves (Figure 9B).

Phase contrast images of cells after 4 days of treatment (96 h) are illustrated in Figures 10A-B. Furthermore, treatment-induced changes in cell morphology indicate analogous anti-proliferative mechanisms between 5FU and the prodrug system.

### Test for competitive inhibition by prodrug

Cells were incubated with 5FU (10 µM for HCT116 cells and 1 µM for BxPC-3 cells) in combination with increasing doses of Pro-5FU (0.1-1,000 µM) and cell viability determined. Incubation of 5FU with increasing concentrations of Pro-5FU up to a prodrug/drug ratio of 100:1 showed no antagonistic effect for either colorectal HCT116 cells (Figure 11A) or pancreatic BxPC-3 cells (Figure 11B). Thus, these results show that the prodrug does not have any detrimental effect on the biochemical pathway acted on by the drug.

### Zeptosens analysis and immunoprecipitation studies

Post-translational modifications of cancer relevant pathways (including p53) were quantified by antibody-based proteomics across time-series studies using Zeptosens Reverse Phase Protein Microarray analysis.

Cells were plated in their respective supplemented media in a 6 well plate at a density of 240,000 cells / mL and incubated for 48 h. Before adding to the cells, Pro-5FU (100 µM for HCT116) and Pd⁰-resins (1 mg/mL) were incubated for 24h to overcome the time relapse required to convert Pro-5FU into 5FU. Cells were treated with the Pd⁰ resin + Pro-5FU combination and the controls (as before: untreated cells; Pd⁰; Pro-5FU; and 5FU) and incubated for 6 and 24 h. Afterwards, the cells were washed with PBS (2 x 3 mL) and lysed with Zeptosens CLB1 lysis buffer (90 µl). Samples were analyzed by Zeptosens RPPA using the following specific conditions. Tumor cell lysates were normalized to a uniform protein concentration with spotting buffer CSBL1 (Zeptosens-Bayer) prior to preparing a final 4-fold concentration series of; 0.2; 0.15; 0.1 and 0.75mg/ml. The diluted concentration series of each sample was printed onto Zeptosens protein microarray chips (ZeptoChip™, Zeptosens-Bayer) under environmentally controlled conditions (constant 50% humidity and 14°C temperature) using a noncontact printer (Nanoplotter 2.1e, GeSiM). A single 400 Pico liter droplet of each lysate concentration was deposited onto the Zeptosens chip (thus representing 4 spots per each biological replicate). A reference grid of AlexaFluor647 conjugate BSA consisting of 4 column X 22 rows was spotted onto each sub-array, each sample concentration series were spotted in between reference columns. After array printing, the arrays were blocked with an aerosol of BSA solution using a custom designed nebulizer device (ZeptoFOG™, Zeptosen-Bayer) for 1 hour. The protein array chips were subsequently washed in double-distilled water and dried prior to performing a dual antibody immunoassay comprising of a 24 hour incubation of primary antibody (the library of primary antibodies used is in Table S1; provider Cell Signaling Technologies) followed by 2.5 hour incubation with secondary Alexa-Fluor conjugated antibody detection reagent (anti-rabbit A647 Fab, Invitrogen). Following secondary antibody incubation and a final wash step in BSA solution, the immunostained arrays were imaged using the ZeptoREADER™ instrument (Zeptosens-Bayer). For each-sub-array, five separate images were acquired using different exposure times ranging from 0.5-10 seconds. Microarray images representing the longest exposure without saturation of fluorescent signal detection were automatically selected for analysis using the ZeptoView™ 3.1 software. A weighted linear fit through the 4-fold concentration series was used to calculate relative fluorescence intensity (RFI) value for each sample replicate. Local normalization of sample signal to the reference BSA grid was used to compensate for any intra- or inter-array/chip variation. Local normalized RFI values were used for all subsequent analysis. Abundance levels of total p53 protein and phosphorylated p53 (Serine 15) were plotted as RFI data calculated by the ZeptoView™ 3.1 software.

As shown in figure 14A, total and Ser15-phosphorylated p53 were overexpressed in HCT116 cells at 24 h following exposure to drug or Pro-5FU / Pd⁰-resin combination, which correlates with the DNA damage response induced by 5FU activity (Stokk,et al. Mol. Cancer, 5, 20 (2006*)).* In contrast, no induction was observed in cells treated with either Pro-5FU or Pd⁰ resin on their own.

### Western Blot analysis.

Cell lysates for HCT116 cells were prepared as described above for Zeptosens analysis. The Protein samples (35 µg) and SeeBlue® Plus2 Pre-Stained Standard (7.5 µg) were separated by SDS PAGE (BioRad 4-15 % gels) and transferred to PVDF membranes (GE). Western blotting was performed using rabbit monoclonal antibodies against human p53 and p53-phospho-serine 15 (1:1000 for both, Cell Signaling Technologies, cat. No. 9282 and 9284 respectively) at 4 °C, overnight. This was followed by 1 hour incubation at room temperature with secondary HRP linked antibody (1:10 000, anti-rabbit IgG, Sigma). The loading control, actin, was mouse monoclonal antibody (1:40 000, Calbiochem, cat. No. CP01) followed by a secondary HRP linked antibody treatment (1:40 000, anti-mouse IgM, Calbiochem). HRP was detected by addition of POD ECL (Roche) and bands visualized using a ChemiDoc™ MP Imager (BioRad).

Effects observed on total and Ser15-phosphorylated p53 in the Zeptosens analysis after incubation with drug or Pro-5FU / Pd⁰-resin combination were corroborated by immunoprecipitation studies (figure 14B).

### Conclusions

The data show that the compounds (i.e. prodrugs) of the invention can be deprotected in a controlled manner using biocompatible palladium catalyst to generate free active drug *in situ,* which exhibits the desired biological activity. The data show that prodrugs of the invention are suitably non-toxic and do not interfere with the active drug pathway, thus providing ideal drug precursors. Furthermore, the by-products produced in the deprotection reaction are also biocompatible (e.g. propargyl groups provide 1-hydroxyacetone as the by-product).

The precise spatial control of prodrug deprotection provided by palladium implants, along with lack of toxicity of the prodrug compounds means that prodrugs of the invention can be deprotected specifically at the disease site, which should thus reduce general systemic concentration of the free drug. This is especially desirable in cancer treatments where side-effects resulting from the drug acting non-specifically on other organs in the body can be severe. This may also in turn allow prodrugs of the invention to be administered in higher doses, providing higher concentrations of drug at the disease site than would have been tolerated through general systemic administration of the active drug due to risk of the side-effects mentioned above.

It will be understood that the invention has been described by way of example only and modifications may be made whilst remaining within the scope of the appended claims.

## Claims

1. A method of preparing a heterocyclic compound or a salt thereof, the method comprising:
a) providing a first compound comprising a first group defined according to formula (II): 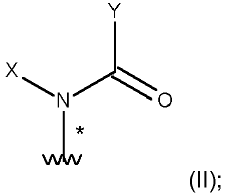 bonded to a second group defined according to formula (III) at the positions indicated by asterisks 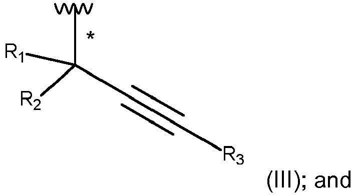
b) cleaving the bond between the first and second groups by reacting the first compound with palladium Pd(0) in aqueous conditions,
wherein
X and Y taken together with the endocyclic nitrogen atom and ring carbonyl group to which they are attached form a heterocyclic group; and
R₁, R₂ and R₃ are selected independently from the group consisting of H, C₁₋₄alkyl, C₂₋₄alkenyl, and phenyl,
wherein the heterocyclic compound or salt thereof is a drug compound selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, flucytosine, stavudine, telbivudine, zidovudine, trifluridine, entecavir and uramustine.

2. The method according to claim 1, wherein the palladium Pd(0) is provided in a scaffold or matrix support.

3. A compound or salt thereof comprising a first group defined according to formula (II) 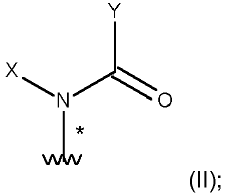 bonded to a second group defined according to formula (III) at the positions indicated by asterisks 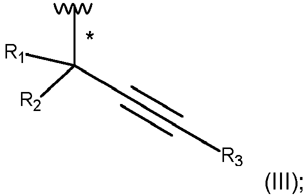 wherein
X and Y taken together with the endocyclic nitrogen atom and ring carbonyl group to which they are attached form a heterocyclic group;
R₁, R₂ and R₃ are selected independently from the group consisting of H, C₁₋₄alkyl, C₂₋₄alkenyl, and phenyl,
wherein the group defined according to formula (II) is a drug residue according to formula (II),
wherein the drug residue according to formula (II) is a residue of a drug selected from the group consisting of 5-fluorouracil, olaparib, ropinirole, pemetrexed, milrinone, amrinone, aciclovir, iodoxuridine, sunitinib, pimobendan, enoximone, cilostazol, nitrofurantoin, aripiprazole, sertindole, ziprasidone, mosapramine, phenobarbital, methylphenobarbital, primidone, lorazepam, nitrazepam, clonazepam, ethotoin, phenytoin, mephenytoin, fosphenytoin, floxuridine, Flucytosine, stavudine, telbivudine, zidovudine, trifluridine, entecavir and uramustine.

4. A compound according to claim 3 selected from the group consisting of: 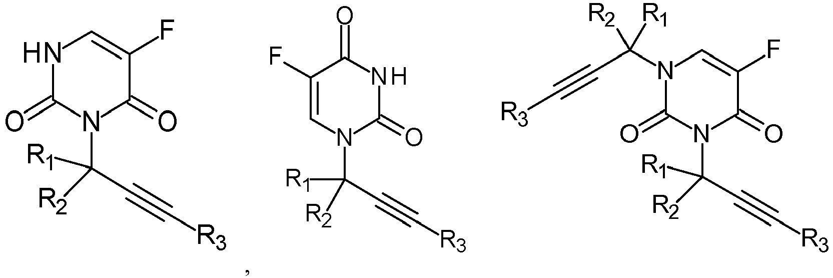 and 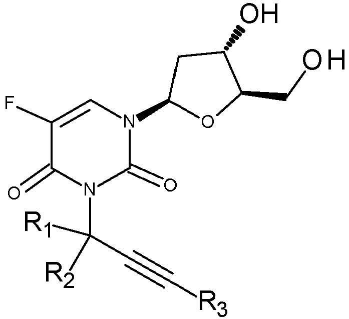 or a salt thereof.

5. A pharmaceutical composition comprising a compound or salt as defined in any of claims 3 or 4 and a pharmaceutically acceptable excipient, optionally wherein the pharmaceutical composition is provided in solid form.

6. A kit comprising a compound or salt as defined in any of claims 3 or 4, or a composition of claim 5; and palladium Pd⁰.

7. A compound or salt according to any one of claims 3 or 4, a composition according to claim 5 or a kit according to claim 6 for use in a method of treatment, wherein the method comprises co-administering the compound or composition and the palladium Pd(0) to a subject, or the method comprises administration of the compound or composition to a subject to which palladium Pd(0) has already been administered, optionally wherein the palladium is administered as an extracellular palladium Pd(0) implant.

8. A compound, salt, composition, or kit for use in the method of treatment according to claim 7 wherein the method of treatment is a method of treating cancer, Parkinson's disease, a viral infection, heart disease, convulsions, psychosis, a bacterial infection or a fungus infection.

9. A palladium Pd(0) implant for use in a method of treatment, wherein the method comprises co-administering a compound or salt according to any one of claims 3 or 4, or a composition according to claim 5 and the palladium Pd(0) implant to the subject, or the method comprises administration of a compound or salt according to any one of claims 3 or 4, or a composition according to claim 5 to a subject, wherein the subject has been pre-implanted with the palladium Pd(0) implant prior to administration of the compound or composition.

10. A palladium Pd(0) implant for use in a method of treatment according to claim 9, wherein the palladium Pd(0) is provided in a scaffold or matrix support.

## Patentansprüche

1. Verfahren zur Herstellung einer heterozyklischen Verbindung oder eines Salzes davon, wobei das Verfahren Folgendes umfasst:
a) Bereitstellen einer ersten Verbindung, die eine erste Gruppe umfasst, die gemäß Formel (II) definiert ist: 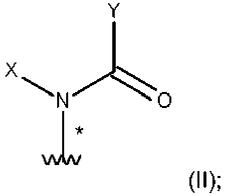 und an Positionen, die durch Asteriske angezeigt sind, an eine zweite Gruppe gebunden ist, die gemäß Formel (III) definiert ist 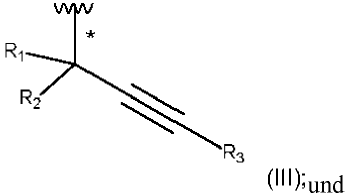
b) Spalten der Verbindung zwischen der ersten und der zweiten Gruppe durch ein Reagieren der ersten Verbindung mit Palladium Pd(0) unter wässrigen Bedingungen, wobei X und Y zusammengenommen mit der endozyklischen Stickstoffatom-Ringcarbonylgruppe, an der sie befestigt sind, eine heterozyklische Gruppe bilden;
und R₁, R₂ und R₃ unabhängig ausgewählt sind aus der Gruppe bestehend aus H, C₁₋₄-Alkyl;C₂₋₄-Alkenyl und Phenyl, wobei die heterozyklische Verbindung oder das Salz davon eine Arzneimittelverbindung ist ausgewählt aus der Gruppe bestehend aus 5-Fluorouracil, Olaparib, Ropinirol, Pemetrexed, Milrinon, Amrinon, Aciclovir, Idoxuridin, Sunitinib, Pimobendan, Enoximon, Cilostazol, Nitrofurantoin, Aripiprazol, Sertindol, Ziprasidon, Mosapramin, Phenobarbital, Methylphenobarbital, Primidon, Lorazepam, Nitrazepam, Clonazepam, Ethotoin, Phenytoin, Mephenytoin, Fosphenytoin, Floxuridin, Flucytosin, Stavudin, Telbivudin, Zidovudin, Trifluridin, Entecavir und Uramustin.

2. Verfahren nach Anspruch 1, wobei das Palladium Pd(0) in einem Gerüst oder einem Matrixträger bereitgestellt ist.

3. Verbindung oder Salz davon, die/das eine erste Gruppe umfasst, die definiert ist gemäß Formel (II) 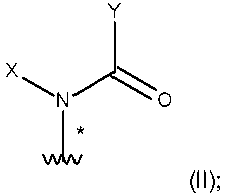 die an den Positionen, die durch Asteriske angezeigt sind, an eine zweite Gruppe gebunden ist, die definiert ist gemäß Formel (III) 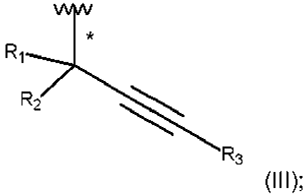 wobei X und Y zusammengenommen mit der endozyklischen Stickstoffatom-Ringcarbonylgruppe, an der sie befestigt sind, eine heterozyklische Gruppe bilden; R₁, R₂ und R₃ unabhängig ausgewählt sind aus der Gruppe bestehend aus H, C₁₋₄-Alkyl, C₂₋₄-Alkenyl und Phenyl, wobei die Gruppe, die gemäß Formel (II) definiert ist ein Arzneimittelrest gemäß Formel (II) ist, wobei der Arzneimittelrest gemäß Formel (II) ein Rest eines Arzneimittels ist ausgewählt aus der Gruppe bestehend aus 5-Fluorouracil, Olaparib, Ropinirol, Pemetrexed, Milrinon, Amrinon, Aciclovir, Idoxuridin, Sunitinib, Pimobendan, Enoximon, Cilostazol, Nitrofurantoin, Aripiprazol, Sertindol, Ziprasidon, Mosapramin, Phenobarbital, Methylphenobarbital, Primidon, Lorazepam, Nitrazepam, Clonazepam, Ethotoin, Phenytoin, Mephenytoin, Fosphenytoin, Floxuridin, Flucytosin, Stavudin, Telbivudin, Zidovudin, Trifluridin, Entecavir und Uramustin.

4. Verbindung nach Anspruch 3 ausgewählt aus der Gruppe bestehend aus: 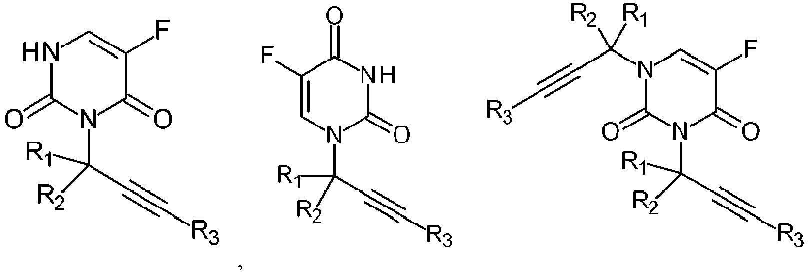 und 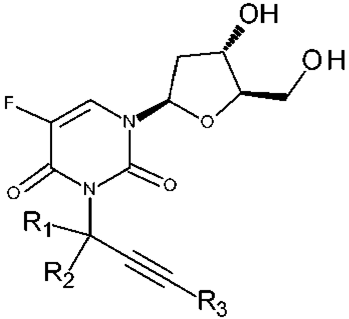 oder einem Salz davon.

5. Pharmazeutische Zusammensetzung umfassend eine Verbindung oder ein Salz nach einem der Ansprüche 3 oder 4 und einen pharmazeutisch annehmbaren Hilfsstoff, optional wobei die pharmazeutische Zusammensetzung in einer festen Form bereitgestellt ist.

6. Set umfassend eine Verbindung oder ein Salz nach einem der Ansprüche 3 oder 4 oder eine Zusammensetzung nach Anspruch 5; und Palladium Pd⁰.

7. Verbindung oder Salz nach einem der Ansprüche 3 oder 4, Zusammensetzung nach Anspruch 5 oder Set nach Anspruch 6 zur Verwendung in einem Behandlungsverfahren, wobei das Verfahren ein gleichzeitiges Verabreichen der Verbindung oder der Zusammensetzung und des Palladiums Pd(0) an ein Subjekt umfasst, oder wobei das Verfahren ein Verabreichen der Verbindung oder der Zusammensetzung an ein Subjekt umfasst, dem bereits Palladium Pd(0) verabreicht worden ist, optional wobei das Palladium als ein extrazelluläres Palladium-Pd(0)-Implantat verabreicht wird.

8. Verbindung, Salz, Zusammensetzung oder Set zur Verwendung in dem Behandlungsverfahren nach Anspruch 7, wobei das Behandlungsverfahren ein Verfahren zur Behandlung von Krebs, der Parkinson-Krankheit, einer Virusinfektion, einer Herzkrankheit, Konvulsionen, Psychosen, einer bakteriellen Infektion oder einer Pilzinfektion ist.

9. Palladium-Pd(0)-Implantat zur Verwendung in einem Behandlungsverfahren, wobei das Verfahren ein gleichzeitiges Verabreichen einer Verbindung oder eines Salzes nach einem der Ansprüche 3 oder 4 oder einer Zusammensetzung nach Anspruch 5 und des Palladium-Pd(0)-Implantats an ein Subjekt umfasst, oder wobei das Verfahren ein Verabreichen einer Verbindung oder eines Salzes nach einem der Ansprüche 3 oder 4 oder einer Zusammensetzung nach Anspruch 5 an ein Subjekt umfasst, wobei das Palladium-Pd(0)-Implantat dem Subjekt vor der Verabreichung der Verbindung oder der Zusammensetzung implantiert wurde.

10. Palladium-Pd(0)-Implantat zur Verwendung in einem Behandlungsverfahren nach Anspruch 9, wobei das Palladium Pd(0) in einem Gerüst oder einem Matrixträger bereitgestellt ist.

## Revendications

1. Procédé de préparation d'un composé hétérocyclique ou d'un sel de celui-ci, le procédé comprenant :
a) la fourniture d'un premier composé comprenant un premier groupe défini selon la formule (II) : 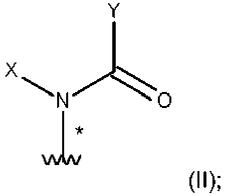 fixé à un second groupe défini selon la formule (III) aux positions indiquées par les astérisques 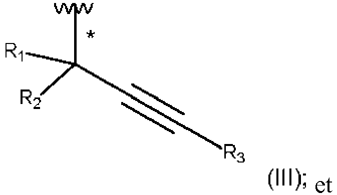
b) le clivage de la liaison entre le premier et le second groupe par mise en réaction du premier composé avec du palladium Pd(0) dans des conditions aqueuses,
X et Y pris conjointement avec l'atome d'azote endocyclique et le groupe carbonyle de cycle auquel ils sont attachés formant un groupe hétérocyclique ; et
R₁, R₂ et R₃ sont choisis indépendamment parmi le groupe constitué par H, alkyle en C₁₋₄, alcényle en C₂₋₄ et phényle, le composé hétérocyclique ou son sel étant un composé médicamenteux choisi parmi le groupe constitué de 5-fluorouracile, olaparib, ropinirole, pémétrexed, milrinone, amrinone, aciclovir, iodoxuridine, sunitinib, pimobendan, énoximone, cilostazol, nitrofurantoïne, aripiprazole, sertindole, ziprasidone, mosapramine, phénobarbital, méthylphénobarbital, primidone, lorazépam, nitrazépam, clonazépam, éthotoïne, phénytoïne, méphénytoïne, fosphénytoïne, floxuridine, flucytosine, stavudine, telbivudine, zidovudine, trifluridine, entécavir et uramustine.

2. Procédé selon la revendication 1, dans lequel le palladium Pd(0) est fourni dans un support d'échafaudage ou de matrice.

3. Composé ou sel de celui-ci comprenant un premier groupe défini selon la formule (II) 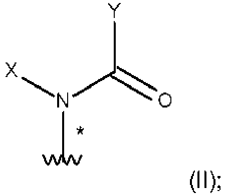 lié à un second groupe défini selon la formule (III) aux positions indiquées par les astérisques 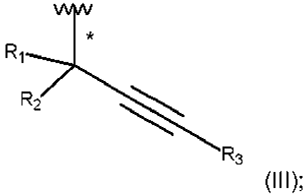 dans lequel
X et Y pris conjointement avec l'azote endocyclique et le groupe carbonyle de cycle auquel ils sont attachées forment un groupe hétérocyclique ;
R₁, R₂ et R₃ sont choisis indépendamment parmi le groupe constitué de H, alkyle en C₁₋₄, alcényle en C₂₋₄, et phényle, le groupe défini selon la formule (II) étant un résidu médicamenteux selon la formule (II), le résidu médicamenteux selon la formule (II) étant un résidu d'un médicament choisi parmi le groupe constitué de 5-fluorouracile, olaparib, ropinirole, pémétrexed, milrinone, amrinone, aciclovir, iodoxuridine, sunitinib, pimobendan, énoximone, cilostazol, nitrofurantoïne, aripiprazole, sertindole, ziprasidone, mosapramine, phénobarbital, méthylphénobarbital, primidone, lorazépam, nitrazépam, clonazépam, éthotoïne, phénytoïne, méphénytoïne, fosphénytoïne, floxuridine, flucytosine, stavudine, telbivudine, zidovudine, trifluridine, entécavir et uramustine.

4. Composé selon la revendication 3 choisi parmi le groupe constitué par : 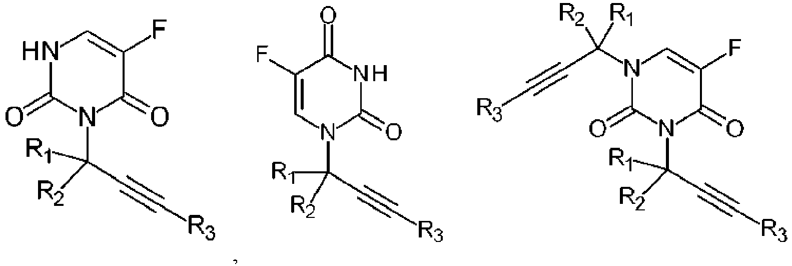 et 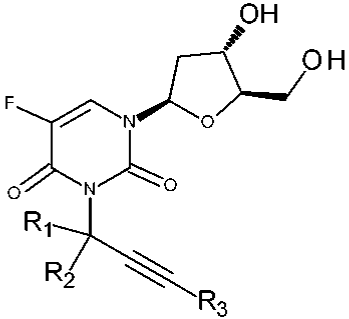 ou un de ses sels.

5. Composition pharmaceutique comprenant un composé ou un sel tel que défini dans l'une quelconque des revendications 3 ou 4 et un excipient pharmaceutiquement acceptable, la composition pharmaceutique étant facultativement fournie sous forme solide.

6. Kit comprenant un composé ou un sel tels que définis dans l'une quelconque des revendications 3 ou 4, ou une composition de la revendication 5 ; et palladium Pd⁰.

7. Composé ou sel selon l'une quelconque des revendications 3 ou 4, composition selon la revendication 5 ou kit selon la revendication 6 pour l'utilisation dans une méthode de traitement, la méthode comprenant la co-administration du composé ou de la composition et du palladium Pd(0) à un sujet, ou la méthode comprenant l'administration du composé ou de la composition à un sujet auquel du palladium Pd(0) a déjà été administré, le palladium étant facultativement administré sous forme d'un implant extracellulaire de palladium Pd(0).

8. Composé, sel, composition ou kit pour l'utilisation dans la méthode de traitement selon la revendication 7, la méthode de traitement étant une méthode pour traiter le cancer, la maladie de Parkinson, une infection virale, une cardiopathie, des convulsions, une psychose, une infection bactérienne ou une infection fongique.

9. Implant de palladium Pd(0) pour l'utilisation dans une méthode de traitement, la méthode comprenant la co-administration d'un composé ou d'un sel selon l'une quelconque des revendications 3 ou 4, ou d'une composition selon la revendication 5 et l'implant de palladium Pd(0) au sujet, ou la méthode comprenant l'administration d'un composé ou d'un sel selon l'une quelconque des revendications 3 ou 4, ou d'une composition selon la revendication 5 à un sujet, le sujet ayant été pré-implanté avec l'implant de palladium Pd(0) avant l'administration du composé ou de la composition.

10. Implant de palladium Pd(0) pour l'utilisation dans une méthode de traitement selon la revendication 9, dans lequel le palladium Pd(0) est fourni dans un support d'échafaudage ou de matrice.
